# EUROPEAN PATENT APPLICATION

(11) **EP 3 757 130 A1**
(43) Date of publication of application: **30.12.2020**
(21) Application number: 20174880.3
(22) Date of filing: 25.09.2014
(51) Int. Cl.: C07K 16/28, A61N 5/00, A61K 39/395

(54) **METHODS FOR TREATING HEMATOLOGIC CANCERS**

(30) Priority: 26.09.2013 US 201361882702 P; 25.06.2014 US 201462017192 P
(62) Document number(s) of the earlier application(s) in accordance with Art. 76 EPC: 14848888.5 / 3 049 442
(71) Applicant: Costim Pharmaceuticals Inc., Cambridge, MA 02139 (US); The Medical College of Wisconsin, Inc., Milwaukee, WI 53226 (US)
(72) Inventor: JOHNSON, Bryon, Duane, Jackson, WI 53037 (US); MILLMAN, Robert, Boston, MA 02115 (US)
(74) Representative: Elkington and Fife LLP

(57) **Abstract**

The present invention relates to methods of treating hematologic cancers using a combination of inhibitors of PD-1 or PD-L1 and TIM-3, LAG-3 or CTLA4.

## Description

### Cross Reference to Related Applications

This application claims the benefit of U.S. Provisional Application No. 61/882,702, filed September 26, 2013 and U.S. Provisional Application No. 62/017,192, filed June 25, 2014, the contents of which are hereby incorporated by reference in their entirety.

### Statement of Rights

This invention was made with government support under Grant NCATS 8UL1TR000055 awarded by the National Institutes of Health. The U.S. government has certain rights in the invention. This statement is included solely to comply with 37 C.F.R. § 401.14(a)(f)(4) and should not be taken as an assertion or admission that the application discloses and/or claims only one invention.

### Background of the Invention

Hematologic malignancies encompass cancers affecting blood, bone marrow, and lymph nodes and are particularly affected by modulation of the immune system since immune system cells are derived from hematologic lineages. Although hematologic cancers have traditionally been treated with conventional drug therapies, such as alkylating and other DNA damaging compounds, it is increasingly becoming recognized that immune checkpoint regulators play critical roles in determining whether hematologic cancer cells are tolerated or attacked by the immune system (Wu et al. (2012) Int. J. Biol. Sci. 8:1420-1430; Nirschl and Drake (2013) Clin. Cancer Res., electronically published July 18; Ceeraz et al. (2013) Trends Immunol., electronically published August 13). However, immune checkpoint regulators, such as CTLA-4, PD-1, VISTA, B7-H2, B7-H3, PD-L1, B7-H4, B7-H6, 2B4, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, LAG-3, BTLA, A2aR and many more, negatively regulate immune response progression based on complex and combinatorial interactions between numerous inputs. While some progress has been made to determine which interventions at which particular nodes of the immune checkpoint regulatory system can be targeted for benefiting the treatment of hematologic cancers (Kearl et al. (2013) J. Immunol. 190:5620-5628; Hallett et al. (2011) Biol. Blood Marrow Transplant. 17:1133-1145; Pardoll et al. (2012) Nat. Rev. Cancer 12:252-264; Brahmer et al. (2012) N. Engl. J. Med. 366:2455-2465; Mocellin et al. (2013) Biochim. Biophys. Acta 1836:187-196; Topalian et al. (2012) N. Engl. J. Med. 366:2443-2454; and Wolchok et al. (2013) N. Engl. J. Med. 369:122-133), it is not currently possible to identify specific interactions having synergistic anti-cancer therapeutic efficacy. Accordingly, there is a great need in the art to define specific and synergistic combinations of immune checkpoint regulators useful for treating hematologic cancers.

### Summary of the Invention

The present invention is based, at least in part, on the discovery that inhibiting or blocking Programmed Death 1 (PD-1) or Programmed Death-1 Ligand (PD-L1) in combination with inhibition of an immune checkpoint inhibitor (e.g., one or more of TIM-3, LAG-3 or CTLA4) results in a synergistic therapeutic benefit for treating a hematologic cancer, *e.g.,* a myeloma. This finding is unexpected given the lack of such benefit observed for inhibiting or blocking other combinations of immune checkpoint regulators.

Accordingly, in one aspect, the invention features a method of treating a subject afflicted with a hematologic cancer comprising administering to the subject an inhibitor of PD-1 or PD-L1, and an inhibitor of an immune checkpoint regulator (*e.g*., an inhibitor of one or more of TIM-3, LAG-3 or CTLA4). In one embodiment, an inhibitor of PD-1 or PD-L1 is administered in combination with an inhibitor of TIM-3. In another embodiment, an inhibitor of PD-1 or PD-L1 is administered in combination with an inhibitor of LAG-3. In yet another embodiment, an inhibitor of PD-1 or PD-L1 is administered in combination with an inhibitor of CTLA-4. Inhibition as described herein can be performed by inhibition at the DNA, RNA or protein level. In embodiments, an inhibitory nucleic acid (*e.g.,* a dsRNA, siRNA or shRNA), can be used to inhibit expression of an inhibitory molecule. In other embodiments, the inhibitor is a polypeptide, *e.g*., a soluble ligand, or an antibody or antigen-binding fragment thereof, that binds to PD-1 or PD-L1, or other immune checkpoint regulator. Examples of each of the aforesaid inhibitors are provided in more detail below. The term "inhibition" or "inhibitor" includes a reduction in a certain parameter, *e.g.,* an activity, of a given molecule, *e.g.,* an immune checkpoint regulator. For example, inhibition of an activity, *e.g*., a PD-1 activity, of at least 5%, 10%, 20%, 30%, 40% or more is included by this term. Thus, inhibition need not be 100%. Activities for the immune checkpoint regulators can be determined as described herein or assays known in the art.

In one embodiment, the inhibitor is a bispecific or multispecific antibody selective for PD-1 or PD-L1 and TIM-3, LAG-3 or CTLA4. In another embodiment, a combination of inhibitors comprising a first inhibitor that selectively inhibits or blocks PD-1 or PD-L1 and a second inhibitor that selectively inhibits or blocks TIM-3, LAG-3 or CTLA4 is provided. In one embodiment, the inhibitor is a soluble ligand, e.g., a soluble ligand of PD-1, PD-L1, TIM-3, LAG-3 or CTLA-4 (*e.g.,* a CTLA-4-Ig). In still another embodiment, the first inhibitor and/or second inhibitor is an antibody or an antigen binding fragment thereof, which specifically binds to PD-1 or PD-L1 and/or TIM-3, LAG-3 or CTLA4. In yet another embodiment, the antibody, or antigen binding fragment thereof, is murine, chimeric, humanized, composite, or human. In another embodiment, the antibody, or antigen fragment thereof, is detectably labeled, comprises an effector domain, comprises an Fc domain, and/or is selected from the group consisting of Fv, Fav, F(ab')2), Fab', dsFv, scFv, sc(Fv)2, and diabodies fragments. In still another embodiment, the antibody, or antigen binding fragment thereof, is conjugated to a cytotoxic agent (*e.g.,* a chemotherapeutic agent, a biologic agent, a toxin, a radioactive isotope, and the like).

For example, an anti-PD-1 or PD-L1 antibody, or antigen binding fragment thereof, can be administered in combination with an anti-LAG-3 antibody or an antigen-binding fragment thereof. In another embodiment, an anti-PD-1 or PD-L1 antibody, or antigen binding fragment thereof, is administered in combination with an anti-TIM-3 antibody or antigen-binding fragment thereof. In yet other embodiments, an anti-PD-1 or PD-L1 antibody, or antigen binding fragment thereof, is administered in combination with an anti-LAG-3 antibody and an anti-TIM-3 antibody, or antigen-binding fragments thereof. In yet other embodiments, an anti-PD-1 or PD-L1 antibody, or antigen binding fragment thereof, is administered in combination with an anti-CTLA-4 antibody, or antigen binding fragment thereof (*e.g*., ipilimumab). Any combination of the aforesaid antibodies can be used in the methods described herein. The combination of antibodies recited herein can be administered separately, *e.g*., as separate antibodies, or linked, *e.g.,* as a bispecific or trispecific antibody molecule. In one embodiment, a bispecific antibody that includes an anti-PD-1 or PD-L1 antibody, or antigen binding fragment thereof, and an anti-TIM-3, anti-LAG-3 antibody or anti-CTLA4 antibody, or antigen-binding fragment thereof, is administered.

In addition to, or in place of antibodies and antigen binding fragments thereof, numerous other agents are contemplated. For example, in one embodiment, the agent comprises an RNA interfering agent which inhibits expression of PD-1 or PD-L1 and/or TIM-3, LAG-3 or CTLA4 (e.g., a small interfering RNA (siRNA), small hairpin RNA (shRNA), or a microRNA (miRNA)). In another embodiment, the agent comprises an antisense oligonucleotide complementary to PD-1 or PD-L1 and/or TIM-3, LAG-3 or CTLA4. In still another embodiment, the agent comprises a peptide or peptidomimetic that inhibits or blocks PD-1 or PD-L1 and/or TIM-3, LAG-3 or CTLA4. In yet another embodiment, the agent comprises a small molecule that inhibits or blocks PD-1 or PD-L1 and/or TIM-3, LAG-3 or CTLA4 *(e.g.,* a small molecule that inhibits a protein-protein interaction between PD-L1 and a PD-L1 receptor and/or TIM-3 and a TIM-3 receptor). In another embodiment, the agent comprises an aptamer that inhibits or blocks PD-1 or PD-L1 and/or TIM-3, LAG-3 or CTLA4.

Numerous adaptations to the methods described herein are contemplated. For example, in one embodiment, that at least one agent is administered in a pharmaceutically acceptable formulation. In another embodiment, the method further comprises administering to the subject a therapeutic agent for treating the hematologic cancer. In still another embodiment, the method further comprises a step of transient or complete lymphodepletion (e.g., sublethal whole body irradiation used for transient lymphodepletion or lethal whole body irradiation used for complete lymphodepletion). In yet another embodiment, the step of lymphodepletion occurs before, concurrently with, or after the step of agent administration. In another embodiment, the hematologic cancer is selected from the group consisting of multiple myeloma, acute lymphocytic leukemia, acute myeloid leukemia, chronic lymphocytic leukemia, small lymphocytic lymphoma, non-Hodgkin's lymphoma, Hodgkin's lymphoma, mantle cell lymphoma, follicular lymphoma, Waldenstrom's macroglobulinemia, B-cell lymphoma and diffuse large B-cell lymphoma, precursor B-lymphoblastic leukemia/lymphoma, B-cell chronic lymphocytic leukemia/small lymphocytic lymphoma, B-cell prolymphocytic leukemia, lymphoplasmacytic lymphoma, splenic marginal zone B-cell lymphoma (with or without villous lymphocytes), hairy cell leukemia, plasma cell myeloma/plasmacytoma, extranodal marginal zone B-cell lymphoma of the MALT type, nodal marginal zone B-cell lymphoma (with or without monocytoid B cells), Burkitt's lymphoma; precursor T-lymphoblastic lymphoma/leukemia, T-cell prolymphocytic leukemia, T-cell granular lymphocytic leukemia, aggressive NK cell leukemia, adult T-cell lymphoma/leukemia (HTLV 1-positive), nasal-type extranodal NK/T-cell lymphoma, enteropathy-type T-cell lymphoma, hepatosplenic γ-δ T-cell lymphoma, subcutaneous panniculitis-like T-cell lymphoma, mycosis fungoides/Sézary syndrome, anaplastic large cell lymphoma (T/null cell, primary cutaneous type), anaplastic large cell lymphoma (T-/null-cell, primary systemic type), peripheral T-cell lymphoma not otherwise characterized, angioimmunoblastic T-cell lymphoma, polycythemia vera (PV), myelodysplastic syndrome (MDS), indolent Non-Hodgkin's Lymphoma (iNHL) and aggressive Non-Hodgkin's Lymphoma (aNHL). In some embodiments, the hematologic cancer is selected from the group consisting of B-cell lymphoma, myeloid leukemia and multiple myeloma or can be multiple myeloma alone. In another embodiment, the subject is a mammal, optionally wherein the mammal is a human.

In another aspect, a kit for treating a subject afflicted with a hematologic cancer comprising an inhibitor of PD-1 or PD-L1, and an inhibitor of an immune checkpoint regulator (*e.g.,* an inhibitor of one or more of TIM-3, LAG-3 or CTLA4), is provided. In one embodiment, the inhibitor is a bispecific or multispecific antibody, or antigen binding fragment thereof, selective for both PD-1 or PD-L1 and TIM-3, LAG-3 or CTLA4. Similarly, a kit for treating a subject afflicted with a hematologic cancer comprising a first agent that selectively inhibits or blocks PD-1 or PD-L1 and a second agent that selectively inhibits or blocks TIM-3, LAG-3 or CTLA4, is provided. In one embodiment, the first agent and/or second agent is an antibody, or an antigen binding fragment thereof, which specifically binds to PD-1 or PD-L1 protein and/or TIM-3, LAG-3 or CTLA4 protein. Any antibody, or antigen binding fragment thereof, provided in a kit can be murine, chimeric, humanized, composite, or human. In another embodiment, the antibody, or antigen binding fragment thereof, is detectably labeled, comprises an effector domain, comprises an Fc domain, and/or is selected from the group consisting of Fv, Fav, F(ab')2), Fab', dsFv, scFv, sc(Fv)2, and diabodies fragments. In still another embodiment, the antibody, or antigen binding fragment thereof, is conjugated to a cytotoxic agent (e.g., a chemotherapeutic agent, a biologic agent, a toxin, and a radioactive isotope). In yet another embodiment, the agent is selected from the group consisting of a) an RNA interfering agent which inhibits expression of PD-1 or PD-L1 and/or TIM-3, LAG-3 or CTLA4, optionally wherein said RNA interfering agent is an small interfering RNA (siRNA), small hairpin RNA (shRNA), or a microRNA (miRNA); b) an antisense oligonucleotide complementary to PD-1 or PD-L1 and/or TIM-3, LAG-3 or CTLA4; c) a peptide or peptidomimetics that inhibits or blocks PD-1 or PD-L1 and/or TIM-3, LAG-3 or CTLA4; d) a small molecule that inhibits or blocks PD-1 or PD-L1 and/or TIM-3, LAG-3 or CTLA4, optionally wherein said small molecule inhibits a protein-protein interaction between PD-L1 and a PD-L1 receptor and/or other immune checkpoint regulator (e.g., TIM-3 and a TIM-3 receptor); and e) an aptamer that inhibits or blocks PD-1 or PD-L1 and/or TIM-3, LAG-3 or CTLA4.

### Brief Description of the Drawings

**Figure 1A** shows representative flow cytometry dot plots depicting accumulation of tumor cells in myeloma bearing mice over time.
**Figure 1B** shows percentages of CD4⁺ and CD8⁺ T cells expressing indicated immune checkpoint proteins in myeloma bearing mice over time. Data shown are representative of more than four independent analyses. * p<0.05, ** p<0.01, *** p<0.001 as compared to T cells from naive non-myeloma bearing mice.
**Figure 1C** shows expression of Tim-3 and PD-1, Lag-3 and PD-1, or 2B4 and PD-1 on gated CD8⁺ or CD4⁺ T cells from bone marrow of moribund mice.
**Figure 2** shows representative flow cytometry dot plots depicting expression of indicated immune checkpoint proteins on CD4⁺ Treg cells in myeloma bearing mice and control mice.
**Figure 3A** shows expression of Tim-3 and PD-1, Lag-3 and PD-1, or 2B4 and PD-1 on gated CD8⁺ T cells from myeloma bearing mice treated with sublethal whole body irradiation and anti-PD-L1 antibody. Data shown are representative of more than four independent analyses.
**Figure 3B** shows frequency of CD8⁺Tim-3⁺, CD8⁺Lag-3⁺ and CD8⁺2B4⁺ cells in spleens of anti-PD-L1 antibody treated myeloma bearing mice compared with spleens of control antibody treated. ***p<0.001.
**Figure 4** shows a schematic diagram of the experimental treatment protocol used to establish and treat myelomas in mice in Example 1.
**Figure 5** shows survival data of myeloma bearing mice treated with *inter alia* a blocking anti-PD-L1 antibody, a blocking anti-TIM-3 antibody, or combinations thereof.
**Figure 6** shows survival data of myeloma bearing mice treated with *inter alia* a blocking anti-PD-L1 antibody, a blocking anti-LAG-3 antibody, or combinations thereof.
**Figure 7** shows survival data of myeloma bearing mice treated with *inter alia* a blocking anti-TIM-3 antibody, a blocking anti-LAG-3 antibody, or combinations thereof.
**Figure 8** shows survival data of myeloma bearing mice treated with *inter alia* a blocking anti-PD-L1 antibody and a blocking anti-PD-1 antibody.
**Figure 9A** shows a schematic diagram of the experimental treatment protocol used to establish and treat myelomas in mice in Example 2.
**Figure 9B** shows survival curves of myeloma bearing mice treated with a blocking anti-Tim-3 antibody only, a blocking anti-Lag-3 antibody only, or in combination with a blocking anti-PD-L1 antibody. Survival was compared with control antibody treated mice or mice treated with anti-PD-L1 antibody only. A combination of anti-Lag-3 and anti-Tim-3 antibodies was also tested. Survival curves represent combined data from three independent experiments; n=10-15 mice per experimental group.
**Figure 9C** shows survival curves of myeloma bearing mice treated with a blocking anti-CTLA4 antibody only, or in combination with a blocking anti-PD-L1 antibody. Survival was compared with control antibody treated mice or mice treated with anti-PD-L1 antibody only. Survival curves represent combined data from three independent experiments; n=10-15 mice per experimental group.
**Figure 9D** shows survival curves of myeloma bearing mice treated with a blocking anti-CD48 antibody only, or in combination with a blocking anti-PD-L1 antibody. Survival was compared with control antibody treated mice or mice treated with anti-PD-L1 antibody only. Survival curves represent combined data from two independent experiments; n=10-15 mice per experimental group.
**Figure 9E** shows survival curves of some of the survivors from Figures 9B-9C rechallenged with 5T33 myeloma cells on day 110. P values were determined by the log-rank test.
**Figure 10A** shows frequencies of tumor-reactive CD8⁺ and CD4⁺ T cells in the spleens and bone marrow of mice treated with combinations of immune checkpoint protein blockade. The graphs are representative of three independent experiments in which the CD8⁺ or CD4⁺ T cells for each group were pooled from 5-7 individual mice.
**Figure 10B** shows levels of cytokine production by CD8⁺ T cells purified from the spleens of mice treated with combinations of immune checkpoint protein blockade. The graphs are representative of two independent experiments in which the CD8⁺ T cells for each group were pooled from 5 individual mice. *p < 0.05, **p < 0.01 as compared with T cells from mice treated with anti-PD-L1 alone.
**Figure 11A** shows expression of PD-1 on gated CD8⁺ T cells from spleen and bone marrow of mice treated with different blocking antibodies or control IgG.
**Figure 11B** shows tumor-reactive IFN-γ-secreting cell frequencies in the presence of anti-PD-L1 or control IgG (10 µg/ml). The graphs are representative of two independent experiments in which the CD8⁺ T cells for each group were pooled from five to seven individual mice. P values were determined by the Student t test. * p<0.05, ** p<0.01, *** p<0.001.
**Figure 12** shows levels of cytokine production by CD4⁺ T cells isolated from spleens of myeloma bearing mice treated with combinations of immune checkpoint protein blockade. The graphs are representative of two independent experiments in which the CD4⁺ T cells for each group were pooled from 5 individual mice. *p < 0.05, **p < 0.01 as compared with T cells from mice treated with anti-PD-L1 alone.
**Figure 13** shows expression of immune checkpoint proteins PD-1, Tim-3, Lag-3 and 2B4 on CD4⁺ and CD8⁺ T cells in mice with other hematologic cancers.
**Figure 14** shows a working model of combined checkpoint blockade and lymphodepleting whole body irradiation.

### Detailed Description of the Invention

Methods are provided for treating a hematologic cancer, for example, *in vitro, ex vivo,* or *in vivo* in a subject, comprising contacting a cancerous cell or administering to a subject a therapeutically effective amount of at least one agent that selectively inhibits or blocks PD-1 or PD-L1 and TIM-3, LAG-3 or CTLA4. In some embodiments, the methods involve a combination of an inhibitor of PD-1 or PD-L1 and an inhibitor of an immune checkpoint inhibitor (*e.g*., an inhibitor of one or more of TIM-3, LAG-3 or CTLA4). In one embodiment, an inhibitor of PD-1 or PD-L1 is administered in combination with an inhibitor of TIM-3. In another embodiment, an inhibitor of PD-1 or PD-L1 is administered in combination with an inhibitor of LAG-3. In yet another embodiment, an inhibitor of PD-1 or PD-L1 is administered in combination with an inhibitor of CTLA-4. Exemplary inhibitors include a bispecific antibody, multispecific antibody, or combination of individual antibodies that inhibit or block an activity of PD-1 or PD-L1 and another immune checkpoint inhibitor. Such combinations can provide therapeutic benefit for treating hematologic cancers. Such discoveries are especially surprising and unexpected given reports regarding the lack of activities reported for immune checkpoint regulators, such as TIM-3, in human T cell activation (see, for example, Leitner et al. (2013) PLoSPathog. 9:e1003253).

It will be appreciated that the methods and compositions described herein may be combined with other treatment regimens and/or other predictive biomarkers and methods of using same. It will also be appreciated that the present invention is not limited to the particular embodiments described herein , but can be carried out in variations well known to the skilled artisan.

### I. Definitions

The articles "a" and "an" are used herein to refer to one or to more than one (*i.e.* to at least one) of the grammatical object of the article. By way of example, "an element" means one element or more than one element.

The "amount" of a marker, *e.g*., expression or copy number of a marker, or protein level of a marker, in a subject is "significantly" higher or lower than the normal amount of a marker, if the amount of the marker is greater or less, respectively, than the normal level by an amount greater than the standard error of the assay employed to assess amount, and preferably at least twice, and more preferably three, four, five, ten or more times that amount. Alternately, the amount of the marker in the subject can be considered "significantly" higher or lower than the normal amount if the amount is at least about two, and preferably at least about three, four, or five times, higher or lower, respectively, than the normal amount of the marker.

The term "altered level of expression" of a marker refers to an expression level or copy number of a marker in a test sample *e.g.,* a sample derived from a subject suffering from cancer, that is greater or less than the standard error of the assay employed to assess expression or copy number, and is preferably at least twice, and more preferably three, four, five or ten or more times the expression level or copy number of the marker or chromosomal region in a control sample (*e.g*., sample from a healthy subject not having the associated disease) and preferably, the average expression level or copy number of the marker or chromosomal region in several control samples. The altered level of expression is greater or less than the standard error of the assay employed to assess expression or copy number, and is preferably at least twice, and more preferably three, four, five or ten or more times the expression level or copy number of the marker in a control sample (*e.g*., sample from a healthy subject not having the associated disease) and preferably, the average expression level or copy number of the marker in several control samples.

The term "altered activity" of a marker refers to an activity of a marker which is increased or decreased in a disease state, *e.g.,* in a cancer sample, as compared to the activity of the marker in a normal, control sample. Altered activity of a marker may be the result of, for example, altered expression of the marker, altered protein level of the marker, altered structure of the marker, or, *e.g.,* an altered interaction with other proteins involved in the same or different pathway as the marker, or altered interaction with transcriptional activators or inhibitors.

Unless otherwise specified herein, the terms "antibody" and "antibodies" broadly encompass naturally-occurring forms of antibodies (*e.g*. IgG, IgA, IgM, IgE) and recombinant antibodies such as single-chain antibodies, chimeric and humanized antibodies and multi-specific antibodies, as well as fragments and derivatives of all of the foregoing, which fragments and derivatives have at least an antigenic binding site. Antibody derivatives may comprise a protein or chemical moiety conjugated to an antibody.

The term "antibody" as used herein also includes an "antigen-binding portion" of an antibody (or simply "antibody portion"). The term "antigen-binding portion", as used herein, refers to one or more fragments of an antibody that retain the ability to specifically bind to an antigen (*e.g*., polypeptide or fragment thereof of PD-1, PD-L1, LAG-3, CTLA-4 and/or TIM-3). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent polypeptides (known as single chain Fv (scFv); see *e.g.,* Bird et al. (1988) Science 242:423-426; and Huston et al. (1988) Proc. Natl. Acad. Sci. USA 85:5879-5883; and Osbourn et al. 1998, Nature Biotechnology 16: 778). Such single chain antibodies are also intended to be encompassed within the term "antigen-binding portion" of an antibody. Any VH and VL sequences of specific scFv can be linked to human immunoglobulin constant region cDNA or genomic sequences, in order to generate expression vectors encoding complete IgG polypeptides or other isotypes. VH and VL can also be used in the generation of Fab, Fv or other fragments of immunoglobulins using either protein chemistry or recombinant DNA technology. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R. J., et al. (1994) Structure 2:1121-1123).

Still further, an antibody or antigen-binding portion thereof may be part of larger immunoadhesion polypeptides, formed by covalent or noncovalent association of the antibody or antibody portion with one or more other proteins or peptides. Examples of such immunoadhesion polypeptides include use of the streptavidin core region to make a tetrameric scFv polypeptide (Kipriyanov, S.M., et al. (1995) Human Antibodies and Hybridomas 6:93-101) and use of a cysteine residue, a marker peptide and a C-terminal polyhistidine tag to make bivalent and biotinylated scFv polypeptides (Kipriyanov, S.M., et al. (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion polypeptides can be obtained using standard recombinant DNA techniques, as described herein.

Antibodies may be polyclonal or monoclonal; xenogeneic, allogeneic, or syngeneic; or modified forms thereof (*e.g.*, humanized, chimeric, etc.). Antibodies may also be fully human. Preferably, antibodies of the invention bind specifically or substantially specifically to PD-1, PD-L1, LAG-3, CTLA-4 and/or TIM-3 polypeptides or fragments thereof. They may also be selective for such antigens such that they can distinguish such antigens from closely related antigens, such as other B7 family members. The terms "monoclonal antibodies" and "monoclonal antibody composition", as used herein, refer to a population of antibody polypeptides that contain only one species of an antigen binding site capable of immunoreacting with a particular epitope of an antigen, whereas the term "polyclonal antibodies" and "polyclonal antibody composition" refer to a population of antibody polypeptides that contain multiple species of antigen binding sites capable of interacting with a particular antigen. A monoclonal antibody composition typically displays a single binding affinity for a particular antigen with which it immunoreacts.

As used herein, a "blocking" antibody or an antibody "antagonist" or "inhibitor" is one which inhibits or reduces at least one biological activity of the antigen(s) it binds. For example, an anti-PD-L1 or anti-TIM-3 antibody binds PD-L1 or TIM-3, respectively, and inhibits the ability of PD-L1 to, for example, bind PD-1, and inhibits the ability of TIM-3 to, for example, bind galectin-9 or phosphatidylserine. In certain embodiments, the blocking antibodies or antagonist antibodies or fragments thereof described herein substantially or completely inhibit a given biological activity of the antigen(s).

The term "body fluid" refers to fluids that are excreted or secreted from the body as well as fluid that are normally not (*e.g*. amniotic fluid, aqueous humor, bile, blood and blood plasma, cerebrospinal fluid, cerumen and earwax, cowper's fluid or pre-ejaculatory fluid, chyle, chyme, stool, female ejaculate, interstitial fluid, intracellular fluid, lymph, menses, breast milk, mucus, pleural fluid, pus, saliva, sebum, semen, serum, sweat, synovial fluid, tears, urine, vaginal lubrication, vitreous humor, vomit).

The term "bispecific antibody" or "multispecific antibody" refers to an antibody that recognized more than one epitope. Such antibodies are useful for targeting different proteins using the same agent. Methods of making such antibodies are well known in art (see, at least U.S. Patent 5,798,229; U.S. Patent 5,989,830; and Holliger et al. (2005) Nat. Biotech. 23:1126-1136).

The terms "cancer" or "tumor" or "hyperproliferative disorder" refer to the presence of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within an animal, or may be a non-tumorigenic cancer cell, such as a leukemia cell. Cancers include, but are not limited to, B cell cancer, *e.g*., multiple myeloma, Waldenstrom's macroglobulinemia, the heavy chain diseases, such as, for example, alpha chain disease, gamma chain disease, and mu chain disease, benign monoclonal gammopathy, and immunocytic amyloidosis, melanomas, breast cancer, lung cancer, bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematologic tissues, and the like. Other non-limiting examples of types of cancers applicable to the methods encompassed by the present invention include human sarcomas and carcinomas, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, colorectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, liver cancer, choriocarcinoma, seminoma, embryonal carcinoma, Wilms' tumor, cervical cancer, bone cancer, brain tumor, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, *e.g*., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease. In some embodiments, cancers are epithlelial in nature and include but are not limited to, bladder cancer, breast cancer, cervical cancer, colon cancer, gynecologic cancers, renal cancer, laryngeal cancer, lung cancer, oral cancer, head and neck cancer, ovarian cancer, pancreatic cancer, prostate cancer, or skin cancer. In other embodiments, the cancer is breast cancer, prostate cancer, lung cancer, or colon cancer. In still other embodiments, the epithelial cancer is non-small-cell lung cancer, nonpapillary renal cell carcinoma, cervical carcinoma, ovarian carcinoma (*e.g*., serous ovarian carcinoma), or breast carcinoma. The epithelial cancers may be characterized in various other ways including, but not limited to, serous, endometrioid, mucinous, clear cell, Brenner, or undifferentiated.

The term "control" refers to any reference standard suitable to provide a comparison to the expression products in the test sample. In one embodiment, the control comprises obtaining a "control sample" from which expression product levels are detected and compared to the expression product levels from the test sample. Such a control sample may comprise any suitable sample, including but not limited to a sample from a control cancer patient (can be stored sample or previous sample measurement) with a known outcome; normal tissue or cells isolated from a subject, such as a normal patient or the cancer patient, cultured primary cells/tissues isolated from a subject such as a normal subject or the cancer patient, adjacent normal cells/tissues obtained from the same organ or body location of the cancer patient, a tissue or cell sample isolated from a normal subject, or a primary cells/tissues obtained from a depository. In another preferred embodiment, the control may comprise a reference standard expression product level from any suitable source, including but not limited to housekeeping genes, an expression product level range from normal tissue (or other previously analyzed control sample), a previously determined expression product level range within a test sample from a group of patients, or a set of patients with a certain outcome (for example, survival for one, two, three, four years, etc.) or receiving a certain treatment (for example, standard of care cancer therapy). It will be understood by those of skill in the art that such control samples and reference standard expression product levels can be used in combination as controls in the methods of the present invention. In one embodiment, the control may comprise normal or non-cancerous cell/tissue sample. In another preferred embodiment, the control may comprise an expression level for a set of patients, such as a set of cancer patients, or for a set of cancer patients receiving a certain treatment, or for a set of patients with one outcome versus another outcome. In the former case, the specific expression product level of each patient can be assigned to a percentile level of expression, or expressed as either higher or lower than the mean or average of the reference standard expression level. In another preferred embodiment, the control may comprise normal cells, cells from patients treated with combination chemotherapy, and cells from patients having benign cancer. In another embodiment, the control may also comprise a measured value for example, average level of expression of a particular gene in a population compared to the level of expression of a housekeeping gene in the same population. Such a population may comprise normal subjects, cancer patients who have not undergone any treatment (*i.e.,* treatment naive), cancer patients undergoing standard of care therapy, or patients having benign cancer. In another preferred embodiment, the control comprises a ratio transformation of expression product levels, including but not limited to determining a ratio of expression product levels of two genes in the test sample and comparing it to any suitable ratio of the same two genes in a reference standard; determining expression product levels of the two or more genes in the test sample and determining a difference in expression product levels in any suitable control; and determining expression product levels of the two or more genes in the test sample, normalizing their expression to expression of housekeeping genes in the test sample, and comparing to any suitable control. In particularly preferred embodiments, the control comprises a control sample which is of the same lineage and/or type as the test sample. In another embodiment, the control may comprise expression product levels grouped as percentiles within or based on a set of patient samples, such as all patients with cancer. In one embodiment a control expression product level is established wherein higher or lower levels of expression product relative to, for instance, a particular percentile, are used as the basis for predicting outcome. In another preferred embodiment, a control expression product level is established using expression product levels from cancer control patients with a known outcome, and the expression product levels from the test sample are compared to the control expression product level as the basis for predicting outcome. As demonstrated by the data below, the methods of the invention are not limited to use of a specific cut-point in comparing the level of expression product in the test sample to the control.

The term "hematologic cancer" refers to cancers of cells derived from the blood. In some embodiments, the hematologic cancer is selected from the group consisting of acute lymphocytic leukemia, myeloid leukemia including acute myeloid leukemia and chronic myelogenous leukemia, chronic lymphocytic leukemia, small lymphocytic lymphoma, multiple myeloma, non-Hodgkin's lymphoma, Hodgkin's lymphoma, mantle cell lymphoma, follicular lymphoma, Waldenstrom's macroglobulinemia, B-cell lymphoma and including diffuse large B-cell lymphoma (including primary mediastinal B-cell lymphoma and intravascular large B-cell lymphoma), follicular lymphoma, chronic lymphocytic leukemia, small lymphocytic lymphoma, mantle cell lymphoma, mucosa-associated lymphoid tissue (MALT) lymphomas 9e.g., extranodal marginal zone B-cell lymphoma of the MALT type, nodal marginal zone B-cell lymphoma (with or without monocytoid B cells)), marginal zone B-cell lymphomas (e.g., nodal marginal zone B-cell lymphoma and splenic marginal zone B-cell lymphoma (with or without villous lymphocytes)), Burkitt lymphoma, lymphoplasmacytic lymphoma (Waldenstrom macroglobulinemia), mediastinal large B cell lymphoma, precursor B-lymphoblastic leukemia/lymphoma and, B-cell chronic lymphocytic leukemia/small lymphocytic lymphoma, B-cell prolymphocytic leukemia, lymphoplasmacytic lymphoma, splenic marginal zone B-cell lymphoma (with or without villous lymphocytes), hairy cell leukemia, plasma cell myeloma/plasmacytoma, extranodal marginal zone B-cell lymphoma of the MALT type, nodal marginal zone B-cell lymphoma (with or without monocytoid B cells), Burkitt's lymphoma; precursor T-lymphoblastic lymphoma/leukemia, T-cell prolymphocytic leukemia, T-cell granular lymphocytic leukemia, aggressive NK cell leukemia, adult T-cell lymphoma/leukemia (HTLV 1-positive), nasal-type extranodal NK/T-cell lymphoma, enteropathy-type T-cell lymphoma, hepatosplenic γ-δ T-cell lymphoma, subcutaneous panniculitis-like T-cell lymphoma, mycosis fungoides/Sézary syndrome, anaplastic large cell lymphoma (T/null cell, primary cutaneous type), anaplastic large cell lymphoma (T-/null-cell, primary systemic type), peripheral T-cell lymphoma not otherwise characterized, angioimmunoblastic T-cell lymphoma, polycythemia vera (PV), myelodysplastic syndrome (MDS). NHL may include indolent Non-Hodgkin's Lymphoma (iNHL) or aggressive Non-Hodgkin's Lymphoma (aNHL).

As used herein, the term "coding region" refers to regions of a nucleotide sequence comprising codons which are translated into amino acid residues, whereas the term "noncoding region" refers to regions of a nucleotide sequence that are not translated into amino acids (*e.g.,* 5' and 3' untranslated regions).

As used herein, the term "complementary" refers to the broad concept of sequence complementarity between regions of two nucleic acid strands or between two regions of the same nucleic acid strand. It is known that an adenine residue of a first nucleic acid region is capable of forming specific hydrogen bonds ("base pairing") with a residue of a second nucleic acid region which is antiparallel to the first region if the residue is thymine or uracil. Similarly, it is known that a cytosine residue of a first nucleic acid strand is capable of base pairing with a residue of a second nucleic acid strand which is antiparallel to the first strand if the residue is guanine. A first region of a nucleic acid is complementary to a second region of the same or a different nucleic acid if, when the two regions are arranged in an antiparallel fashion, at least one nucleotide residue of the first region is capable of base pairing with a residue of the second region. Preferably, the first region comprises a first portion and the second region comprises a second portion, whereby, when the first and second portions are arranged in an antiparallel fashion, at least about 50%, and preferably at least about 75%, at least about 90%, or at least about 95% of the nucleotide residues of the first portion are capable of base pairing with nucleotide residues in the second portion. More preferably, all nucleotide residues of the first portion are capable of base pairing with nucleotide residues in the second portion.

As used herein, the term "determining a suitable treatment regimen for the subject" is taken to mean the determination of a treatment regimen *(i.e.,* a single therapy or a combination of different therapies that are used for the prevention and/or treatment of the cancer in the subject) for a subject that is started, modified and/or ended based or essentially based or at least partially based on the results of the analysis according to the present invention. One example is starting an adjuvant therapy after surgery whose purpose is to decrease the risk of recurrence, another would be to modify the dosage of a particular chemotherapy. The determination can, in addition to the results of the analysis according to the present invention, be based on personal characteristics of the subject to be treated. In most cases, the actual determination of the suitable treatment regimen for the subject will be performed by the attending physician or doctor.

"Homologous" as used herein, refers to nucleotide sequence similarity between two regions of the same nucleic acid strand or between regions of two different nucleic acid strands. When a nucleotide residue position in both regions is occupied by the same nucleotide residue, then the regions are homologous at that position. A first region is homologous to a second region if at least one nucleotide residue position of each region is occupied by the same residue. Homology between two regions is expressed in terms of the proportion of nucleotide residue positions of the two regions that are occupied by the same nucleotide residue. By way of example, a region having the nucleotide sequence 5'-ATTGCC-3' and a region having the nucleotide sequence 5'-TATGGC-3' share 50% homology. Preferably, the first region comprises a first portion and the second region comprises a second portion, whereby, at least about 50%, and preferably at least about 75%, at least about 90%, or at least about 95% of the nucleotide residue positions of each of the portions are occupied by the same nucleotide residue. More preferably, all nucleotide residue positions of each of the portions are occupied by the same nucleotide residue.

As used herein, the term "host cell" is intended to refer to a cell into which a nucleic acid of the invention, such as a recombinant expression vector of the invention, has been introduced. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

The term "humanized antibody," as used herein, is intended to include antibodies made by a non-human cell having variable and constant regions which have been altered to more closely resemble antibodies that would be made by a human cell. For example, by altering the non-human antibody amino acid sequence to incorporate amino acids found in human germline immunoglobulin sequences. Humanized antibodies may include amino acid residues not encoded by human germline immunoglobulin sequences (*e.g*., mutations introduced by random or site-specific mutagenesis *in vitro* or by somatic mutation *in vivo*), for example in the CDRs. The term "humanized antibody", as used herein, also includes antibodies in which CDR sequences derived from the germline of another mammalian species, such as a mouse, have been grafted onto human framework sequences.

As used herein, the term "immune cell" refers to cells that play a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes.

As used herein, the term "immune checkpoints" or "immune checkpoint regulators" means a group of molecules on the cell surface of CD4+ and CD8+ T cells. These molecules fine-tune immune responses by down-modulating or inhibiting an immune response, e.g., an anti-tumor immune response. Immune checkpoint proteins are known in the art and include, without limitation, CTLA-4, PD-1, VISTA, B7-H2, B7-H3, PD-L1, B7-H4, B7-H6, 2B4, ICOS, HVEM, PD-L2, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, LAG-3, BTLA, and A2aR (see, for example, WO 2012/177624). Immunotherapeutic agents that can act as immune checkpoint inhibitors useful in the methods of the present invention, include, but are not limited to, inhibitors of PD-1, PD-L1, TIM-3, LAG-3 and CTLA-4 (*e.g*., soluble peptide inhibitors or antibodies, *e.g*., anti-PD-1 antibodies, anti-PD-L1 antibodies, anti-CTLA-4 antibodies, anti-TIM-3 antibodies, and anti-LAG-3 antibodies).

As used herein, the term "immune response" includes T cell mediated and/or B cell mediated immune responses. Exemplary immune responses include T cell responses, *e.g*., cytokine production and cellular cytotoxicity. In addition, the term immune response includes immune responses that are indirectly effected by T cell activation, *e.g*., antibody production (humoral responses) and activation of cytokine responsive cells, *e.g*., macrophages.

As used herein, the term "immunotherapeutic agent" can include any molecule, peptide, antibody or other agent which can stimulate a host immune system to generate an immune response to a tumor or cancer in the subject. Various immunotherapeutic agents are useful in the compositions and methods described herein.

An "inducible" promoter is a nucleotide sequence which, when operably linked with a polynucleotide which encodes or specifies a gene product, causes the gene product to be produced in a living human cell substantially only when an inducer which corresponds to the promoter is present in the cell.

As used herein, the term "inhibit" refers to any decrease in, for example a particular action, function, or interaction. For example, cancer is "inhibited" if at least one symptom of the cancer is reduced, slowed, or delayed. As used herein, cancer is also "inhibited" if recurrence or metastasis of the cancer is reduced, slowed, or delayed.

As used herein, the term "interaction," when referring to an interaction between two molecules, refers to the physical contact (*e.g*., binding) of the molecules with one another. Generally, such an interaction results in an activity (which produces a biological effect) of one or both of said molecules.

An "isolated antibody" is intended to refer to an antibody that is substantially free of other antibodies having different antigenic specificities (*e.g*., an isolated antibody that specifically binds PD-L1 polypeptide or a fragment thereof, or TIM-3 polypeptide or a fragment thereof, is substantially free of antibodies that specifically bind antigens other than said polypeptide or a fragment thereof). Moreover, an isolated antibody may be substantially free of other cellular material and/or chemicals.

As used herein, an "isolated protein" refers to a protein that is substantially free of other proteins, cellular material, separation medium, and culture medium when isolated from cells or produced by recombinant DNA techniques, or chemical precursors or other chemicals when chemically synthesized. An "isolated" or "purified" protein or biologically active portion thereof is substantially free of cellular material or other contaminating proteins from the cell or tissue source from which the antibody, polypeptide, peptide or fusion protein is derived, or substantially free from chemical precursors or other chemicals when chemically synthesized. The language "substantially free of cellular material" includes preparations, in which compositions of the invention are separated from cellular components of the cells from which they are isolated or recombinantly produced. In one embodiment, the language "substantially free of cellular material" includes preparations of having less than about 30%, 20%, 10%, or 5% (by dry weight) of cellular material. When an antibody, polypeptide, peptide or fusion protein or fragment thereof, *e.g.,* a biologically active fragment thereof, is recombinantly produced, it is also preferably substantially free of culture medium, *i.e.,* culture medium represents less than about 20%, more preferably less than about 10%, and most preferably less than about 5% of the volume of the protein preparation.

A "kit" is any manufacture (*e.g*. a package or container) comprising at least one reagent, *e.g.* a probe or small molecule, for specifically detecting and/or affecting the expression of a marker of the invention. The kit may be promoted, distributed, or sold as a unit for performing the methods of the present invention. The kit may comprise one or more reagents necessary to express a composition useful in the methods of the present invention. In certain embodiments, the kit may further comprise a reference standard, *e.g.,* a nucleic acid encoding a protein that does not affect or regulate signaling pathways controlling cell growth, division, migration, survival or apoptosis. One skilled in the art can envision many such control proteins, including, but not limited to, common molecular tags (*e.g.,* green fluorescent protein and beta-galactosidase), proteins not classified in any of pathway encompassing cell growth, division, migration, survival or apoptosis by GeneOntology reference, or ubiquitous housekeeping proteins. Reagents in the kit may be provided in individual containers or as mixtures of two or more reagents in a single container. In addition, instructional materials which describe the use of the compositions within the kit can be included.

A "marker" is a gene whose altered level of expression in a tissue or cell from its expression level in normal or healthy tissue or cell is associated with a disease state, such as cancer. A "marker nucleic acid" is a nucleic acid (*e.g.,* mRNA, cDNA) encoded by or corresponding to a marker of the invention. Such marker nucleic acids include DNA (*e.g.,* cDNA) comprising the entire or a partial sequence of any of the nucleic acid sequences set forth in the Sequence Listing or the complement of such a sequence. The marker nucleic acids also include RNA comprising the entire or a partial sequence of any of the nucleic acid sequences set forth in the Sequence Listing or the complement of such a sequence, wherein all thymidine residues are replaced with uridine residues. A "marker protein" is a protein encoded by or corresponding to a marker of the invention. A marker protein comprises the entire or a partial sequence of any of the sequences set forth in the Sequence Listing. The terms "protein" and "polypeptide" are used interchangeably.

The "normal" level of expression of a marker is the level of expression of the marker in cells of a subject, *e.g.,* a human patient, not afflicted with a cancer. An "over-expression" or "significantly higher level of expression" of a marker refers to an expression level in a test sample that is greater than the standard error of the assay employed to assess expression, and is preferably at least twice, and more preferably 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher than the expression activity or level of the marker in a control sample (*e.g.,* sample from a healthy subject not having the marker associated disease) and preferably, the average expression level of the marker in several control samples. A "significantly lower level of expression" of a marker refers to an expression level in a test sample that is at least twice, and more preferably 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more lower than the expression level of the marker in a control sample (*e.g.,* sample from a healthy subject not having the marker associated disease) and preferably, the average expression level of the marker in several control samples.

An "over-expression" or "significantly higher level of expression" of a marker refers to an expression level in a test sample that is greater than the standard error of the assay employed to assess expression, and is preferably at least twice, and more preferably 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher than the expression activity or level of the marker in a control sample (*e.g.,* sample from a healthy subject not having the marker associated disease) and preferably, the average expression level of the marker in several control samples. A "significantly lower level of expression" of a marker refers to an expression level in a test sample that is at least twice, and more preferably 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more lower than the expression level of the marker in a control sample (*e.g.,* sample from a healthy subject not having the marker associated disease) and preferably, the average expression level of the marker in several control samples.

The term "peripheral blood cell subtypes" refers to cell types normally found in the peripheral blood including, but is not limited to, eosinophils, neutrophils, T cells, monocytes, NK cells, granulocytes, and B cells.

The term "probe" refers to any molecule which is capable of selectively binding to a specifically intended target molecule, for example, a nucleotide transcript or protein encoded by or corresponding to a marker. Probes can be either synthesized by one skilled in the art, or derived from appropriate biological preparations. For purposes of detection of the target molecule, probes may be specifically designed to be labeled, as described herein. Examples of molecules that can be utilized as probes include, but are not limited to, RNA, DNA, proteins, antibodies, and organic molecules.

The term "prognosis" includes a prediction of the probable course and outcome of cancer or the likelihood of recovery from the disease. In some embodiments, the use of statistical algorithms provides a prognosis of cancer in an individual. For example, the prognosis can be surgery, development of a clinical subtype of cancer (*e.g.,* hematologic cancers, such as multiple myeloma), development of one or more clinical factors, development of intestinal cancer, or recovery from the disease.

The term "response to cancer therapy" or "outcome of cancer therapy" relates to any response of the hyperproliferative disorder (*e.g.,* cancer) to a cancer therapy, preferably to a change in tumor mass and/or volume after initiation of neoadjuvant or adjuvant chemotherapy. Hyperproliferative disorder response may be assessed, for example for efficacy or in a neoadjuvant or adjuvant situation, where the size of a tumor after systemic intervention can be compared to the initial size and dimensions as measured by CT, PET, mammogram, ultrasound or palpation. Response may also be assessed by caliper measurement or pathological examination of the tumor after biopsy or surgical resection for solid cancers. Responses may be recorded in a quantitative fashion like percentage change in tumor volume or in a qualitative fashion like "pathological complete response" (pCR), "clinical complete remission" (cCR), "clinical partial remission" (cPR), "clinical stable disease" (cSD), "clinical progressive disease" (cPD) or other qualitative criteria. Assessment of hyperproliferative disorder response may be done early after the onset of neoadjuvant or adjuvant therapy, *e.g.,* after a few hours, days, weeks or preferably after a few months. A typical endpoint for response assessment is upon termination of neoadjuvant chemotherapy or upon surgical removal of residual tumor cells and/or the tumor bed. This is typically three months after initiation of neoadjuvant therapy. In some embodiments, clinical efficacy of the therapeutic treatments described herein may be determined by measuring the clinical benefit rate (CBR). The clinical benefit rate is measured by determining the sum of the percentage of patients who are in complete remission (CR), the number of patients who are in partial remission (PR) and the number of patients having stable disease (SD) at a time point at least 6 months out from the end of therapy. The shorthand for this formula is CBR=CR+PR+SD over 6 months. In some embodiments, the CBR for a particular cancer therapeutic regimen is at least 25%, 30%, 35%, 40%, 45%, 50%, 55%, 60%, 65%, 70%, 75%, 80%, 85%, or more. In other embodiments, the percentage of patients who are in either CR, PR, and/or SD in any combination at least 30 days, 2 months, 3 months, 4 months, 5 months, 6 months, 7 months, 8 months, 9 months, 10 months, 11 months, 12 months, 18 months, 24 months, 30 months, 36 months, 60 months, or longer is at least 25%, 30%, 35%, 40%, 45%, 50%, 55%, 60%, 65%, 70%, 75%, 80%, 85%, or more. In some embodiments, the percentage is 100% over such a time period. Additional criteria for evaluating the response to cancer therapies are related to "survival," which includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.,* time of diagnosis or start of treatment) and end point (*e.g.,* death, recurrence or metastasis). In addition, criteria for efficacy of treatment can be expanded to include response to chemotherapy, probability of survival, probability of metastasis within a given time period, and probability of tumor recurrence. For example, in order to determine appropriate threshold values, a particular cancer therapeutic regimen can be administered to a population of subjects and the outcome can be correlated to copy number, level of expression, level of activity, etc. of a marker determined prior to administration of any cancer therapy. The outcome measurement may be pathologic response to therapy given in the neoadjuvant setting. Alternatively, outcome measures, such as overall survival and disease-free survival can be monitored over a period of time for subjects following cancer therapy for whom the measurement values are known. In certain embodiments, the same doses of cancer therapeutic agents are administered to each subject. In related embodiments, the doses administered are standard doses known in the art for cancer therapeutic agents. The period of time for which subjects are monitored can vary. For example, subjects may be monitored for at least 2, 4, 6, 8, 10, 12, 14, 16, 18, 20, 25, 30, 35, 40, 45, 50, 55, or 60 months. Biomarker threshold values that correlate to outcome of a cancer therapy can be determined using methods such as those described in the Examples section. Outcomes can also be measured in terms of a "hazard ratio" (the ratio of death rates for one patient group to another; provides likelihood of death at a certain time point), "overall survival" (OS), and/or "progression free survival." In certain embodiments, the prognosis comprises likelihood of overall survival rate at 1 year, 2 years, 3 years, 4 years, or any other suitable time point. The significance associated with the prognosis of poor outcome in all aspects of the present invention is measured by techniques known in the art. For example, significance may be measured with calculation of odds ratio. In a further embodiment, the significance is measured by a percentage. In one embodiment, a significant risk of poor outcome is measured as odds ratio of 0.8 or less or at least about 1.2, including by not limited to: 0.1, 0.2, 0.3, 0.4, 0.5, 0.6, 0.7, 0.8, 1.2, 1.3, 1.4, 1.5, 1.6, 1.7, 1.8, 1.9, 2.0, 2.5, 3.0, 4.0, 5.0, 10.0, 15.0, 20.0, 25.0, 30.0 and 40.0. In a further embodiment, a significant increase or reduction in risk is at least about 20%, including but not limited to about 25%, 30%, 35%, 40%, 45%, 50%, 55%, 60%, 65%, 70%, 75%, 80%, 85%, 90%, 95% and 98%. In a further embodiment, a significant increase in risk is at least about 50%. Thus, the present invention further provides methods for making a treatment decision for a cancer patient, comprising carrying out the methods for prognosing a cancer patient according to the different aspects and embodiments of the present invention, and then weighing the results in light of other known clinical and pathological risk factors, in determining a course of treatment for the cancer patient. For example, a cancer patient that is shown by the methods of the invention to have an increased risk of poor outcome by combination chemotherapy treatment can be treated with more aggressive therapies, including but not limited to radiation therapy, peripheral blood stem cell transplant, bone marrow transplant, or novel or experimental therapies under clinical investigation.

The term "resistance" refers to an acquired or natural resistance of a cancer sample or a mammal to a cancer therapy (*i.e.,* being nonresponsive to or having reduced or limited response to the therapeutic treatment), such as having a reduced response to a therapeutic treatment by 25% or more, for example, 30%, 40%, 50%, 60%, 70%, 80%, or more, to 2-fold, 3-fold, 4-fold, 5-fold, 10-fold, 15-fold, 20-fold or more. The reduction in response can be measured by comparing with the same cancer sample or mammal before the resistance is acquired, or by comparing with a different cancer sample or a mammal who is known to have no resistance to the therapeutic treatment. A typical acquired resistance to chemotherapy is called "multidrug resistance." The multidrug resistance can be mediated by P-glycoprotein or can be mediated by other mechanisms, or it can occur when a mammal is infected with a multi drug-resistant microorganism or a combination of microorganisms. The determination of resistance to a therapeutic treatment is routine in the art and within the skill of an ordinarily skilled clinician, for example, can be measured by cell proliferative assays and cell death assays as described herein as "sensitizing." In some embodiments, the term "reverses resistance" means that the use of a second agent in combination with a primary cancer therapy (*e.g*., chemotherapeutic or radiation therapy) is able to produce a significant decrease in tumor volume at a level of statistical significance *(e.g.,* p<0.05) when compared to tumor volume of untreated tumor in the circumstance where the primary cancer therapy (*e.g*., chemotherapeutic or radiation therapy) alone is unable to produce a statistically significant decrease in tumor volume compared to tumor volume of untreated tumor. This generally applies to tumor volume measurements made at a time when the untreated tumor is growing log rhythmically.

An "RNA interfering agent" as used herein, is defined as any agent which interferes with or inhibits expression of a target gene, *e.g.,* a marker of the invention, by RNA interference (RNAi). Such RNA interfering agents include, but are not limited to, nucleic acid molecules including RNA molecules which are homologous to the target gene, *e.g.,* a marker of the invention, or a fragment thereof, short interfering RNA (siRNA), and small molecules which interfere with or inhibit expression of a target gene by RNA interference (RNAi).

"RNA interference (RNAi)" is an evolutionally conserved process whereby the expression or introduction of RNA of a sequence that is identical or highly similar to a target gene results in the sequence specific degradation or specific post-transcriptional gene silencing (PTGS) of messenger RNA (mRNA) transcribed from that targeted gene (*see* Coburn and Cullen (2002) J. Virol. 76(18): 9225), thereby inhibiting expression of the target gene. In one embodiment, the RNA is double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex that recognizes and cleaves target mRNAs. RNAi can also be initiated by introducing nucleic acid molecules, e.g., synthetic siRNAs or RNA interfering agents, to inhibit or silence the expression of target genes. As used herein, "inhibition of target gene expression" or "inhibition of marker gene expression" includes any decrease in expression or protein activity or level of the target gene *(e.g.,* a marker gene of the invention) or protein encoded by the target gene, *e.g.,* a marker protein of the invention. The decrease may be of at least 30%, 40%, 50%, 60%, 70%, 80%, 90%, 95% or 99% or more as compared to the expression of a target gene or the activity or level of the protein encoded by a target gene which has not been targeted by an RNA interfering agent.

The term "sample" used for detecting or determining the presence or level of at least one biomarker is typically whole blood, plasma, serum, saliva, urine, stool *(e.g.,* feces), tears, and any other bodily fluid *(e.g.,* as described above under the definition of "body fluids"), or a tissue sample (*e.g.,* biopsy) such as a small intestine, colon sample, or surgical resection tissue. In certain instances, the method of the present invention further comprises obtaining the sample from the individual prior to detecting or determining the presence or level of at least one marker in the sample.

The term "sensitize" means to alter cancer cells or tumor cells in a way that allows for more effective treatment of the associated cancer with a cancer therapy (*e.g*., chemotherapeutic or radiation therapy. In some embodiments, normal cells are not affected to an extent that causes the normal cells to be unduly injured by the cancer therapy (e.g., chemotherapy or radiation therapy). An increased sensitivity or a reduced sensitivity to a therapeutic treatment is measured according to a known method in the art for the particular treatment and methods described herein below, including, but not limited to, cell proliferative assays (Tanigawa N, Kern D H, Kikasa Y, Morton D L, Cancer Res 1982; 42: 2159-2164), cell death assays (Weisenthal L M, Shoemaker R H, Marsden J A, Dill P L, Baker J A, Moran E M, Cancer Res 1984; 94: 161-173; Weisenthal L M, Lippman M E, Cancer Treat Rep 1985; 69: 615-632; Weisenthal L M, In: Kaspers G J L, Pieters R, Twentyman P R, Weisenthal L M, Veerman A J P, eds. Drug Resistance in Leukemia and Lymphoma. Langhorne, P A: Harwood Academic Publishers, 1993: 415-432; Weisenthal L M, Contrib Gynecol Obstet 1994; 19: 82-90). The sensitivity or resistance may also be measured in animal by measuring the tumor size reduction over a period of time, for example, 6 month for human and 4-6 weeks for mouse. A composition or a method sensitizes response to a therapeutic treatment if the increase in treatment sensitivity or the reduction in resistance is 25% or more, for example, 30%, 40%, 50%, 60%, 70%, 80%, or more, to 2-fold, 3-fold, 4-fold, 5-fold, 10-fold, 15-fold, 20-fold or more, compared to treatment sensitivity or resistance in the absence of such composition or method. The determination of sensitivity or resistance to a therapeutic treatment is routine in the art and within the skill of an ordinarily skilled clinician. It is to be understood that any method described herein for enhancing the efficacy of a cancer therapy can be equally applied to methods for sensitizing hyperproliferative or otherwise cancerous cells (*e.g*., resistant cells) to the cancer therapy.

"Short interfering RNA" (siRNA), also referred to herein as "small interfering RNA" is defined as an agent which functions to inhibit expression of a target gene, *e.g.,* by RNAi. An siRNA may be chemically synthesized, may be produced by *in vitro* transcription, or may be produced within a host cell. In one embodiment, siRNA is a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, or 22 nucleotides in length, and may contain a 3' and/or 5' overhang on each strand having a length of about 0, 1, 2, 3, 4, or 5 nucleotides. The length of the overhang is independent between the two strands, *i.e.,* the length of the over hang on one strand is not dependent on the length of the overhang on the second strand. Preferably the siRNA is capable of promoting RNA interference through degradation or specific post-transcriptional gene silencing (PTGS) of the target messenger RNA (mRNA). In another embodiment, an siRNA is a small hairpin (also called stem loop) RNA (shRNA). In one embodiment, these shRNAs are composed of a short (*e.g.,* 19-25 nucleotide) antisense strand, followed by a 5-9 nucleotide loop, and the analogous sense strand. Alternatively, the sense strand may precede the nucleotide loop structure and the antisense strand may follow. These shRNAs may be contained in plasmids, retroviruses, and lentiviruses and expressed from, for example, the pol III U6 promoter, or another promoter (*see, e.g.,* Stewart, et al. (2003) RNA Apr; 9(4):493-501 incorporated by reference herein). RNA interfering agents, e.g., siRNA molecules, may be administered to a subject having or at risk for having cancer, to inhibit expression of a marker gene of the invention, e.g., a marker gene which is overexpressed in cancer (such as the markers listed in Table 3) and thereby treat, prevent, or inhibit cancer in the subject.

As used herein, "subject" refers to any healthy animal, mammal or human, or any animal, mammal or human afflicted with a cancer, *e.g.,* lung, ovarian, pancreatic, liver, breast, prostate, and colon carcinomas, as well as melanoma and multiple myeloma. The term "subject" is interchangeable with "subject".

The language "substantially free of chemical precursors or other chemicals" includes preparations of antibody, polypeptide, peptide or fusion protein in which the protein is separated from chemical precursors or other chemicals which are involved in the synthesis of the protein. In one embodiment, the language "substantially free of chemical precursors or other chemicals" includes preparations of antibody, polypeptide, peptide or fusion protein having less than about 30% (by dry weight) of chemical precursors or non-antibody, polypeptide, peptide or fusion protein chemicals, more preferably less than about 20% chemical precursors or non-antibody, polypeptide, peptide or fusion protein chemicals, still more preferably less than about 10% chemical precursors or non-antibody, polypeptide, peptide or fusion protein chemicals, and most preferably less than about 5% chemical precursors or non- antibody, polypeptide, peptide or fusion protein chemicals.

As used herein, the term "survival" includes all of the following: survival until mortality, also known as overall survival (wherein said mortality may be either irrespective of cause or tumor related); "recurrence-free survival" (wherein the term recurrence shall include both localized and distant recurrence); metastasis free survival; disease free survival (wherein the term disease shall include cancer and diseases associated therewith). The length of said survival may be calculated by reference to a defined start point (*e.g.* time of diagnosis or start of treatment) and end point (*e.g.* death, recurrence or metastasis). In addition, criteria for efficacy of treatment can be expanded to include response to chemotherapy, probability of survival, probability of metastasis within a given time period, and probability of tumor recurrence.

The term "synergistic effect" refers to the combined effect of two or more anticancer agents can be greater than the sum of the separate effects of the anticancer agents or alone. In some embodiments, in can provide for similar efficacy of monotherapy but with other unexpected improvements relative to monotherapy, such as reducing unwanted side effects.

A "tissue-specific" promoter is a nucleotide sequence which, when operably linked with a polynucleotide which encodes or specifies a gene product, causes the gene product to be produced in a living human cell substantially only if the cell is a cell of the tissue type corresponding to the promoter.

A "transcribed polynucleotide" or "nucleotide transcript" is a polynucleotide (e.g. an mRNA, hnRNA, a cDNA, or an analog of such RNA or cDNA) which is complementary to or homologous with all or a portion of a mature mRNA made by transcription of a marker of the invention and normal post-transcriptional processing (*e.g.* splicing), if any, of the RNA transcript, and reverse transcription of the RNA transcript.

An "underexpression" or "significantly lower level of expression or copy number" of a marker refers to an expression level or copy number in a test sample that is greater than the standard error of the assay employed to assess expression or copy number, but is preferably at least twice, and more preferably three, four, five or ten or more times less than the expression level or copy number of the marker in a control sample (*e.g.,* sample from a healthy subject not afflicted with cancer) and preferably, the average expression level or copy number of the marker in several control samples.

As used herein, the term "vector" refers to a nucleic acid capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.,* bacterial vectors having a bacterial origin of replication and episomal mammalian vectors). Other vectors (*e.g.,* non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (*e.g.,* replication defective retroviruses, adenoviruses and adeno-associated viruses), which serve equivalent functions.

There is a known and definite correspondence between the amino acid sequence of a particular protein and the nucleotide sequences that can code for the protein, as defined by the genetic code (shown below). Likewise, there is a known and definite correspondence between the nucleotide sequence of a particular nucleic acid and the amino acid sequence encoded by that nucleic acid, as defined by the genetic code.

**GENETIC CODE**

| | |
|---|---|
| Alanine (Ala, A) | GCA, GCC, GCG, GCT |
| Arginine (Arg, R) | AGA, ACG, CGA, CGC, CGG, CGT |
| Asparagine (Asn, N) | AAC, AAT |
| Aspartic acid (Asp, D) | GAC, GAT |
| Cysteine (Cys, C) | TGC, TGT |
| Glutamic acid (Glu, E) | GAA, GAG |
| Glutamine (Gln, Q) | CAA, CAG |
| Glycine (Gly, G) | GGA, GGC, GGG, GGT |
| Histidine (His, H) | CAC, CAT |
| Isoleucine (Ile, I) | ATA, ATC, ATT |
| Leucine (Leu, L) | CTA, CTC, CTG, CTT, TTA, TTG |
| Lysine (Lys, K) | AAA, AAG |
| Methionine (Met, M) | ATG |
| Phenylalanine (Phe, F) | TTC, TTT |
| Proline (Pro, P) | CCA, CCC, CCG, CCT |
| Serine (Ser, S) | AGC, AGT, TCA, TCC, TCG, TCT |
| Threonine (Thr, T) | ACA, ACC, ACG, ACT |
| Tryptophan (Trp, W) | TGG |
| Tyrosine (Tyr, Y) | TAC, TAT |
| Valine (Val, V) | GTA, GTC, GTG, GTT |
| Termination signal (end) | TAA, TAG, TGA |

An important and well known feature of the genetic code is its redundancy, whereby, for most of the amino acids used to make proteins, more than one coding nucleotide triplet may be employed (illustrated above). Therefore, a number of different nucleotide sequences may code for a given amino acid sequence. Such nucleotide sequences are considered functionally equivalent since they result in the production of the same amino acid sequence in all organisms (although certain organisms may translate some sequences more efficiently than they do others). Moreover, occasionally, a methylated variant of a purine or pyrimidine may be found in a given nucleotide sequence. Such methylations do not affect the coding relationship between the trinucleotide codon and the corresponding amino acid.

In view of the foregoing, the nucleotide sequence of a DNA or RNA coding for a fusion protein or polypeptide of the invention (or any portion thereof) can be used to derive the fusion protein or polypeptide amino acid sequence, using the genetic code to translate the DNA or RNA into an amino acid sequence. Likewise, for fusion protein or polypeptide amino acid sequence, corresponding nucleotide sequences that can encode the fusion protein or polypeptide can be deduced from the genetic code (which, because of its redundancy, will produce multiple nucleic acid sequences for any given amino acid sequence). Thus, description and/or disclosure herein of a nucleotide sequence which encodes a fusion protein or polypeptide should be considered to also include description and/or disclosure of the amino acid sequence encoded by the nucleotide sequence. Similarly, description and/or disclosure of a fusion protein or polypeptide amino acid sequence herein should be considered to also include description and/or disclosure of all possible nucleotide sequences that can encode the amino acid sequence.

Finally, nucleic acid and amino acid sequence information for the loci and biomarkers of the present invention (*e.g.,* biomarkers listed in Table 1) are well known in the art and readily available on publicly available databases, such as the National Center for Biotechnology Information (NCBI). For example, exemplary nucleic acid and amino acid sequences derived from publicly available sequence databases are provided below.

For example, the term "PD-1" refers to a member of the immunoglobulin gene superfamily that functions as a coinhibitory receptor having PD-L1 and PD-L2 as known ligands. PD-1 was previously identified using a subtraction cloning based approach to select for proteins involved in apoptotic cell death. PD-1 is a member of the CD28/CTLA-4 family of molecules based on its ability to bind to PD-L1. Like CTLA-4, PD-1 is rapidly induced on the surface of T-cells in response to anti-CD3 (Agata et al. 25 (1996) Int. Immunol. 8:765). In contrast to CTLA-4, however, PD-1 is also induced on the surface of B-cells (in response to anti-IgM). PD-1 is also expressed on a subset of thymocytes and myeloid cells (Agata *et al.* (1996) *supra;* Nishimura et al. (1996) Int. Immunol. 8:773).

The nucleic acid and amino acid sequences of a representative human PD-1 biomarker is available to the public at the GenBank database under NM_005018.2 and NP_005009.2 and is shown in Table 1 (see also Ishida et al. (1992) 20 EMBO J 11:3887; Shinohara et al. (1994) Genomics 23:704; U.S. Patent 5,698,520). PD-1 has an extracellular region containing immunoglobulin superfamily domain, a transmembrane domain, and an intracellular region including an immunoreceptor tyrosine-based inhibitory motif (ITIM) (Ishida et al. (1992) EMBO J. 11:3887; Shinohara et al. (1994) Genomics 23:704; and U.S. Patent 5,698,520). These features also define a larger family of polypeptides, called the immunoinhibitory receptors, which also includes gp49B, PIR-B, and the killer inhibitory receptors (KIRs) (Vivier and Daeron (1997) Immunol. Today 18:286). It is often assumed that the tyrosyl phosphorylated ITIM motif of these receptors interacts with SH2-domain containing phosphatases, which leads to inhibitory signals. A subset of these immunoinhibitory receptors bind to MHC polypeptides, for example the KIRs, and CTLA4 binds to B7-1 and B7-2. It has been proposed that there is a phylogenetic relationship between the MHC and B7 genes (Henry et al. (1999) Immunol. Today 20(6):285-8). Nucleic acid and polypeptide sequences of PD-1 orthologs in organisms other than humans are well known and include, for example, mouse PD-1 (NM_008798.2 and NP_032824.1), rat PD-1 (NM_001106927.1 and NP_001100397.1), dog PD-1 (XM_543338.3 and XP_543338.3), cow PD-1 (NM_001083506.1 and NP_001076975.1), and chicken PD-1 (XM_422723.3 and XP_422723.2).

PD-1 polypeptides are inhibitory receptors capable of transmitting an inhibitory signal to an immune cell to thereby inhibit immune cell effector function, or are capable of promoting costimulation *(e.g.,* by competitive inhibition) of immune cells, *e.g.,* when present in soluble, monomeric form. Preferred PD-1 family members share sequence identity with PD-1 and bind to one or more B7 family members, *e.g.,* B7-1, B7-2, PD-1 ligand, and/or other polypeptides on antigen presenting cells.

The term "PD-1 activity," includes the ability of a PD-1 polypeptide to modulate an inhibitory signal in an activated immune cell, *e.g.,* by engaging a natural PD-1 ligand on an antigen presenting cell. PD-1 transmits an inhibitory signal to an immune cell in a manner similar to CTLA4. Modulation of an inhibitory signal in an immune cell results in modulation of proliferation of, and/or cytokine secretion by, an immune cell. Thus, the term "PD-1 activity" includes the ability of a PD-1 polypeptide to bind its natural ligand(s), the ability to modulate immune cell costimulatory or inhibitory signals, and the ability to modulate the immune response.

The term "PD-1 ligand" refers to binding partners of the PD-1 receptor and includes both PD-L1 (Freeman et al. (2000) J. Exp. Med. 192:1027) and PD-L2 (Latchman et al. (2001) Nat. Immunol. 2:261). At least two types of human PD-1 ligand polypeptides exist. PD-1 ligand proteins comprise a signal sequence, and an IgV domain, an IgC domain, a transmembrane domain, and a short cytoplasmic tail. Both PD-L1 (See Freeman et al. (2000) J. Exp. Med. 192:1027 for sequence data) and PD-L2 (See Latchman et al. (2001) Nat. Immunol. 2:261 for sequence data) are members of the B7 family of polypeptides. Both PD-L1 and PD-L2 are expressed in placenta, spleen, lymph nodes, thymus, and heart. Only PD-L2 is expressed in pancreas, lung and liver, while only PD-L1 is expressed in fetal liver. Both PD-1 ligands are upregulated on activated monocytes and dendritic cells, although PD-L1 expression is broader. For example, PD-L1 is known to be constitutively expressed and upregulated to higher levels on murine hematopoietic cells (*e.g.,* T cells, B cells, macrophages, dendritic cells (DCs), and bone marrow-derived mast cells) and non-hematopoietic cells (e.g., endothelial, epithelial, and muscle cells), whereas PD-L2 is inducibly expressed on DCs, macrophages, and bone marrow-derived mast cells (see, Butte et al. (2007) Immunity 27:111).

PD-1 ligands comprise a family of polypeptides having certain conserved structural and functional features. The term "family" when used to refer to proteins or nucleic acid molecules, is intended to mean two or more proteins or nucleic acid molecules having a common structural domain or motif and having sufficient amino acid or nucleotide sequence homology, as defined herein. Such family members can be naturally or non-naturally occurring and can be from either the same or different species. For example, a family can contain a first protein of human origin, as well as other, distinct proteins of human origin or alternatively, can contain homologues of non-human origin. Members of a family may also have common functional characteristics. PD-1 ligands are members of the B7 family of polypeptides. The term "B7 family" or "B7 polypeptides" as used herein includes costimulatory polypeptides that share sequence homology with B7 polypeptides, e.g., with B7-1 (CD80), B7-2 (CD86), inducible costimulatory ligand (ICOS-L), B7-H3, B7-H4, VISTA, B7-H6, B7h (Swallow et al. (1999) Immunity 11:423), and/or PD-1 ligands (e.g., PD-L1 or PD-L2). For example, human B7-1 and B7-2 share approximately 26% amino acid sequence identity when compared using the BLAST program at NCBI with the default parameters (Blosum62 matrix with gap penalties set at existence 11 and extension 1 (see the NCBI website). The term B7 family also includes variants of these polypeptides which are capable of modulating immune cell function. The B7 family of molecules share a number of conserved regions, including signal domains, IgV domains and the IgC domains. IgV domains and the IgC domains are art-recognized Ig superfamily member domains. These domains correspond to structural units that have distinct folding patterns called Ig folds. Ig folds are comprised of a sandwich of two β sheets, each consisting of anti-parallel β strands of 5-10 amino acids with a conserved disulfide bond between the two sheets in most, but not all, IgC domains of Ig, TCR, and MHC molecules share the same types of sequence patterns and are called the C1-set within the Ig superfamily. Other IgC domains fall within other sets. IgV domains also share sequence patterns and are called V set domains. IgV domains are longer than IgC domains and contain an additional pair of β strands.

The term "PD-L1" refers to a specific PD-1 ligand. Two forms of human PD-L1 molecules have been identified. One form is a naturally occurring PD-L1 soluble polypeptide, *i.e.,* having a short hydrophilic domain at the COOH-terminal end and no transmembrane domain, and is referred to herein as PD-L1S (shown in Table 1 as SEQ ID NO: 4). The second form is a cell-associated polypeptide, *i.e.,* having a transmembrane and cytoplasmic domain, referred to herein as PD-L1M (shown in SEQ ID NO: 6). The nucleic acid and amino acid sequences of representative human PD-L1 biomarkers regarding PD-L1M are also available to the public at the GenBank database under NM_014143.3 and NP_054862.1. PD-L1 proteins comprise a signal sequence, and an IgV domain and an IgC domain. The signal sequence of SEQ ID NO: 4 is shown from about amino acid 1 to about amino acid 18. The signal sequence of SEQ ID NO: 6 is shown :from about amino acid 1 to about amino acid 18. The IgV domain of SEQ ID NO: 4 is shown from about amino acid 19 to about amino acid 134 and the IgV domain of SEQ ID NO: 6 is shown from about amino acid 19 to about amino acid 134. The IgC domain of SEQ ID NO: 4 is shown from about amino acid 135 to about amino acid 227 and the IgC domain of SEQ ID NO: 6 is shown from about amino acid 135 to about amino acid 227. The hydrophilic tail of the PD-L1 exemplified in SEQ ID NO: 4 comprises a hydrophilic tail shown from about amino acid 228 to about amino acid 245. The PD-L1 polypeptide exemplified in SEQ ID NO: 6 comprises a transmembrane domain shown from about amino acids 239 to about amino acid 259 of SEQ ID NO: 6 and a cytoplasmic domain shown of about 30 amino acids from 260 to about amino acid 290 of SEQ ID NO: 6. In addition, nucleic acid and polypeptide sequences of PD-L1 orthologs in organisms other than humans are well known and include, for example, mouse PD-L1 (NM_021893.3 and NP_068693.1), rat PD-L1 (NM_001191954.1 and NP_001178883.1), dog PD-L1 (XM_541302.3 and XP_541302.3), cow PD-L1 (NM_001163412.1 and NP_001156884.1), and chicken PD-L1 (XM_424811.3 and XP_424811.3).

The term "TIM-3" refers to a type I cell-surface glycoprotein that comprises an N-terminal immunoglobulin (Ig)-like domain, a mucin domain with O-linked glycosylations and with N-linked glycosylations close to the membrane, a single transmembrane domain, and a cytoplasmic region with tyrosine phosphorylation motif(s) (see, for example, U.S. Pat. Publ. 2013/0156774). TIM-3 is a member of the T cell/transmembrane, immunoglobulin, and mucin (TIM) gene family. Nucleic acid and polypeptide sequences of human TIM-3 are well known in the art and are publicly available, for example, as described in NM_032782.4 and NP_116171.3. The term, as described above for useful markers such as PD-L1 and PD-1, encompasses any naturally occurring allelic, splice variants, and processed forms thereof. Typically, TIM-3 refers to human TIM-3 and can include truncated forms or fragments of the TIM-3 polypeptide. In addition, nucleic acid and polypeptide sequences of TIM-3 orthologs in organisms other than humans are well known and include, for example, mouse TIM-3 (NM_134250.2 and NP_599011.2), chimpanzee TIM-3 (XM_518059.4 and XP_518059.3), dog TIM-3 (NM_001254715.1 and NP_001241644.1), cow TIM-3 (NM_001077105.2 and NP_001070573.1), and rat TIM-3 (NM_001100762.1 and NP_001094232.1). In addition, neutralizing anti-TIM-3 antibodies are well known in the art (see, at least U.S. Pat. Publ. 2013/0183688, Ngiow et al. (2011) Cancer Res. 71:3540-3551; and antibody 344823 from R&D Biosystems, as well as clones 2C23, 5D12, 2E2, 4A4, and IG5, which are all published and thus publicly available).

TIM-3 was originally identified as a mouse Th1-specific cell surface protein that was expressed after several rounds of *in vitro* Th1 differentiation, and was later shown to also be expressed on Th17 cells. In humans, TIM-3 is expressed on a subset of activated CD4+ T cells, on differentiated Th1 cells, on some CD8+ T cells, and at lower levels on Th17 cells (Hastings et al. (2009) Eur. J. Immunol. 39:2492-2501). TIM-3 is also expressed on cells of the innate immune system including mouse mast cells, subpopulations of macrophages and dendritic cells (DCs), NK and NKT cells, human monocytes, human dendritic cells, and on murine primary bronchial epithelial cell lines. TIM-3 expression is regulated by the transcription factor T-bet. TIM-3 can generate an inhibitory signal resulting in apoptosis of Th1 and Tc1 cells, and can mediate phagocytosis of apoptotic cells and cross-presentation of antigen. Polymorphisms in TIM-1 and TIM-3 can reciprocally regulate the direction of T-cell responses (Freeman et al. (2010) Immunol. Rev. 235:172-89).

TIM-3 has several known ligands, including galectin-9, phosphatidylserine, and HMGB1. For eample, galectin-9 is an S-type lectin with two distinct carbohydrate recognition domains joined by a long flexible linker, and has an enhanced affinity for larger poly-N-acetyllactosamine-containing structures. Galectin-9 does not have a signal sequence and is localized in the cytoplasm. However, it can be secreted and exerts its function by binding to glycoproteins on the target cell surface via their carbohydrate chains (Freeman et al. (2010) Immunol. Rev. 235:172-89). Engagement of TIM-3 by galectin-9 leads to Th1 cell death and a consequent decline in IFN-gamma. production. When given *in vivo,* galectin-9 had beneficial effects in several murine disease models, including an EAE model, a mouse model of arthritis, in cardiac and skin allograft transplant models, and contact hypersensitivity and psoriatic models (Freeman et al. (2010) Immunol. Rev. 235:172-89). Residues important for TIM-3 binding to galectin-9 include TIM-3(44), TIM-3(74), and TIM-3(100), which undergo N- and/or O-glycosylation. It is also known that TIM-3 mediates T-cell dysfunction associated with chronic viral infections (Golden-Mason et al. (2009) J. Virol. 83:9122-9130; Jones et al. (2008) J. Exp. Med. 205:2763-2779) and increases HIV-1-specific T cell responses when blocked *ex vivo* (Golden-Mason et al. (2009) J. Virol. 83:9122-9130). In addition, in chronic HCV infection, TIM-3 expression was increased on CD4+ and CD8+ T cells, specifically HCV-specific CD8+ cytotoxic T cells (CTLs) in chronic HCV infection and treatment with a blocking monoclonal antibody to TIM-3 reversed HCV-specific T cell exhaustion (Jones et al. (2008) J. Exp. Med. 205:2763-2779).

The term "LAG-3," also known as CD223, refers to a member of the immunoglobulin supergene family and is structurally and genetically related to CD4 (see, U.S. Pat. Publ. 2011/0150892). LAG-3 is generally known as a membrane protein encoded by a gene located on the distal part of the short arm of chromosome 12, near the CD4 gene, suggesting that the LAG-3 gene may have evolved through gene duplication (Triebel et al. (1990) J. Exp. Med. 171:1393-1405). However, secreted forms of the protein are known (e.g., for human and mouse TIM-3). Nucleic acid and polypeptide sequences of human LAG-3 are well known in the art and are publicly available, for example, as described in NM_002286.5 and NP_002277.4.

The term encompasses any naturally occurring allelic, splice variants, and processed forms thereof. Typically, LAG-3 refers to human LAG-3 and can include truncated forms or fragments of the LAG-3 polypeptide. In addition, nucleic acid and polypeptide sequences of LAG-3 orthologs in organisms other than humans are well known and include, for example, mouse LAG-3 (NM_008479.2 and NP_032505.1), chimpanzee LAG-3 (XM_508966.4 and XP_508966.2), monkey LAG-3 (XM_001108923.2 and XP_001108923.1), cow LAG-3 (NM_00124949.1 and NP_001232878.1), rat LAG-3 (NM_212513.2 and NP_997678.2), and chicken LAG-3 (XM_416510.3, XP_416510.2, XM_004938117.1, and XP_004938174.1). In addition, neutralizing anti-LAG-3 antibodies are well known in the art (see, at least U.S. Pat. Publs. 2011/0150892 and 2010/0233183; Macon-Lemaitre and Triebel (2005) Immunology 115:170-178; Drake et al. (2006) J. Clin. Oncol. 24:2573; Richter et al. (2010) Int. Immunol. 22:13-23).

LAG-3 is not expressed on resting peripheral blood lymphocytes but is expressed on activated T cells and NK cells and has a number of functions (see, U.S. Pat. Publ. 2011/0150892). Similar to CD4, LAG-3 has been demonstrated to interact with MHC Class II molecules but, unlike CD4, LAG-3 does not interact with the human immunodeficiency virus gp120 protein (Baixeras et al. (1992) J. Exp. Med. 176:327-337). Studies using a soluble LAG-3 immunoglobulin fusion protein (sLAG-3Ig) demonstrated direct and specific binding of LAG-3 to MHC class II on the cell surface (Huard et al. (1996) Eur. J. Immunol. 26:1180-1186). *In in vitro* studies of antigen-specific T cell responses, the addition of anti-LAG-3 antibodies led to increased T cell proliferation and higher expression of activation antigens such as CD25, supporting a role for the LAG-/MHC class II interaction in down-regulating antigen-dependent stimulation of CD4+ T lymphocytes (Huard et al. (1994) Eur. J. Immunol. 24:3216-3221). The intra-cytoplasmic region of LAG-3 has been demonstrated to interact with a protein termed LAP, which is thought to be a signal transduction molecule involved in the downregulation of the CD3/TCR activation pathway (Iouzalen et al. (2001) Eur. J. Immunol. 31:2885-2891). Furthermore, CD4+CD25+ regulatory T cells (T_{reg}) have been shown to express LAG-3 upon activation and antibodies to LAG-3 inhibit suppression by induced regulatory T cells, both *in vitro* and *in vivo,* suggesting that LAG-3 contributes to the suppressor activity of regulatory T cells (Huang et al. (2004) Immunity 21:503-513). Still further, LAG-3 has been shown to negatively regulate T cell homeostasis by regulatory T cells in both T cell-dependent and independent mechanisms (Workman and Vignali (2005) J. Immunol. 174:688-695).

In certain circumstances, LAG-3 also has been shown to have immunostimulatory effects. For example, LAG-3 transfected tumor cells transplanted into syngeneic mice showed marked growth reduction or complete regression as compared to untransfected tumor cells, suggesting that LAG-3 expression on the tumor cells stimulated an anti-tumor response by triggering antigen presenting cells via MHC class II molecules (Prigent et al. (1999) Eur. J. Immunol. 29:3867-3876). Additionally, soluble LAG-3 Ig fusion protein has been shown to stimulate both humoral and cellular immune responses when administered to mice together with an antigen, indicating that soluble LAG-3Ig can function as a vaccine adjuvant (El Mir and Triebel (2000) J. Immunol. 164:5583-5589). Furthermore, soluble human LAG-3Ig has been shown to amplify the *in vitro* generation of type I tumor-specific immunity (Casati et al. (2006) Cancer Res. 66:4450-4460). The functional activity of LAG-3 is reviewed further in Triebel (2003) Trends Immunol. 24:619-622.

CTLA-4 is a T cell surface molecule that was originally identified by differential screening of a murine cytolytic T cell cDNA library, Brunet et al. (1987) Nature 328:267-270. The role of CTLA-4 as a second receptor for B7 is discussed in Linsley et al. (1991) J. Exp. Med. 174:561-569. Freeman et al. (1993) Science 262:907-909 discusses CTLA-4 in B7 deficient mice. Ligands for CTLA-4 are described in Lenschow et al. (1993) P.N.A.S. 90:11054-11058. Linsley et al. (1992) Science 257:792-795 describes immunosuppression in vivo by a soluble form of CTLA-4. Lenschow et al. (1992) Science 257:789-792 discusses long term survival of pancreatic islet grafts induced by CTLA-4lg. It is suggested in Walunas et al. (1994) Immunity 1:405-413, that CTLA-4 can function as a negative regulator of T cell activation. The amino acid and nucleotide sequence of CTLA-4 (*e.g.,* human CTLA-4) are known in the art (*e.g.,* as described in US 5,811,097 and US 5,434,131, incorporated herein by reference).

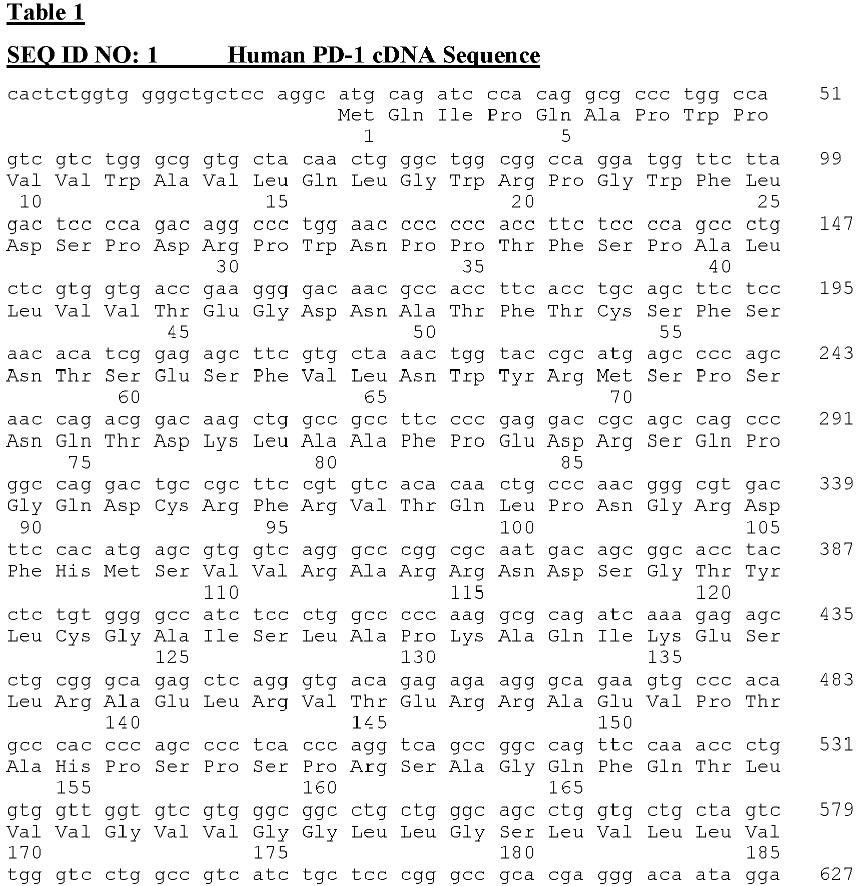

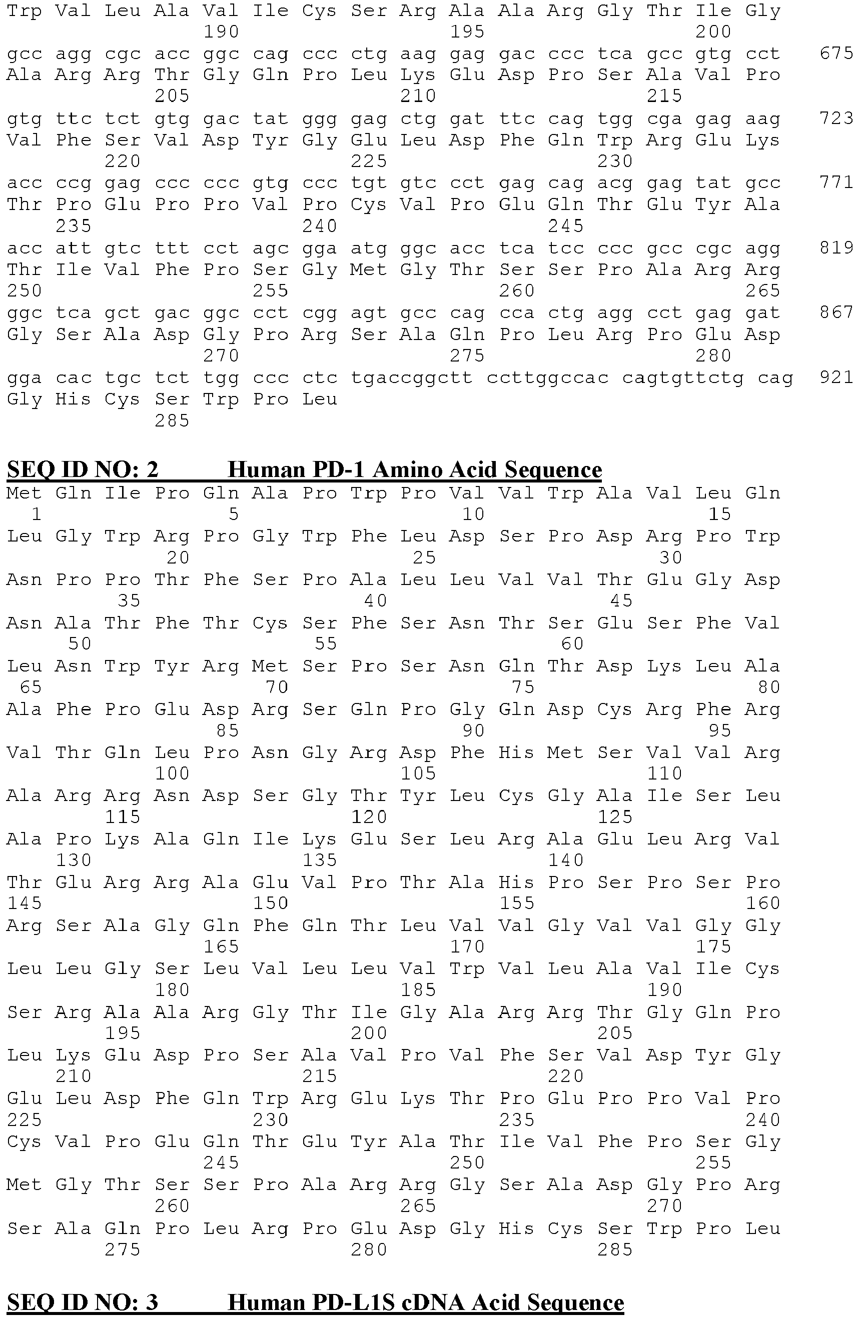

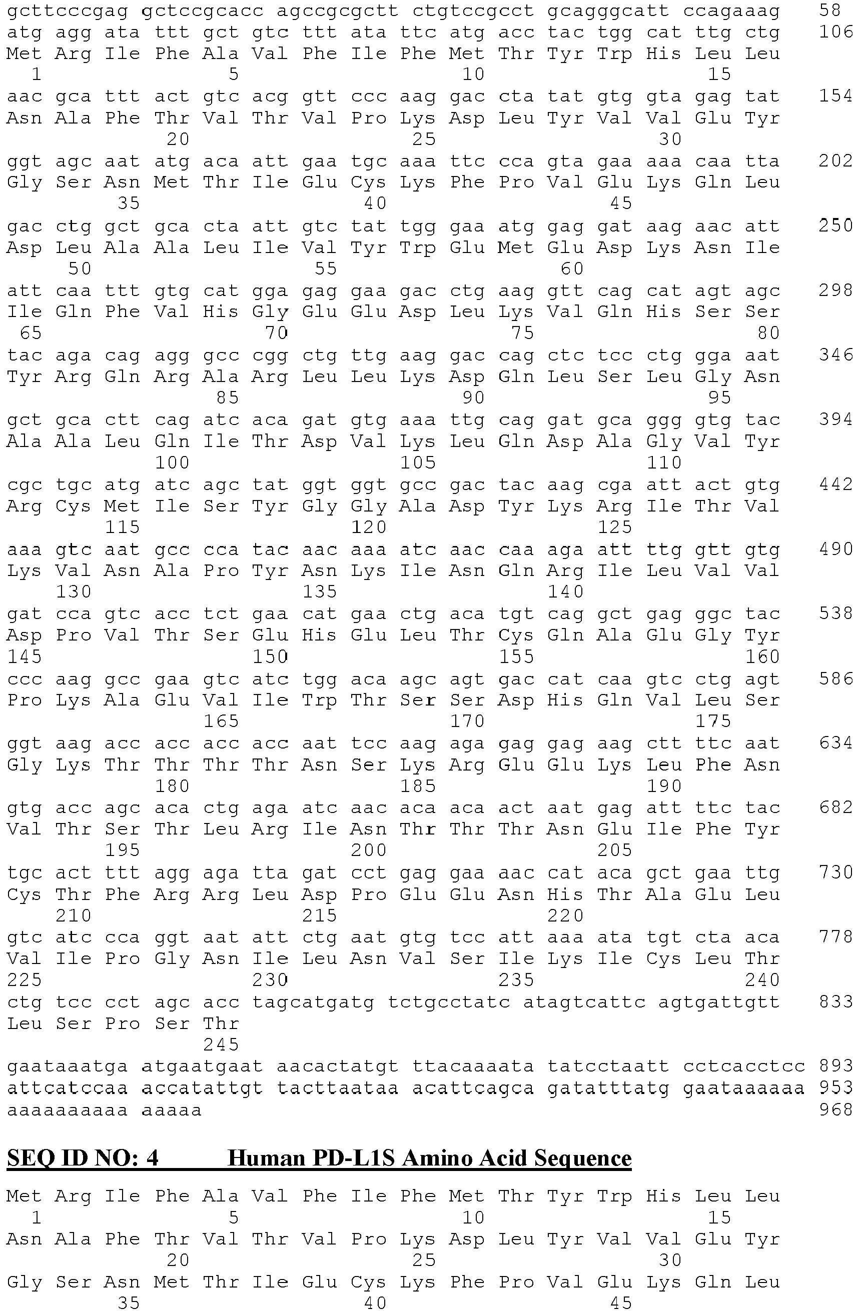

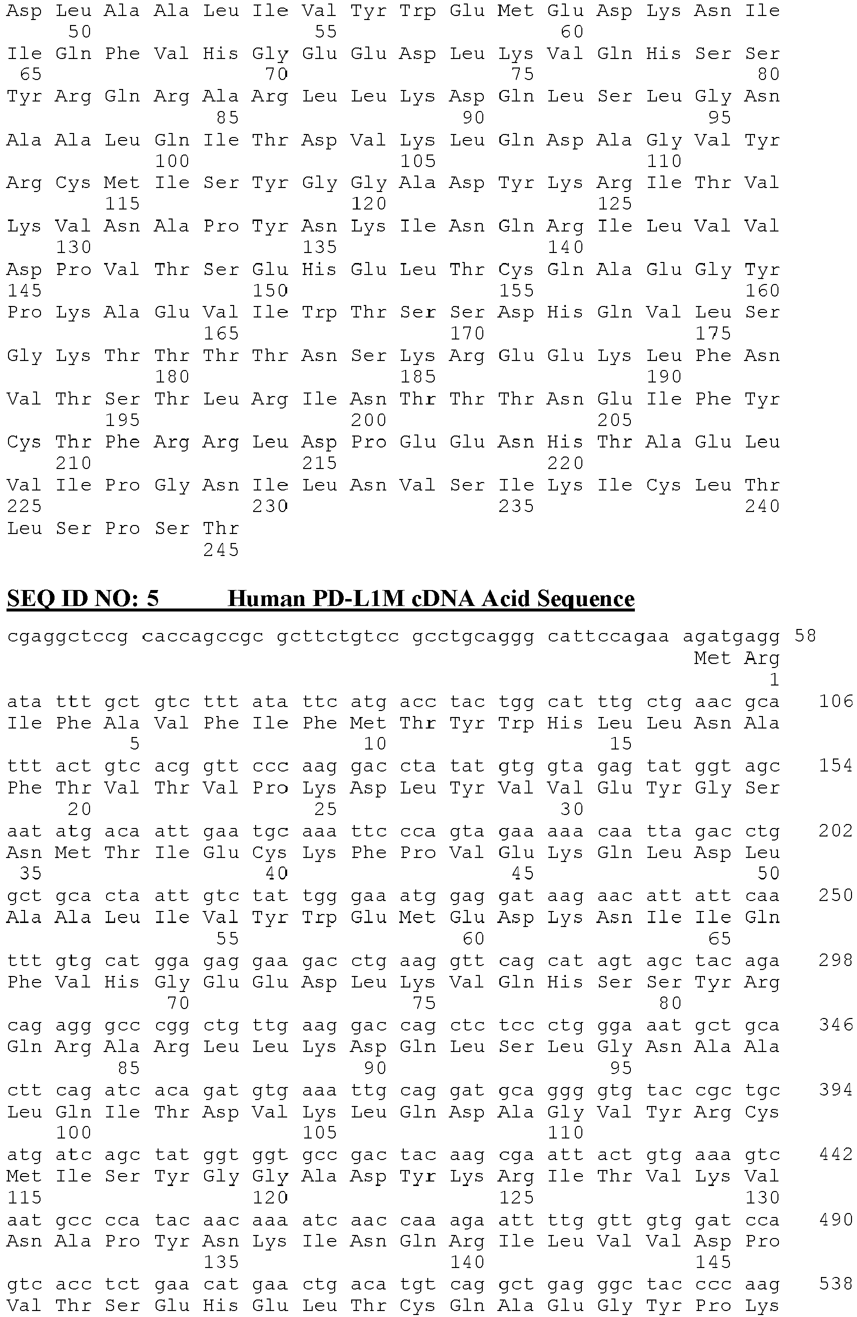

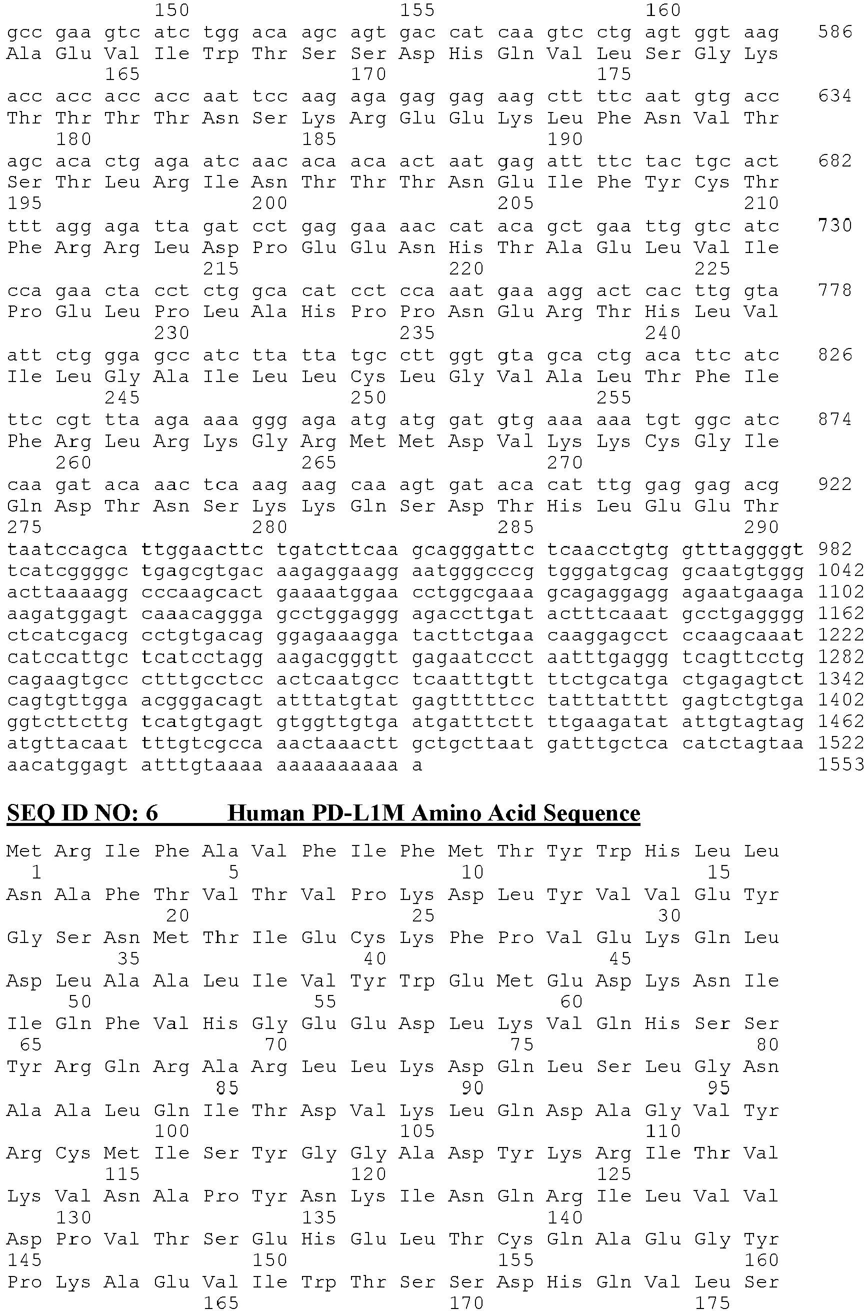

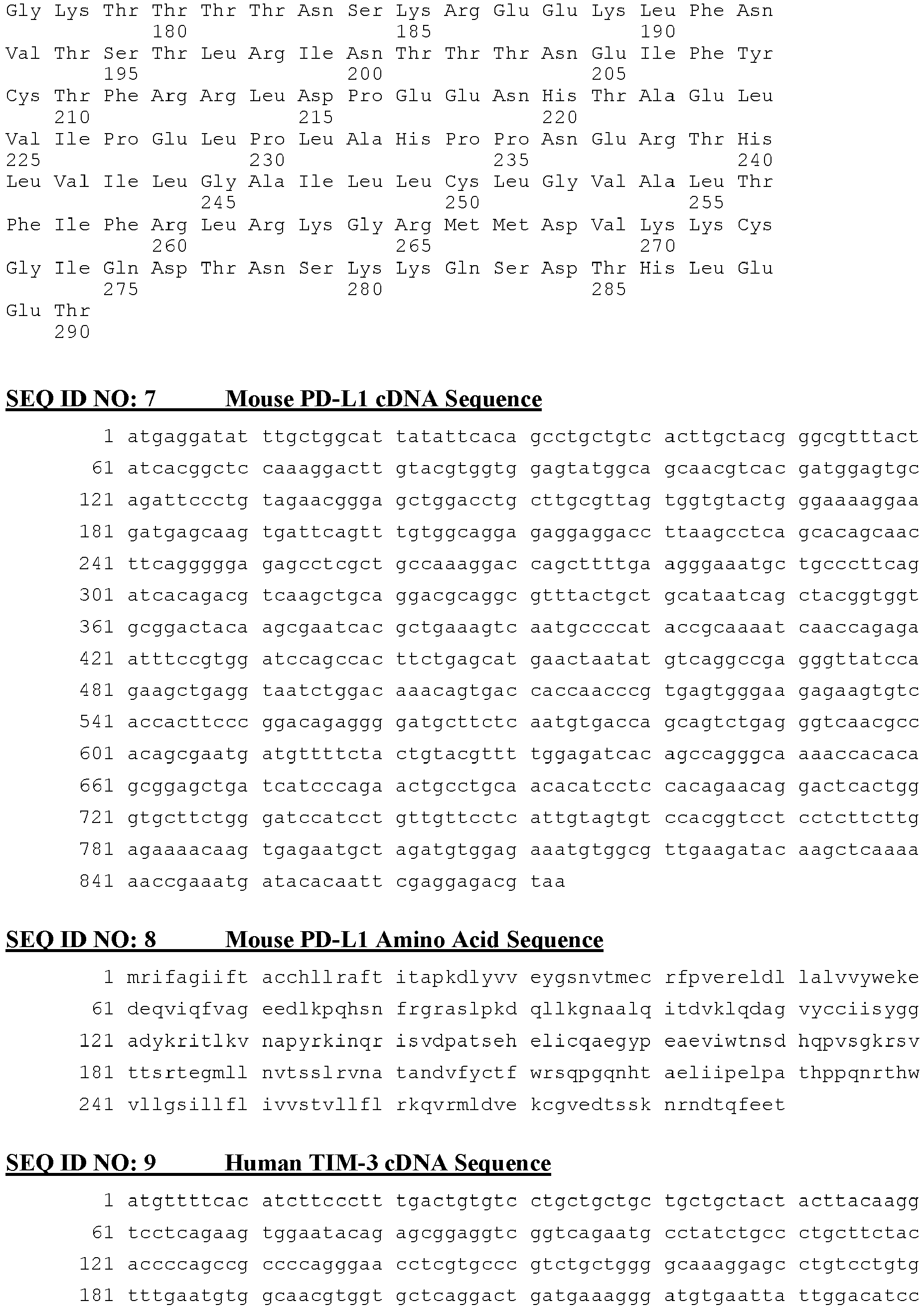

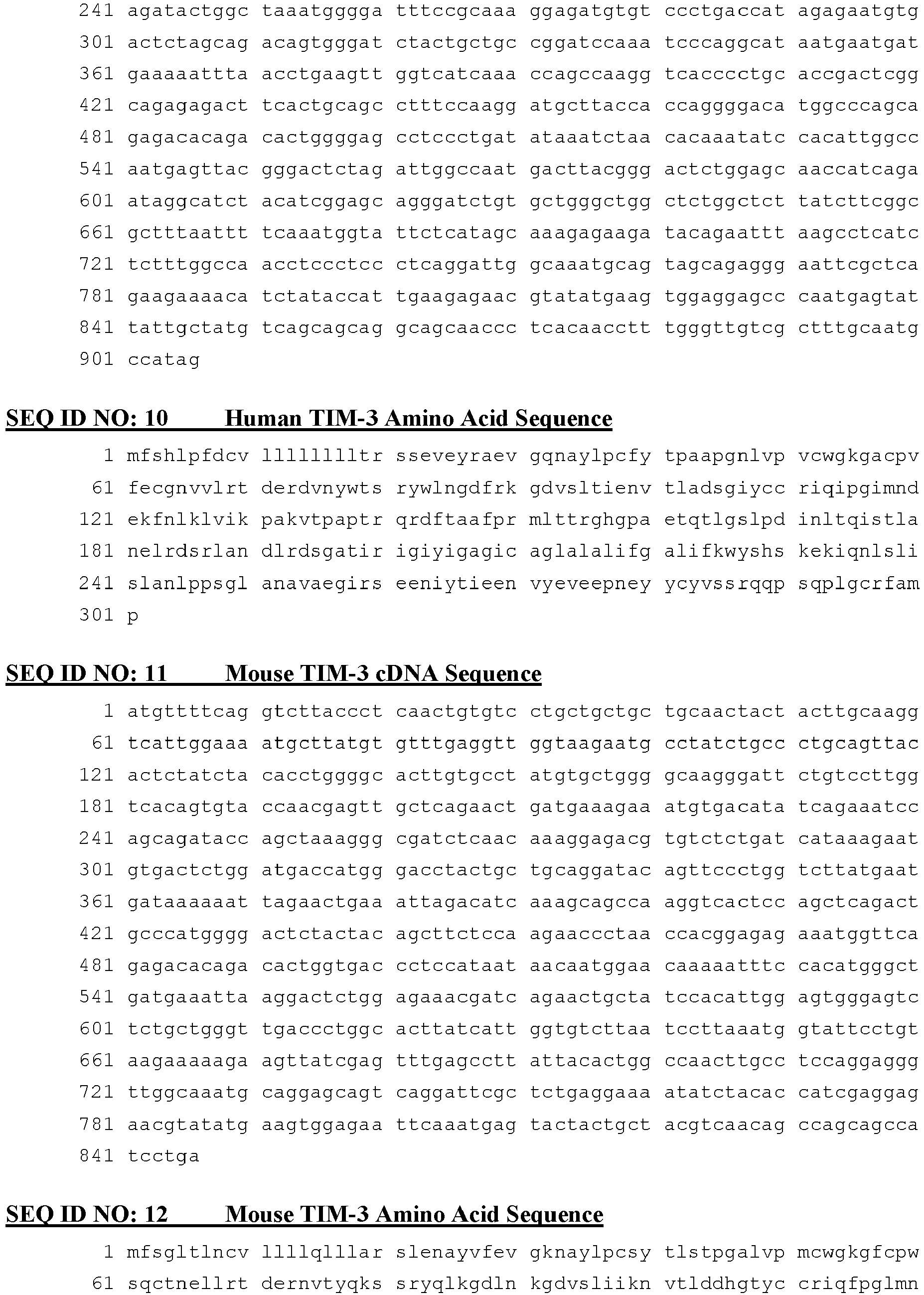

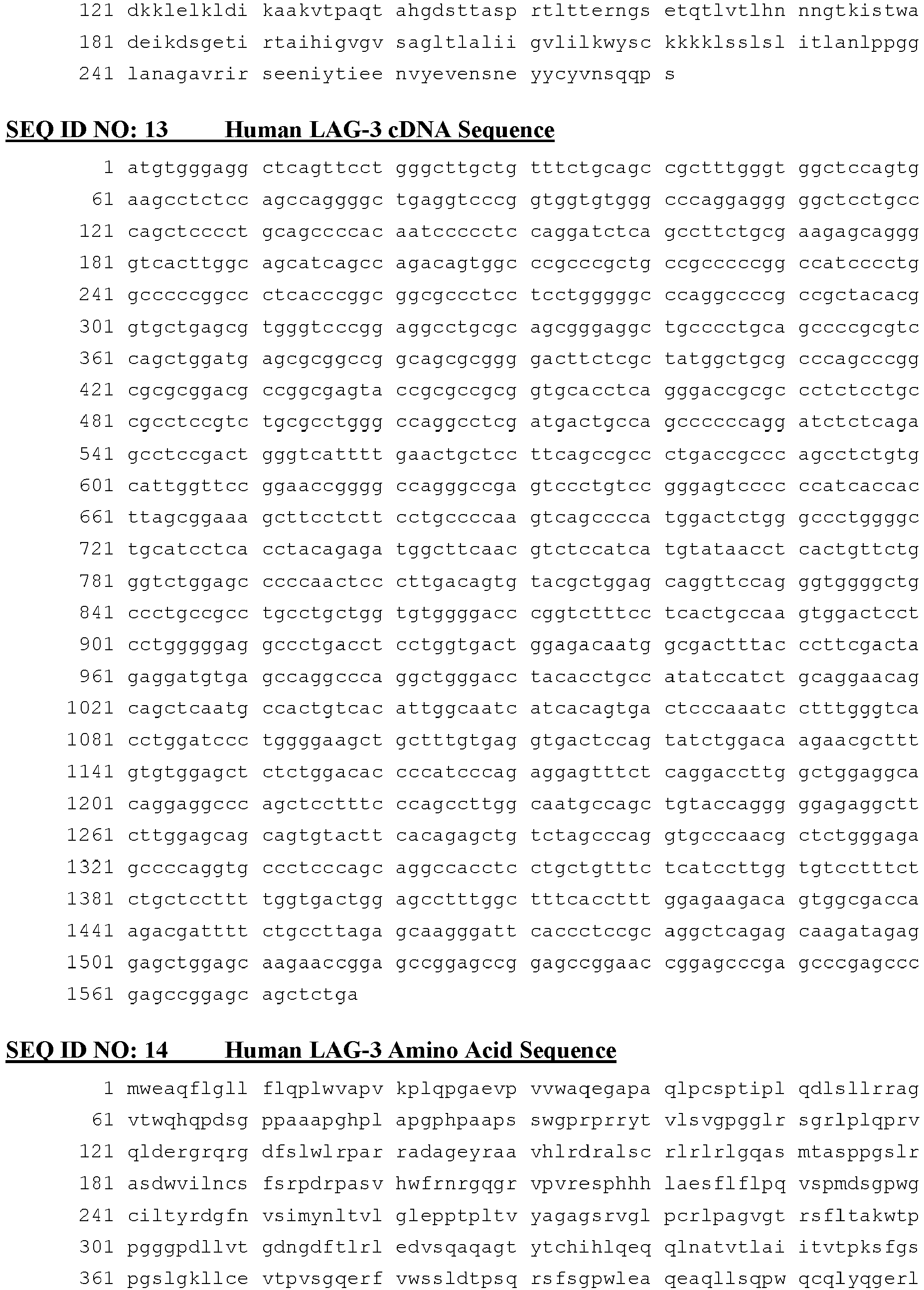

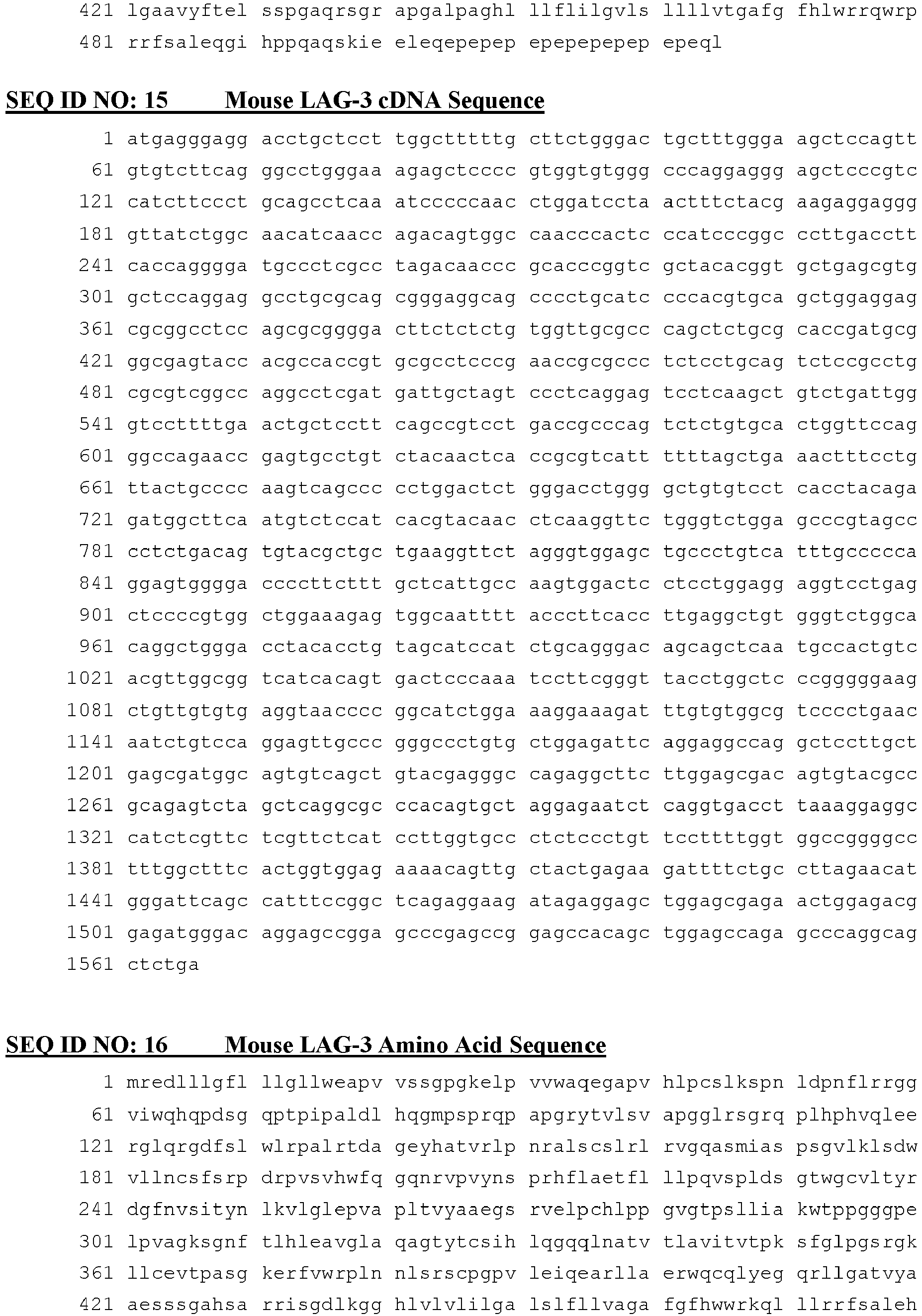

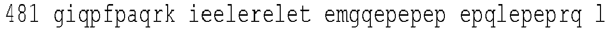

Inhibitors of immune checkpoint modulators are known in the art, and can be used in any of the methods described herein. For example, anti-PD-1 antibodies or soluble polypeptide inhibitors can be used. In some embodiments, the anti-PD-1 antibody is chosen from MDX-1106, Merck 3475 or CT- 011. MDX-1106, also known as MDX-1106-04, ONO-4538, BMS-936558 or Nivolumab. In some embodiments, the anti-PD- 1 antibody is Nivolumab (CAS Registry Number: 946414-94-4). Nivolumab (also referred to as BMS-936558 or MDX1106; Bristol-Myers Squibb) is a fully human IgG4 monoclonal antibody which specifically blocks PD1. Nivolumab (clone 5C4) and other human monoclonal antibodies that specifically bind to PD1 are disclosed in US 8,008,449 and WO2006/121168. Lambrolizumab (also referred to as MK03475; Merck) is a humanized IgG4 monoclonal antibody that binds to PD1. Lambrolizumab and other humanized anti-PD1 antibodies are disclosed in US 8,354,509 and WO2009/114335. Pidilizumab (CT-011; Cure Tech) is a humanized IgGlk monoclonal antibody that binds to PD1. Pidilizumab and other humanized anti-PD1 monoclonal antibodies are disclosed in WO2009/101611. Other anti-PD1 antibodies include AMP 514 (Amplimmune), among others, *e.g*., anti-PD1 antibodies disclosed in US 8,609,089, US 2010028330, and/or US 20120114649. In some embodiments, the PD- 1 inhibitor is an immunoadhesin (*e.g*., an immunoadhesin comprising an extracellular or PD- 1 binding portion of PD-L 1 or PD-L2 fused to a constant region (*e.g.,* an Fc region of an immunoglobulin sequence). In some embodiments, the PD-1 inhibitor is AMP-224. AMP-224 (B7-DCIg; Amplimmune; e.g., disclosed in WO2010/027827 and WO2011/066342), is a PD-L2 Fc fusion soluble receptor that blocks the interaction between PD1 and B7-H1.

In other embodiments, anti-PD-L1 antibodies or soluble polypeptide inhibitors can be used. In some embodiments, the PD-L1 inhibitor is anti-PD-L1 antibody. In other embodiments, the anti-PD-L1 binding antagonist is chosen from YW243.55.S70, MPDL3280A or MDX-1105. MDX-1105, also known as BMS-936559, is an anti-PD-L1 antibody described in WO2007/005874. MDPL3280A (Genentech / Roche) is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. Antibody YW243.55.S70 (heavy and light chain variable region sequences shown in SEQ ID Nos. 20 and 21 , respectively) is an anti-PD-L1 described in WO 2010/077634.

Exemplary anti-CTLA4 antibodies that can be used in the methods disclosed herein include, but are not limited to, Tremelimumab (IgG2 monoclonal antibody available from Pfizer, formerly known as ticilimumab, CP-675,206); and Ipilimumab (CTLA-4 antibody, also known as MDX-010, CAS No. 477202-00-9). Antibodies to T cell costimulatory molecules such as CTLA-4 (e.g., U.S. Pat. No. 5,811,097), CTLA-4 inhibitor (e.g., CP-675,206, ipilimumab)

### II. Methods of Treating Hematologic Cancers

### a. Agents Useful For Treating Hematologic Cancers

It is demonstrated herein that inhibiting or blocking a function of PD-1 or PD-L1 and TIM-3, LAG-3 or CTLA-4 synergistically and significantly blocks the establishment and progression of malignancies (e.g., hematologic cancers such as multiple myeloma) in all subjects analyzed. Inhibition or blockade of PD-1 or PD-L1 and TIM-3, LAG-3 or CTLA-4 function can block the establishment and progression of similar malignancies. Thus, the agents of the present invention described herein that modulate the interaction between, for example, PD-1 or PD-L1 and TIM-3, LAG-3 or CTLA-4, whether directly or indirectly, can upregulate or downregulate the immune system and, thereby, upregulate or downregulate an immune response.

PD-1, PD-L1 and TIM-3, LAG-3 and CTLA-4 are immune checkpoint regulators that deliver co-inhibitory immune signals. Thus, in one embodiment, agents that neutralize an activity of PD-1 or PD-L1 and TIM-3, LAG-3 or CTLA-4 can prevent inhibitory signaling and upregulate an immune response. In another embodiment, agents which directly block the interaction between PD-1 or PD-L1 and its natural receptor(s), and TIM-3, LAG-3 or CTLA-4 and its natural receptor(s) (*e.g*., blocking antibodies) can prevent inhibitory signaling and upregulate an immune response. Alternatively, agents that indirectly block the interaction between PD-1 or PD-L1 and its natural receptor(s), and TIM-3, LAG-3 or CTLA-4 and its natural receptor(s) can prevent inhibitory signaling and upregulate an immune response. For example, soluble B7-1 or soluble PD-1, by binding to a PD-L1 polypeptide indirectly reduces the effective concentration of PD-L1 polypeptide available to bind to the immunoinhibitor receptor, PD-1. Exemplary agents for upregulating an immune response include antibodies against PD-1, PD-L1, LAG-3, CTLA-4 and/or TIM-3 that block the interaction between the immune checkpoint regulator and its natural receptor(s); a non-activating form of PD-1, PD-L1, LAG-3, CTLA-4 and/or TIM-3 (*e.g.,* a dominant negative polypeptide), small molecules or peptides that block the interaction between the immune checkpoint regulator and its natural receptor(s); fusion proteins (*e.g.* the extracellular portion of -1, PD-L1, LAG-3, CTLA-4 or TIM-3 fused to the Fc portion of an antibody or immunoglobulin) that bind to their natural receptor(s); nucleic acid molecules that block PD-1, PD-L1, LAG-3, CTLA-4 and/or TIM-3 gene expression, *e.g.,* transcription or translation; and the like.

Additional agents useful in the methods of the present invention include antibodies, small molecules, peptides, peptidomimetics, natural ligands, and derivatives of natural ligands, that can either bind and/or inactivate or inhibit protein biomarkers of the invention, including the biomarkers listed in Table 1, or fragments thereof; as well as RNA interference, antisense, nucleic acid aptamers, etc. that can downregulate the expression and/or activity of the biomarkers of the invention, including the biomarkers listed in Table 1, or fragments thereof.

In one embodiment, isolated nucleic acid molecules that specifically hybridize with or encode one or more biomarkers of the invention, listed in Table 1 for example, or biologically active portions thereof. As used herein, the term "nucleic acid molecule" is intended to include DNA molecules (*i.e.,* cDNA or genomic DNA) and RNA molecules (*i.e.,* mRNA) and analogs of the DNA or RNA generated using nucleotide analogs. The nucleic acid molecule can be single-stranded or double-stranded, but preferably is double-stranded DNA. An "isolated" nucleic acid molecule is one which is separated from other nucleic acid molecules which are present in the natural source of the nucleic acid. Preferably, an "isolated" nucleic acid is free of sequences which naturally flank the nucleic acid (*i.e.,* sequences located at the 5' and 3' ends of the nucleic acid) in the genomic DNA of the organism from which the nucleic acid is derived. For example, in various embodiments, the isolated nucleic acid molecules corresponding to the one or more biomarkers listed in Table 1 or described herein can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived (*i.e.,* a lymphoma cell). Moreover, an "isolated" nucleic acid molecule, such as a cDNA molecule, can be substantially free of other cellular material, or culture medium when produced by recombinant techniques, or chemical precursors or other chemicals when chemically synthesized.

A nucleic acid molecule of the present invention, *e.g.,* a nucleic acid molecule having the nucleotide sequence of one or more biomarkers listed in Table 1 or a nucleotide sequence which is at least about 50%, preferably at least about 60%, more preferably at least about 70%, yet more preferably at least about 80%, still more preferably at least about 90%, and most preferably at least about 95% or more (*e.g*., about 98%) homologous to the nucleotide sequence of one or more biomarkers listed in Table 1 or a portion thereof *(i.e.,* 100, 200, 300, 400, 450, 500, or more nucleotides), can be isolated using standard molecular biology techniques and the sequence information provided herein. For example, a human cDNA can be isolated from a human cell line using all or portion of the nucleic acid molecule, or fragment thereof, as a hybridization probe and standard hybridization techniques (*i.e.,* as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989). Moreover, a nucleic acid molecule encompassing all or a portion of the nucleotide sequence of one or more biomarkers listed in Table 1 or a nucleotide sequence which is at least about 50%, preferably at least about 60%, more preferably at least about 70%, yet more preferably at least about 80%, still more preferably at least about 90%, and most preferably at least about 95% or more homologous to the nucleotide sequence, or fragment thereof, can be isolated by the polymerase chain reaction using oligonucleotide primers designed based upon the sequence of the one or more biomarkers listed in Table 1, or fragment thereof, or the homologous nucleotide sequence. For example, mRNA can be isolated from muscle cells (*i.e.,* by the guanidinium-thiocyanate extraction procedure of Chirgwin et al. (1979) Biochemistry 18: 5294-5299) and cDNA can be prepared using reverse transcriptase (*i.e.,* Moloney MLV reverse transcriptase, available from Gibco/BRL, Bethesda, MD; or AMV reverse transcriptase, available from Seikagaku America, Inc., St. Petersburg, FL). Synthetic oligonucleotide primers for PCR amplification can be designed according to well-known methods in the art. A nucleic acid of the invention can be amplified using cDNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to the nucleotide sequence of one or more biomarkers listed in Table 1 can be prepared by standard synthetic techniques, *i.e.,* using an automated DNA synthesizer.

Probes based on the nucleotide sequences of one or more biomarkers listed in Table 1 can be used to detect or confirm the desired transcripts or genomic sequences encoding the same or homologous proteins. In preferred embodiments, the probe further comprises a label group attached thereto, *i.e.,* the label group can be a radioisotope, a fluorescent compound, an enzyme, or an enzyme co-factor. Such probes can be used as a part of a diagnostic test kit for identifying cells or tissue which express one or more biomarkers listed in Table 1, such as by measuring a level of one or more biomarkers nucleic acid in a sample of cells from a subject, *i.e.,* detecting mRNA levels of one or more biomarkers listed in Table 1.

Nucleic acid molecules encoding proteins corresponding to one or more biomarkers listed in Table 1, or portions thereof, from different species are also contemplated. For example, rat or monkey cDNA can be identified based on the nucleotide sequence of a human and/or mouse sequence and such sequences are well known in the art. In one embodiment, the nucleic acid molecule(s) of the invention encodes a protein or portion thereof which includes an amino acid sequence which is sufficiently homologous to an amino acid sequence of one or more biomarkers listed in Table 1, such that the protein or portion thereof modulates (e.g., enhance), one or more of the following biological activities: a) binding to the biomarker; b) modulating the copy number of the biomarker; c) modulating the expression level of the biomarker; and d) modulating the activity level of the biomarker.

As used herein, the language "sufficiently homologous" refers to proteins or portions thereof which have amino acid sequences which include a minimum number of identical or equivalent (e.g., an amino acid residue which has a similar side chain as an amino acid residue in one or more biomarkers listed in Table 1, or fragment thereof) amino acid residues to an amino acid sequence of the biomarker, or fragment thereof, such that the protein or portion thereof modulates (e.g., enhance) one or more of the following biological activities: a) binding to the biomarker; b) modulating the copy number of the biomarker; c) modulating the expression level of the biomarker; and d) modulating the activity level of the biomarker.

In another embodiment, the protein is at least about 50%, preferably at least about 60%, more preferably at least about 70%, 75%, 80%, 85%, 90%, 91%, 92%, 93%, 94%, 95%, 96%, 97%, 98%, 99% or more homologous to the entire amino acid sequence of the biomarker, or a fragment thereof.

Portions of proteins encoded by nucleic acid molecules of the one or more biomarkers listed in Table 1 are preferably biologically active portions of the protein. As used herein, the term "biologically active portion" of one or more biomarkers listed in Table 1 is intended to include a portion, *e.g.,* a domain/motif, that has one or more of the biological activities of the full-length protein.

Standard binding assays, *e.g.,* immunoprecipitations and yeast two-hybrid assays, as described herein, or functional assays, *e.g.,* RNAi or overexpression experiments, can be performed to determine the ability of the protein or a biologically active fragment thereof to maintain a biological activity of the full-length protein.

The invention further encompasses nucleic acid molecules that differ from the nucleotide sequence of the one or more biomarkers listed in Table 1, or fragment thereof due to degeneracy of the genetic code and thus encode the same protein as that encoded by the nucleotide sequence, or fragment thereof. In another embodiment, an isolated nucleic acid molecule of the invention has a nucleotide sequence encoding a protein having an amino acid sequence of one or more biomarkers listed in Table 1, or fragment thereof, or a protein having an amino acid sequence which is at least about 70%, 75%, 80%, 85%, 90%, 91%, 92%, 93%, 94%, 95%, 96%, 97%, 98%, 99% or more homologous to the amino acid sequence of the one or more biomarkers listed in Table 1, or fragment thereof. In another embodiment, a nucleic acid encoding a polypeptide consists of nucleic acid sequence encoding a portion of a full-length fragment of interest that is less than 195, 190, 185, 180, 175, 170, 165, 160, 155, 150, 145, 140, 135, 130, 125, 120, 115, 110, 105, 100, 95, 90, 85, 80, 75, or 70 amino acids in length.

It will be appreciated by those skilled in the art that DNA sequence polymorphisms that lead to changes in the amino acid sequences of the one or more biomarkers listed in Table 1 may exist within a population (*e.g.,* a mammalian and/or human population). Such genetic polymorphisms may exist among individuals within a population due to natural allelic variation. As used herein, the terms "gene" and "recombinant gene" refer to nucleic acid molecules comprising an open reading frame encoding one or more biomarkers listed in Table 1, preferably a mammalian, *e.g.,* human, protein. Such natural allelic variations can typically result in 1-5% variance in the nucleotide sequence of the one or more biomarkers listed in Table 1. Any and all such nucleotide variations and resulting amino acid polymorphisms in the one or more biomarkers listed in Table 1 that are the result of natural allelic variation and that do not alter the functional activity of the one or more biomarkers listed in Table 1 are intended to be within the scope of the invention. Moreover, nucleic acid molecules encoding one or more biomarkers listed in Table 1 from other species.

In addition to naturally-occurring allelic variants of the one or more biomarkers listed in Table 1 that may exist in the population, the skilled artisan will further appreciate that changes can be introduced by mutation into the nucleotide sequence, or fragment thereof, thereby leading to changes in the amino acid sequence of the encoded one or more biomarkers listed in Table 1, without altering the functional ability of the one or more biomarkers listed in Table 1. For example, nucleotide substitutions leading to amino acid substitutions at "non-essential" amino acid residues can be made in the sequence, or fragment thereof. A "non-essential" amino acid residue is a residue that can be altered from the wild-type sequence of the one or more biomarkers listed in Table 1 without altering the activity of the one or more biomarkers listed in Table 1, whereas an "essential" amino acid residue is required for the activity of the one or more biomarkers listed in Table 1. Other amino acid residues, however, (*e.g.,* those that are not conserved or only semi-conserved between mouse and human) may not be essential for activity and thus are likely to be amenable to alteration without altering the activity of the one or more biomarkers listed in Table 1.

The comparison of sequences and determination of percent homology between two sequences can be accomplished using a mathematical algorithm. Preferably, the alignment can be performed using the Clustal Method. Multiple alignment parameters include GAP Penalty =10, Gap Length Penalty = 10. For DNA alignments, the pairwise alignment parameters can be Htuple=2, Gap penalty=5, Window=4, and Diagonal saved=4. For protein alignments, the pairwise alignment parameters can be Ktuple=1, Gap penalty=3, Window=5, and Diagonals Saved=5.

In a preferred embodiment, the percent identity between two amino acid sequences is determined using the Needleman and Wunsch (J. Mol. Biol. (48):444-453 (1970)) algorithm which has been incorporated into the GAP program in the GCG software package (available online), using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available online), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4:11-17 (1989)) which has been incorporated into the ALIGN program (version 2.0) (available online), using a PAM120 weight residue table, a gap length penalty of 12 and a gap penalty of 4.

An isolated nucleic acid molecule encoding a protein homologous to one or more biomarkers listed in Table 1, or fragment thereof, can be created by introducing one or more nucleotide substitutions, additions or deletions into the nucleotide sequence, or fragment thereof, or a homologous nucleotide sequence such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.,* alanine, valine, leucine, isoleucine, proline, phenylalanine, methionine, tryptophan), branched side chains (*e.g.,* threonine, valine, isoleucine) and aromatic side chains (*e.g.,* tyrosine, phenylalanine, tryptophan, histidine). Thus, a predicted nonessential amino acid residue in one or more biomarkers listed in Table 1 is preferably replaced with another amino acid residue from the same side chain family. Alternatively, in another embodiment, mutations can be introduced randomly along all or part of the coding sequence of the one or more biomarkers listed in Table 1, such as by saturation mutagenesis, and the resultant mutants can be screened for an activity described herein to identify mutants that retain desired activity. Following mutagenesis, the encoded protein can be expressed recombinantly according to well-known methods in the art and the activity of the protein can be determined using, for example, assays described herein.

The levels of one or more biomarkers listed in Table 1 may be assessed by any of a wide variety of well-known methods for detecting expression of a transcribed molecule or protein. Non-limiting examples of such methods include immunological methods for detection of proteins, protein purification methods, protein function or activity assays, nucleic acid hybridization methods, nucleic acid reverse transcription methods, and nucleic acid amplification methods.

In preferred embodiments, the levels of one or more biomarkers listed in Table 1 are ascertained by measuring gene transcript (e.g., mRNA), by a measure of the quantity of translated protein, or by a measure of gene product activity. Expression levels can be monitored in a variety of ways, including by detecting mRNA levels, protein levels, or protein activity, any of which can be measured using standard techniques. Detection can involve quantification of the level of gene expression (*e.g.,* genomic DNA, cDNA, mRNA, protein, or enzyme activity), or, alternatively, can be a qualitative assessment of the level of gene expression, in particular in comparison with a control level. The type of level being detected will be clear from the context.

In a particular embodiment, the mRNA expression level can be determined both by *in situ* and by *in vitro* formats in a biological sample using methods known in the art. The term "biological sample" is intended to include tissues, cells, biological fluids and isolates thereof, isolated from a subject, as well as tissues, cells and fluids present within a subject. Many expression detection methods use isolated RNA. For *in vitro* methods, any RNA isolation technique that does not select against the isolation of mRNA can be utilized for the purification of RNA from cells (see, *e.g.,* Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155).

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding one or more biomarkers listed in Table 1. Other suitable probes for use in the diagnostic assays of the invention are described herein. Hybridization of an mRNA with the probe indicates that one or more biomarkers listed in Table 1 is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array, *e.g.,* an Affymetrix™ gene chip array. A skilled artisan can readily adapt known mRNA detection methods for use in detecting the level of the one or more biomarkers listed in Table 1.

An alternative method for determining mRNA expression level in a sample involves the process of nucleic acid amplification, *e.g.,* by RT-PCR (the experimental embodiment set forth in Mullis, 1987, U.S. Patent No. 4,683,202), ligase chain reaction (Barany, 1991, Proc. Natl. Acad. Sci. USA, 88:189-193), self-sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques well-known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the cells prior to detection. In such methods, a cell or tissue sample is prepared/processed using known histological methods. The sample is then immobilized on a support, typically a glass slide, and then contacted with a probe that can hybridize to the one or more biomarkers listed in Table 1.

As an alternative to making determinations based on the absolute expression level, determinations may be based on the normalized expression level of one or more biomarkers listed in Table 1. Expression levels are normalized by correcting the absolute expression level by comparing its expression to the expression of a non-biomarker gene, *e.g.,* a housekeeping gene that is constitutively expressed. Suitable genes for normalization include housekeeping genes such as the actin gene, or epithelial cell-specific genes. This normalization allows the comparison of the expression level in one sample, *e.g.,* a subject sample, to another sample, *e.g.,* a normal sample, or between samples from different sources.

The level or activity of a protein corresponding to one or more biomarkers listed in Table 1 can also be detected and/or quantified by detecting or quantifying the expressed polypeptide. The polypeptide can be detected and quantified by any of a number of means well known to those of skill in the art. These may include analytic biochemical methods such as electrophoresis, capillary electrophoresis, high performance liquid chromatography (HPLC), thin layer chromatography (TLC), hyperdiffusion chromatography, and the like, or various immunological methods such as fluid or gel precipitin reactions, immunodiffusion (single or double), immunoelectrophoresis, radioimmunoassay (RIA), enzyme-linked immunosorbent assays (ELISAs), immunofluorescent assays, Western blotting, and the like. A skilled artisan can readily adapt known protein/antibody detection methods for use in determining whether cells express the biomarker of interest.

The present invention further provides soluble, purified and/or isolated polypeptide forms of one or more biomarkers listed in Table 1, or fragments thereof. In addition, it is to be understood that any and all attributes of the polypeptides described herein, such as percentage identities, polypeptide lengths, polypeptide fragments, biological activities, antibodies, *etc.* can be combined in any order or combination with respect to any biomarker listed in Table 1 and combinations thereof.

In one aspect, a polypeptide may comprise a full-length amino acid sequence corresponding to one or more biomarkers listed in Table 1 or a full-length amino acid sequence with 1 to about 20 conservative amino acid substitutions. An amino acid sequence of any described herein can also be at least 50, 55, 60, 65, 70, 75, 80, 85, 90, 91, 92, 93, 94, 95, 96, 97, 98, 99, or 99.5% identical to the full-length sequence of one or more biomarkers listed in Table 1, which is either described herein, well known in the art, or a fragment thereof. In another aspect, the present invention contemplates a composition comprising an isolated polypeptide corresponding to one or more biomarkers listed in Table 1 and less than about 25%, or alternatively 15%, or alternatively 5%, contaminating biological macromolecules or polypeptides.

The present invention further provides compositions related to producing, detecting, or characterizing such polypeptides, or fragment thereof, such as nucleic acids, vectors, host cells, and the like. Such compositions may serve as compounds that modulate the expression and/or activity of one or more biomarkers described herein or, for example, listed in Table 1.

An isolated polypeptide or a fragment thereof (or a nucleic acid encoding such a polypeptide) corresponding to one or more biomarkers of the invention, including the biomarkers listed in Table 1 or fragments thereof, can be used as an immunogen to generate antibodies that bind to said immunogen, using standard techniques for polyclonal and monoclonal antibody preparation according to well-known methods in the art. An antigenic peptide comprises at least 8 amino acid residues and encompasses an epitope present in the respective full length molecule such that an antibody raised against the peptide forms a specific immune complex with the respective full length molecule. Preferably, the antigenic peptide comprises at least 10 amino acid residues. In one embodiment such epitopes can be specific for a given polypeptide molecule from one species, such as mouse or human (*i.e*., an antigenic peptide that spans a region of the polypeptide molecule that is not conserved across species is used as immunogen; such non conserved residues can be determined using an alignment such as that provided herein).

In one embodiment, an antibody binds substantially specifically to PD-L1 and inhibits or blocks its immunoinhibitory function, such as by interrupting its interaction with an inhibitory receptor like PD-1. In another embodiment, an antibody binds substantially specifically to TIM-3 and inhibits or blocks its immunoinhibitory function, such as by interrupting its interaction with galectin-9 or phosphatidylserine.

For example, a polypeptide immunogen typically is used to prepare antibodies by immunizing a suitable subject (*e.g.,* rabbit, goat, mouse or other mammal) with the immunogen. An appropriate immunogenic preparation can contain, for example, a recombinantly expressed or chemically synthesized molecule or fragment thereof to which the immune response is to be generated. The preparation can further include an adjuvant, such as Freund's complete or incomplete adjuvant, or similar immunostimulatory agent. Immunization of a suitable subject with an immunogenic preparation induces a polyclonal antibody response to the antigenic peptide contained therein.

Polyclonal antibodies can be prepared as described above by immunizing a suitable subject with a polypeptide immunogen. The polypeptide antibody titer in the immunized subject can be monitored over time by standard techniques, such as with an enzyme linked immunosorbent assay (ELISA) using immobilized polypeptide. If desired, the antibody directed against the antigen can be isolated from the mammal (*e.g.,* from the blood) and further purified by well-known techniques, such as protein A chromatography, to obtain the IgG fraction. At an appropriate time after immunization, *e.g.,* when the antibody titers are highest, antibody-producing cells can be obtained from the subject and used to prepare monoclonal antibodies by standard techniques, such as the hybridoma technique (originally described by Kohler and Milstein (1975) Nature 256:495-497) (see also Brown et al. (1981) J. Immunol. 127:539-46; Brown et al. (1980) J. Biol. Chem. 255:4980-83; Yeh et al. (1976) Proc. Natl. Acad. Sci. 76:2927-31; Yeh et al. (1982) Int. J. Cancer 29:269-75), the more recent human B cell hybridoma technique (Kozbor et al. (1983) Immunol. Today 4:72), the EBV-hybridoma technique (Cole et al. (1985) Monoclonal Antibodies and Cancer Therapy, Alan R. Liss, Inc., pp. 77-96) or trioma techniques. The technology for producing monoclonal antibody hybridomas is well known (see generally Kenneth, R. H. in Monoclonal Antibodies: A New Dimension In Biological Analyses, Plenum Publishing Corp., New York, New York (1980); Lerner, E. A. (1981) Yale J. Biol. Med. 54:387-402; Gefter, M. L. et al. (1977) Somatic Cell Genet. 3:231-36). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds to the polypeptide antigen, preferably specifically.

Any of the many well-known protocols used for fusing lymphocytes and immortalized cell lines can be applied for the purpose of generating a monoclonal antibody against one or more biomarkers of the invention, including the biomarkers listed in Table 1, or a fragment thereof (see, *e.g.,* Galfre, G. et al. (1977) Nature 266:55052; Gefter *et al.* (1977) *supra*; Lerner (1981) *supra*; Kenneth (1980) *supra*). Moreover, the ordinary skilled worker will appreciate that there are many variations of such methods which also would be useful. Typically, the immortal cell line (*e.g.,* a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g*., the P3-NS1/1-Ag4-1, P3-x63-Ag8.653 or Sp2/O-Ag14 myeloma lines. These myeloma lines are available from the American Type Culture Collection (ATCC), Rockville, MD. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind a given polypeptide, *e.g*., using a standard ELISA assay.

As an alternative to preparing monoclonal antibody-secreting hybridomas, a monoclonal specific for one of the above described polypeptides can be identified and isolated by screening a recombinant combinatorial immunoglobulin library (*e.g*., an antibody phage display library) with the appropriate polypeptide to thereby isolate immunoglobulin library members that bind the polypeptide. Kits for generating and screening phage display libraries are commercially available (*e.g.,* the Pharmacia *Recombinant Phage Antibody System,* Catalog No. 27-9400-01; and the Stratagene *SurfZAP*™ *Phage Display Kit,* Catalog No. 240612). Additionally, examples of methods and reagents particularly amenable for use in generating and screening an antibody display library can be found in, for example, Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Biotechnology (NY) 9:1369-1372; Hay et al. (1992) Hum. Antibod. Hybridomas 3:81-85; Huse et al. (1989) Science 246:1275-1281; Griffiths et al. (1993) EMBO J. 12:725-734; Hawkins et al. (1992) J. Mol. Biol. 226:889-896; Clarkson et al. (1991) Nature 352:624-628; Gram et al. (1992) Proc. Natl. Acad. Sci. USA 89:3576-3580; Garrard et al. (1991) Biotechnology (NY) 9:1373-1377; Hoogenboom et al. (1991) Nucleic Acids Res. 19:4133-4137; Barbas et al. (1991) Proc. Natl. Acad. Sci. USA 88:7978-7982; and McCafferty et al. (1990) Nature 348:552-554.

Since it is well known in the art that antibody heavy and light chain CDR3 domains play a particularly important role in the binding specificity/affinity of an antibody for an antigen, the recombinant monoclonal antibodies of the present invention prepared as set forth above preferably comprise the heavy and light chain CDR3s of variable regions of the antibodies described herein and well known in the art. Similarly, the antibodies can further comprise the CDR2s of variable regions of said antibodies. The antibodies can further comprise the CDR1s of variable regions of said antibodies. In other embodiments, the antibodies can comprise any combinations of the CDRs.

The CDR1, 2, and/or 3 regions of the engineered antibodies described above can comprise the exact amino acid sequence(s) as those of variable regions of the present invention disclosed herein. However, the ordinarily skilled artisan will appreciate that some deviation from the exact CDR sequences may be possible while still retaining the ability of the antibody to bind a desired target, such as PD-L1, TIM-3, or LAG-3 effectively (*e.g*., conservative sequence modifications). Accordingly, in another embodiment, the engineered antibody may be composed of one or more CDRs that are, for example, 50%, 60%, 70%, 80%, 85%, 90%, 91%, 92%, 93%, 94%, 95%, 96%, 97%, 98%, 99%, or 99.5% identical to one or more CDRs of the present invention described herein or otherwise publicly available.

The structural features of non-human or human antibodies (*e.g*., a rat anti-mouse/antihuman PD-L1 antibody) can be used to create structurally related human antibodies that retain at least one functional property of the antibodies of the present invention, such as binding to PD-L1, TIM-3, or LAG-3. Another functional property includes inhibiting binding of the original known, non-human or human antibodies in a competition ELISA assay.

In some embodiments, monoclonal antibodies capable of binding and inhibiting/blocking PD-L1, TIM-3, and/or LAG-3 are provided, comprising a heavy chain wherein the variable domain comprises at least a CDR having a sequence that is at least 80%, 85%, 90%, 91%, 92%, 93%, 94%, 95%, 96%, 97%, 98%, 99%, 99.5% or 100% identical from the group of heavy chain variable domain CDRs presented herein or otherwise publicly available.

Similarly, monoclonal antibodies binding and inhibiting/blocking PD-L1, TIM-3, and/or LAG-3, comprising a light chain wherein the variable domain comprises at least a CDR having a sequence that is at least 80%, 85%, 90%, 91%, 92%, 93%, 94%, 95%, 96%, 97%, 98%, 99%, 99.5% or 100% identical from the group of light chain variable domain CDRs presented herein or otherwise publicly available, are also provided.

Monoclonal antibodies capable of binding and inhibiting/blocking PD-L1, TIM-3, and/or LAG-3, comprising a heavy chain wherein the variable domain comprises at least a CDR having a sequence that is at least 80%, 85%, 90%, 91%, 92%, 93%, 94%, 95%, 96%, 97%, 98%, 99%, 99.5% or 100% identical from the group of heavy chain variable domain CDRs presented herein or otherwise publicly available; and comprising a light chain wherein the variable domain comprises at least a CDR having a sequence that is at least 80%, 85%, 90%, 91%, 92%, 93%, 94%, 95%, 96%, 97%, 98%, 99%, 99.5% or 100% identical from the group of light chain variable domain CDRs presented herein or otherwise publicly available, are also provided.

A skilled artisan will note that such percentage homology is equivalent to and can be achieved by introducing 1, 2, 3, 4, 5, 6, 7, 8, 9, 10, or more conservative amino acid substitutions within a given CDR.

The monoclonal antibodies of the present invention can comprise a heavy chain, wherein the variable domain comprises at least a CDR having a sequence selected from the group consisting of the heavy chain variable domain CDRs presented herein or otherwise publicly available and a light chain, wherein the variable domain comprises at least a CDR having a sequence selected from the group consisting of the light chain variable domain CDRs presented herein or otherwise publicly available.

Such monoclonal antibodies can comprise a light chain, wherein the variable domain comprises at least a CDR having a sequence selected from the group consisting of CDR-L1, CDR-L2, and CDR-L3, as described herein; and/or a heavy chain, wherein the variable domain comprises at least a CDR having a sequence selected from the group consisting of CDR-H1, CDR-H2, and CDR-H3, as described herein. In some embodiments, the monoclonal antibodies capable of binding human Gall comprises or consists of CDR-L1, CDR-L2, CDR-L3, CDR-H1, CDR-H2, and CDR-H3, as described herein.

The heavy chain variable domain of the monoclonal antibodies of the present invention can comprise or consist of the vH amino acid sequence set forth herein or otherwise publicly available and/or the light chain variable domain of the monoclonal antibodies of the present invention can comprise or consist of the vκ amino acid sequence set forth herein or otherwise publicly available.

The present invention further provides fragments of said monoclonal antibodies which include, but are not limited to, Fv, Fab, F(ab')2, Fab', dsFv, scFv, sc(Fv)2 and diabodies; and multispecific antibodies formed from antibody fragments.

Other fragments of the monoclonal antibodies of the present invention are also contemplated. For example, individual immunoglobulin heavy and/or light chains are provided, wherein the variable domains thereof comprise at least a CDR presented herein or otherwise publicly available. In one embodiment, the immunoglobulin heavy chain comprises at least a CDR having a sequence that is at least 80%, 85%, 90%, 91%, 92%, 93%, 94%, 95%, 96%, 97%, 98%, 99%, 99.5% or 100% identical from the group of heavy chain or light chain variable domain CDRs presented herein or otherwise publicly available. In another embodiment, an immunoglobulin light chain comprises at least a CDR having a sequence that is at least 80%, 85%, 90%, 91%, 92%, 93%, 94%, 95%, 96%, 97%, 98%, 99%, 99.5% or 100% identical from the group of light chain or heavy chain variable domain CDRs presented herein or otherwise publicly available, are also provided.

In some embodiments, the immunoglobulin heavy and/or light chain comprises a variable domain comprising at least one of CDR-L1, CDR-L2, CDR-L3, CDR-H1, CDR-H2, or CDR-H3 described herein. Such immunoglobulin heavy chains can comprise or consist of at least one of CDR-H1, CDR-H2, and CDR-H3. Such immunoglobulin light chains can comprise or consist of at least one of CDR-L1, CDR-L2, and CDR-L3.

In other embodiments, an immunoglobulin heavy and/or light chain according to the present invention comprises or consists of a vH or vκ variable domain sequence, respectively, provided herein or otherwise publicly available.

The present invention further provides polypeptides which have a sequence selected from the group consisting of vH variable domain, vκ variable domain, CDR-L1, CDR-L2, CDR-L3, CDR-H1, CDR-H2, and CDR-H3 sequences described herein.

Antibodies, immunoglobulins, and polypeptides of the invention can be used in an isolated (*e.g.,* purified) form or contained in a vector, such as a membrane or lipid vesicle (*e.g.* a liposome).

Amino acid sequence modification(s) of the antibodies described herein are contemplated. For example, it may be desirable to improve the binding affinity and/or other biological properties of the antibody. It is known that when a humanized antibody is produced by simply grafting only CDRs in VH and VL of an antibody derived from a non-human animal in FRs of the VH and VL of a human antibody, the antigen binding activity is reduced in comparison with that of the original antibody derived from a non-human animal. It is considered that several amino acid residues of the VH and VL of the non-human antibody, not only in CDRs but also in FRs, are directly or indirectly associated with the antigen binding activity. Hence, substitution of these amino acid residues with different amino acid residues derived from FRs of the VH and VL of the human antibody would reduce binding activity and can be corrected by replacing the amino acids with amino acid residues of the original antibody derived from a non-human animal.

Modifications and changes may be made in the structure of the antibodies described herein, and in the DNA sequences encoding them, and still obtain a functional molecule that encodes an antibody and polypeptide with desirable characteristics. For example, certain amino acids may be substituted by other amino acids in a protein structure without appreciable loss of activity. Since the interactive capacity and nature of a protein define the protein's biological functional activity, certain amino acid substitutions can be made in a protein sequence, and, of course, in its DNA encoding sequence, while nevertheless obtaining a protein with like properties. It is thus contemplated that various changes may be made in the antibodies sequences of the invention, or corresponding DNA sequences which encode said polypeptides, without appreciable loss of their biological activity.

In making the changes in the amino sequences of polypeptide, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (<RTI 3.5); asparagine (-3.5); lysine (-3.9); and arginine (-4.5).

It is known in the art that certain amino acids may be substituted by other amino acids having a similar hydropathic index or score and still result in a protein with similar biological activity, i.e. still obtain a biological functionally equivalent protein.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

Another type of amino acid modification of the antibody of the invention may be useful for altering the original glycosylation pattern of the antibody to, for example, increase stability. By "altering" is meant deleting one or more carbohydrate moieties found in the antibody, and/or adding one or more glycosylation sites that are not present in the antibody. Glycosylation of antibodies is typically N-linked. "N-linked" refers to the attachment of the carbohydrate moiety to the side chain of an asparagine residue. The tripeptide sequences asparagine-X-serine and asparagines-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. Addition of glycosylation sites to the antibody is conveniently accomplished by altering the amino acid sequence such that it contains one or more of the above-described tripeptide sequences (for N-linked glycosylation sites). Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, orhydroxyproline, (e) aromatic residues such as those of phenylalanine, tyrosine, or tryptophan, or (f) the amide group of glutamine. For example, such methods are described in WO87/05330.

Similarly, removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by *Sojahr et al.* (1987) and by Edge *et al.* (1981). Enzymatic cleavage of carbohydrate moieties on antibodies can be achieved by the use of a variety of endo- and exo-glycosidases as described by Thotakura *et al.* (1987).

Other modifications can involve the formation of immunoconjugates. For example, in one type of covalent modification, antibodies or proteins are covalently linked to one of a variety of non proteinaceous polymers, *e.g*., polyethylene glycol, polypropylene glycol, or polyoxyalkylenes, in the manner set forth in U.S. Pat. No. 4,640,835; 4,496,689; 4,301,144; 4,670,417; 4,791,192 or 4,179,337.

Conjugation of antibodies or other proteins of the present invention with heterologous agents can be made using a variety of bifunctional protein coupling agents including but not limited to N-succinimidyl (2-pyridyldithio) propionate (SPDP), succinimidyl (N-maleimidomethyl)cyclohexane-1-carboxylate, iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6diisocyanate), and bis-active fluorine compounds (such as 1,5-difluoro-2,4-dinitrobenzene). For example, carbon labeled 1-isothiocyanatobenzyl methyldiethylene triaminepentaacetic acid (MX-DTPA) is an exemplary chelating agent for conjugation of radionucleotide to the antibody (WO 94/11026).

In another aspect, the present invention features antibodies conjugated to a therapeutic moiety, such as a cytotoxin, a drug, and/or a radioisotope. When conjugated to a cytotoxin, these antibody conjugates are referred to as "immunotoxins." A cytotoxin or cytotoxic agent includes any agent that is detrimental to (*e.g.,* kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g.*, vincristine and vinblastine). An antibody of the present invention can be conjugated to a radioisotope, *e.g*., radioactive iodine, to generate cytotoxic radiopharmaceuticals for treating a related disorder, such as a cancer.

Conjugated antibodies, in addition to therapeutic utility, can be useful for diagnostically or prognostically to monitor polypeptide levels in tissue as part of a clinical testing procedure, *e.g.,* to determine the efficacy of a given treatment regimen. Detection can be facilitated by coupling (*i e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate (FITC), rhodamine, dichlorotriazinylamine fluorescein, dansyl chloride or phycoerythrin (PE); an example of a luminescent material includes luminol; examples of bioluminescent materials include luciferase, luciferin, and aequorin, and examples of suitable radioactive material include ¹²⁵I, ¹³¹I, ³⁵S, or ³H. [0134] As used herein, the term "labeled", with regard to the antibody, is intended to encompass direct labeling of the antibody by coupling (*i.e.,* physically linking) a detectable substance, such as a radioactive agent or a fluorophore (*e.g.* fluorescein isothiocyanate (FITC) or phycoerythrin (PE) or Indocyanine (Cy5)) to the antibody, as well as indirect labeling of the antibody by reactivity with a detectable substance.

The antibody conjugates of the present invention can be used to modify a given biological response. The therapeutic moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, *Pseudomonas* exotoxin, or diphtheria toxin; a protein such as tumor necrosis factor or interferon-.gamma.; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other cytokines or growth factors.

Techniques for conjugating such therapeutic moiety to antibodies are well known, see, *e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243 56 (Alan R. Liss, Inc. 1985); Hellstrom et al., "Antibodies For Drug Delivery", in Controlled Drug Delivery (2nd Ed.), Robinson et al. (eds.), pp. 623 53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475 506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303 16 (Academic Press 1985), and Thorpe et al., "The Preparation And Cytotoxic Properties Of Antibody-Toxin Conjugates", Immunol. Rev., 62:119 58 (1982).

In some embodiments, conjugations can be made using a "cleavable linker" facilitating release of the cytotoxic agent or growth inhibitory agent in a cell. For example, an acid-labile linker, peptidase-sensitive linker, photolabile linker, dimethyl linker or disulfide-containing linker (See *e.g.* U.S. Pat. No. 5,208,020) may be used. Alternatively, a fusion protein comprising the antibody and cytotoxic agent or growth inhibitory agent may be made, by recombinant techniques or peptide synthesis. The length of DNA may comprise respective regions encoding the two portions of the conjugate either adjacent one another or separated by a region encoding a linker peptide which does not destroy the desired properties of the conjugate.

Additionally, recombinant polypeptide antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson et al. International Patent Publication PCT/US86/02269; Akira *et al.* European Patent Application 184,187; Taniguchi, M. European Patent Application 171,496; Morrison *et al.* European Patent Application 173,494; Neuberger et al. PCT Application WO 86/01533; Cabilly et al. U.S. Patent No. 4,816,567; Cabilly *et al.* European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. 84:214-218; Nishimura et al. (1987) Cancer Res. 47:999-1005; Wood et al. (1985) Nature 314:446-449; Shaw et al. (1988) J. Natl. Cancer Inst. 80:1553-1559); Morrison, S. L. (1985) Science 229:1202-1207; Oi et al. (1986) Biotechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

In addition, humanized antibodies can be made according to standard protocols such as those disclosed in U.S. Patent 5,565,332. In another embodiment, antibody chains or specific binding pair members can be produced by recombination between vectors comprising nucleic acid molecules encoding a fusion of a polypeptide chain of a specific binding pair member and a component of a replicable generic display package and vectors containing nucleic acid molecules encoding a second polypeptide chain of a single binding pair member using techniques known in the art, *e.g.,* as described in U.S. Patents 5,565,332, 5,871,907, or 5,733,743. The use of intracellular antibodies to inhibit protein function in a cell is also known in the art (see *e.g.,* Carlson, J. R. (1988) Mol. Cell. Biol. 8:2638-2646; Biocca, S. et al. (1990) EMBO J. 9:101-108; Werge, T. M. et al. (1990) FEBS Lett. 274:193-198; Carlson, J. R. (1993) Proc. Natl. Acad. Sci. USA 90:7427-7428; Marasco, W. A. et al. (1993) Proc. Natl. Acad. Sci. USA 90:7889-7893; Biocca, S. et al. (1994) Biotechnology (NY) 12:396-399; Chen, S-Y. et al. (1994) Hum. Gene Ther. 5:595-601; Duan, L et al. (1994) Proc. Natl. Acad. Sci. USA 91:5075-5079; Chen, S-Y. et al. (1994) Proc. Natl. Acad. Sci. USA 91:5932-5936; Beerli, R. R. et al. (1994) J. Biol. Chem. 269:23931-23936; Beerli, R. R. et al. (1994) Biochem. Biophys. Res. Commun. 204:666-672; Mhashilkar, A. M. etal. (1995) EMBO J. 14:1542-1551; Richardson, J. H. et al. (1995) Proc. Natl. Acad. Sci. USA 92:3137-3141; PCT Publication No. WO 94/02610 by Marasco et al.; and PCT Publication No. WO 95/03832 by Duan et al.).

Additionally, fully human antibodies could be made against biomarkers of the invention, including the biomarkers listed in Table 1, or fragments thereof. Fully human antibodies can be made in mice that are transgenic for human immunoglobulin genes, *e.g.* according to Hogan, et al., "Manipulating the Mouse Embryo: A Laboratory Manuel," Cold Spring Harbor Laboratory. Briefly, transgenic mice are immunized with purified immunogen. Spleen cells are harvested and fused to myeloma cells to produce hybridomas. Hybridomas are selected based on their ability to produce antibodies which bind to the immunogen. Fully human antibodies would reduce the immunogenicity of such antibodies in a human.

In one embodiment, an antibody for use in the instant invention is a bispecific or multispecific antibody. A bispecific antibody has binding sites for two different antigens within a single antibody polypeptide. Antigen binding may be simultaneous or sequential. Triomas and hybrid hybridomas are two examples of cell lines that can secrete bispecific antibodies. Examples of bispecific antibodies produced by a hybrid hybridoma or a trioma are disclosed in U.S. Patent 4,474,893. Bispecific antibodies have been constructed by chemical means (Staerz et al. (1985) Nature 314:628, and Perez et al. (1985) Nature 316:354) and hybridoma technology (Staerz and Bevan (1986) Proc. Natl. Acad. Sci. USA, 83:1453, and Staerz and Bevan (1986) Immunol. Today 7:241). Bispecific antibodies are also described in U.S. Patent 5,959,084. Fragments of bispecific antibodies are described in U.S. Patent 5,798,229.

Bispecific agents can also be generated by making heterohybridomas by fusing hybridomas or other cells making different antibodies, followed by identification of clones producing and co-assembling both antibodies. They can also be generated by chemical or genetic conjugation of complete immunoglobulin chains or portions thereof such as Fab and Fv sequences. The antibody component can bind to a polypeptide or a fragment thereof of one or more biomarkers of the invention, including one or more biomarkers listed in Table 1, or a fragment thereof. In one embodiment, the bispecific antibody could specifically bind to both a polypeptide or a fragment thereof and its natural binding partner(s) or a fragment(s) thereof.

In another aspect of this invention, peptides or peptide mimetics can be used to antagonize the activity of one or more biomarkers of the invention, including one or more biomarkers listed in Table 1, or a fragment(s) thereof. In one embodiment, variants of one or more biomarkers listed in Table 1 which function as a modulating agent for the respective full length protein, can be identified by screening combinatorial libraries of mutants, *e.g*., truncation mutants, for antagonist activity. In one embodiment, a variegated library of variants is generated by combinatorial mutagenesis at the nucleic acid level and is encoded by a variegated gene library. A variegated library of variants can be produced, for instance, by enzymatically ligating a mixture of synthetic oligonucleotides into gene sequences such that a degenerate set of potential polypeptide sequences is expressible as individual polypeptides containing the set of polypeptide sequences therein. There are a variety of methods which can be used to produce libraries of polypeptide variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential polypeptide sequences. Methods for synthesizing degenerate oligonucleotides are known in the art (see, *e.g.,* Narang, S. A. (1983) Tetrahedron 39:3; Itakura et al. (1984) Annu. Rev. Biochem. 53:323; Itakura et al. (1984) Science 198:1056; Ike et al. (1983) Nucleic Acid Res. 11:477.

In addition, libraries of fragments of a polypeptide coding sequence can be used to generate a variegated population of polypeptide fragments for screening and subsequent selection of variants of a given polypeptide. In one embodiment, a library of coding sequence fragments can be generated by treating a double stranded PCR fragment of a polypeptide coding sequence with a nuclease under conditions wherein nicking occurs only about once per polypeptide, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the polypeptide.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of polypeptides. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of interest (Arkin and Youvan (1992) Proc. Natl. Acad. Sci. USA 89:7811-7815; Delagrave et al. (1993) Protein Eng. 6(3):327-331). In one embodiment, cell based assays can be exploited to analyze a variegated polypeptide library. For example, a library of expression vectors can be transfected into a cell line which ordinarily synthesizes one or more biomarkers of the invention, including one or more biomarkers listed in Table 1, or a fragment thereof. The transfected cells are then cultured such that the full length polypeptide and a particular mutant polypeptide are produced and the effect of expression of the mutant on the full length polypeptide activity in cell supernatants can be detected, *e.g*., by any of a number of functional assays. Plasmid DNA can then be recovered from the cells which score for inhibition, or alternatively, potentiation of full length polypeptide activity, and the individual clones further characterized.

Systematic substitution of one or more amino acids of a polypeptide amino acid sequence with a D-amino acid of the same type (*e.g.,* D-lysine in place of L-lysine) can be used to generate more stable peptides. In addition, constrained peptides comprising a polypeptide amino acid sequence of interest or a substantially identical sequence variation can be generated by methods known in the art (Rizo and Gierasch (1992) Annu. Rev. Biochem. 61:387, incorporated herein by reference); for example, by adding internal cysteine residues capable of forming intramolecular disulfide bridges which cyclize the peptide.

The amino acid sequences disclosed herein will enable those of skill in the art to produce polypeptides corresponding peptide sequences and sequence variants thereof. Such polypeptides can be produced in prokaryotic or eukaryotic host cells by expression of polynucleotides encoding the peptide sequence, frequently as part of a larger polypeptide. Alternatively, such peptides can be synthesized by chemical methods. Methods for expression of heterologous proteins in recombinant hosts, chemical synthesis of polypeptides, and *in vitro* translation are well known in the art and are described further in Maniatis et al. Molecular Cloning: A Laboratory Manual (1989), 2nd Ed., Cold Spring Harbor, N.Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11: 255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing, which are incorporated herein by reference).

Peptides can be produced, typically by direct chemical synthesis. Peptides can be produced as modified peptides, with nonpeptide moieties attached by covalent linkage to the N-terminus and/or C-terminus. In certain preferred embodiments, either the carboxy-terminus or the amino-terminus, or both, are chemically modified. The most common modifications of the terminal amino and carboxyl groups are acetylation and amidation, respectively. Amino-terminal modifications such as acylation (*e.g.,* acetylation) or alkylation (*e.g.,* methylation) and carboxy-terminal-modifications such as amidation, as well as other terminal modifications, including cyclization, can be incorporated into various embodiments of the invention. Certain amino-terminal and/or carboxy-terminal modifications and/or peptide extensions to the core sequence can provide advantageous physical, chemical, biochemical, and pharmacological properties, such as: enhanced stability, increased potency and/or efficacy, resistance to serum proteases, desirable pharmacokinetic properties, and others. Peptides disclosed herein can be used therapeutically to treat disease, *e.g*., by altering costimulation in a patient.

Peptidomimetics (Fauchere (1986) Adv. Drug Res. 15:29; Veber and Freidinger (1985) TINS p.392; and Evans et al. (1987) J. Med. Chem. 30:1229, which are incorporated herein by reference) are usually developed with the aid of computerized molecular modeling. Peptide mimetics that are structurally similar to therapeutically useful peptides can be used to produce an equivalent therapeutic or prophylactic effect. Generally, peptidomimetics are structurally similar to a paradigm polypeptide (*i.e.,* a polypeptide that has a biological or pharmacological activity), but have one or more peptide linkages optionally replaced by a linkage selected from the group consisting of: -CH2NH-, -CH2S-, -CH2-CH2-, -CH=CH- (cis and trans), -COCH2-, - CH(OH)CH2-, and -CH2SO-, by methods known in the art and further described in the following references: Spatola, A. F. in "Chemistry and Biochemistry of Amino Acids, Peptides, and Proteins" Weinstein, B., ed., Marcel Dekker, New York, p. 267 (1983); Spatola, A. F., Vega Data (March 1983), Vol. 1, Issue 3, "Peptide Backbone Modifications" (general review); Morley, J. S. (1980) Trends Pharm. Sci. pp. 463-468 (general review); Hudson, D. et al. (1979) Int. J. Pept. Prot. Res. 14:177-185 (-CH2NH-, CH2CH2-); Spatola, A. F. et al. (1986) Life Sci. 38:1243-1249 (-CH2-S); Hann, M. M. (1982) J. Chem. Soc. Perkin Trans. I. 307-314 (-CH-CH-, cis and trans); Almquist, R. G. et al. (190) J. Med. Chem. 23:1392-1398 (-COCH2-); Jennings-White, C. et al. (1982) Tetrahedron Lett. 23:2533 (-COCH2-); Szelke, M. *et al.* European Appln. EP 45665 (1982) CA: 97:39405 (1982)(-CH(OH)CH2-); Holladay, M. W. et al. (1983) Tetrahedron Lett. (1983) 24:4401-4404 (-C(OH)CH2-); and Hruby, V. J. (1982) Life Sci. (1982) 31:189-199 (-CH2-S-); each of which is incorporated herein by reference. A particularly preferred non-peptide linkage is -CH2NH-. Such peptide mimetics may have significant advantages over polypeptide embodiments, including, for example: more economical production, greater chemical stability, enhanced pharmacological properties (half-life, absorption, potency, efficacy, etc.), altered specificity (*e.g.,* a broad-spectrum of biological activities), reduced antigenicity, and others. Labeling of peptidomimetics usually involves covalent attachment of one or more labels, directly or through a spacer (*e.g*., an amide group), to non-interfering position(s) on the peptidomimetic that are predicted by quantitative structure-activity data and/or molecular modeling. Such non-interfering positions generally are positions that do not form direct contacts with the macropolypeptides(s) to which the peptidomimetic binds to produce the therapeutic effect. Derivatization (*e.g*., labeling) of peptidomimetics should not substantially interfere with the desired biological or pharmacological activity of the peptidomimetic.

Also encompassed by the present invention are small molecules which can modulate (either enhance or inhibit) interactions, e.g., between biomarkers described herein or listed in Table 1 and their natural binding partners. The small molecules of the present invention can be obtained using any of the numerous approaches in combinatorial library methods known in the art, including: spatially addressable parallel solid phase or solution phase libraries; synthetic library methods requiring deconvolution; the 'one-bead one-compound' library method; and synthetic library methods using affinity chromatography selection. (Lam, K. S. (1997) Anticancer Drug Des. 12:145).

Examples of methods for the synthesis of molecular libraries can be found in the art, for example in: DeWitt et al. (1993) Proc. Natl. Acad. Sci. USA 90:6909; Erb et al. (1994) Proc. Natl. Acad. Sci. USA 91:11422; Zuckermann et al. (1994) J. Med. Chem. 37:2678; Cho et al. (1993) Science 261:1303; Carrell et al. (1994) Angew. Chem. Int. Ed. Engl. 33:2059; Carell etal. (1994) Angew. Chem. Int. Ed. Engl. 33:2061; and in Gallop et al. (1994) J. Med. Chem. 37:1233.

Libraries of compounds can be presented in solution (e.g., Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc. Natl. Acad. Sci. USA 89:1865-1869) or on phage (Scott and Smith (1990) Science 249:386-390); (Devlin (1990) Science 249:404-406); (Cwirla et al. (1990) Proc. Natl. Acad. Sci. USA 87:6378-6382); (Felici (1991) J. Mol. Biol. 222:301-310); (Ladner *supra*.)*.* Compounds can be screened in cell based or non-cell based assays. Compounds can be screened in pools (e.g. multiple compounds in each testing sample) or as individual compounds.

The invention also relates to chimeric or fusion proteins of the biomarkers of the invention, including the biomarkers listed in Table 1, or fragments thereof. As used herein, a "chimeric protein" or "fusion protein" comprises one or more biomarkers of the invention, including one or more biomarkers listed in Table 1, or a fragment thereof, operatively linked to another polypeptide having an amino acid sequence corresponding to a protein which is not substantially homologous to the respective biomarker. In a preferred embodiment, the fusion protein comprises at least one biologically active portion of one or more biomarkers of the invention, including one or more biomarkers listed in Table 1, or fragments thereof. Within the fusion protein, the term "operatively linked" is intended to indicate that the biomarker sequences and the non-biomarker sequences are fused in-frame to each other in such a way as to preserve functions exhibited when expressed independently of the fusion. The "another" sequences can be fused to the N-terminus or C-terminus of the biomarker sequences, respectively.

Such a fusion protein can be produced by recombinant expression of a nucleotide sequence encoding the first peptide and a nucleotide sequence encoding the second peptide. The second peptide may optionally correspond to a moiety that alters the solubility, affinity, stability or valency of the first peptide, for example, an immunoglobulin constant region. In another preferred embodiment, the first peptide consists of a portion of a biologically active molecule (*e.g.* the extracellular portion of the polypeptide or the ligand binding portion). The second peptide can include an immunoglobulin constant region, for example, a human Cγ1 domain or Cy 4 domain (*e.g.,* the hinge, CH2 and CH3 regions of human IgCγ1, or human IgCy4, see *e.g.,* Capon et al. U.S. Patents 5,116,964; 5,580,756; 5,844,095 and the like, incorporated herein by reference). Such constant regions may retain regions which mediate effector function (*e.g.* Fc receptor binding) or may be altered to reduce effector function. A resulting fusion protein may have altered solubility, binding affinity, stability and/or valency (*i.e.,* the number of binding sites available per polypeptide) as compared to the independently expressed first peptide, and may increase the efficiency of protein purification. Fusion proteins and peptides produced by recombinant techniques can be secreted and isolated from a mixture of cells and medium containing the protein or peptide. Alternatively, the protein or peptide can be retained cytoplasmically and the cells harvested, lysed and the protein isolated. A cell culture typically includes host cells, media and other byproducts. Suitable media for cell culture are well known in the art. Protein and peptides can be isolated from cell culture media, host cells, or both using techniques known in the art for purifying proteins and peptides. Techniques for transfecting host cells and purifying proteins and peptides are known in the art.

Preferably, a fusion protein of the invention is produced by standard recombinant DNA techniques. For example, DNA fragments coding for the different polypeptide sequences are ligated together in-frame in accordance with conventional techniques, for example employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992).

Particularly preferred Ig fusion proteins include the extracellular domain portion or variable region-like domain of PD-L1, TIM-3, LAG-3, or other biomarker listed in Table 1, coupled to an immunoglobulin constant region (e.g., the Fc region). The immunoglobulin constant region may contain genetic modifications which reduce or eliminate effector activity inherent in the immunoglobulin structure. For example, DNA encoding the extracellular portion of a polypeptide of interest can be joined to DNA encoding the hinge, CH2 and CH3 regions of human IgGγ1 and/or IgGγ4 modified by site directed mutagenesis, *e.g*., as taught in WO 97/28267.

In another embodiment, the fusion protein contains a heterologous signal sequence at its N-terminus. In certain host cells (*e.g*., mammalian host cells), expression and/or secretion of a polypeptide can be increased through use of a heterologous signal sequence.

The fusion proteins of the invention can be used as immunogens to produce antibodies in a subject. Such antibodies may be used to purify the respective natural polypeptides from which the fusion proteins were generated, or in screening assays to identify polypeptides which inhibit the interactions between one or more biomarkers polypeptide or a fragment thereof and its natural binding partner(s) or a fragment(s) thereof.

Also provided herein are compositions comprising one or more nucleic acids comprising or capable of expressing at least 1, 2, 3, 4, 5, 10, 20 or more small nucleic acids or antisense oligonucleotides or derivatives thereof, wherein said small nucleic acids or antisense oligonucleotides or derivatives thereof in a cell specifically hybridize (*e.g*., bind) under cellular conditions, with cellular nucleic acids (*e.g*., small non-coding RNAS such as miRNAs, pre-miRNAs, pri-miRNAs, miRNA*, anti-miRNA, a miRNA binding site, a variant and/or functional variant thereof, cellular mRNAs or a fragments thereof). In one embodiment, expression of the small nucleic acids or antisense oligonucleotides or derivatives thereof in a cell can inhibit expression or biological activity of cellular nucleic acids and/or proteins, *e.g*., by inhibiting transcription, translation and/or small nucleic acid processing of, for example, one or more biomarkers of the invention, including one or more biomarkers listed in Table 1, or fragment(s) thereof. In one embodiment, the small nucleic acids or antisense oligonucleotides or derivatives thereof are small RNAs (*e.g*., microRNAs) or complements of small RNAs. In another embodiment, the small nucleic acids or antisense oligonucleotides or derivatives thereof can be single or double stranded and are at least six nucleotides in length and are less than about 1000, 900, 800, 700, 600, 500, 400, 300, 200, 100, 50, 40, 30, 25, 24, 23, 22, 21,20, 19, 18, 17, 16, 15, or 10 nucleotides in length. In another embodiment, a composition may comprise a library of nucleic acids comprising or capable of expressing small nucleic acids or antisense oligonucleotides or derivatives thereof, or pools of said small nucleic acids or antisense oligonucleotides or derivatives thereof. A pool of nucleic acids may comprise about 2-5, 5-10, 10-20, 10-30 or more nucleic acids comprising or capable of expressing small nucleic acids or antisense oligonucleotides or derivatives thereof.

In one embodiment, binding may be by conventional base pair complementarity, or, for example, in the case of binding to DNA duplexes, through specific interactions in the major groove of the double helix. In general, "antisense" refers to the range of techniques generally employed in the art, and includes any process that relies on specific binding to oligonucleotide sequences.

It is well known in the art that modifications can be made to the sequence of a miRNA or a pre-miRNA without disrupting miRNA activity. As used herein, the term "functional variant" of a miRNA sequence refers to an oligonucleotide sequence that varies from the natural miRNA sequence, but retains one or more functional characteristics of the miRNA (*e.g*. cancer cell proliferation inhibition, induction of cancer cell apoptosis, enhancement of cancer cell susceptibility to chemotherapeutic agents, specific miRNA target inhibition). In some embodiments, a functional variant of a miRNA sequence retains all of the functional characteristics of the miRNA. In certain embodiments, a functional variant of a miRNA has a nucleobase sequence that is a least about 60%, 65%, 70%, 75%, 80%, 85%, 90%, 91%, 92%, 93%, 94%, 95%, 96%, 97%, 98% or 99% identical to the miRNA or precursor thereof over a region of about 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the functional variant hybridizes to the complement of the miRNA or precursor thereof under stringent hybridization conditions. Accordingly, in certain embodiments the nucleobase sequence of a functional variant is capable of hybridizing to one or more target sequences of the miRNA.

miRNAs and their corresponding stem-loop sequences described herein may be found in miRBase, an online searchable database of miRNA sequences and annotation, found on the world wide web at microrna.sanger.ac.uk. Entries in the miRBase Sequence database represent a predicted hairpin portion of a miRNA transcript (the stem-loop), with information on the location and sequence of the mature miRNA sequence. The miRNA stem-loop sequences in the database are not strictly precursor miRNAs (pre-miRNAs), and may in some instances include the pre-miRNA and some flanking sequence from the presumed primary transcript. The miRNA nucleobase sequences described herein encompass any version of the miRNA, including the sequences described in Release 10.0 of the miRBase sequence database and sequences described in any earlier Release of the miRBase sequence database. A sequence database release may result in the re-naming of certain miRNAs. A sequence database release may result in a variation of a mature miRNA sequence.

In some embodiments, miRNA sequences of the invention may be associated with a second RNA sequence that may be located on the same RNA molecule or on a separate RNA molecule as the miRNA sequence. In such cases, the miRNA sequence may be referred to as the active strand, while the second RNA sequence, which is at least partially complementary to the miRNA sequence, may be referred to as the complementary strand. The active and complementary strands are hybridized to create a double-stranded RNA that is similar to a naturally occurring miRNA precursor. The activity of a miRNA may be optimized by maximizing uptake of the active strand and minimizing uptake of the complementary strand by the miRNA protein complex that regulates gene translation. This can be done through modification and/or design of the complementary strand.

In some embodiments, the complementary strand is modified so that a chemical group other than a phosphate or hydroxyl at its 5' terminus. The presence of the 5' modification apparently eliminates uptake of the complementary strand and subsequently favors uptake of the active strand by the miRNA protein complex. The 5' modification can be any of a variety of molecules known in the art, including NH₂, NHCOCH₃, and biotin.

In another embodiment, the uptake of the complementary strand by the miRNA pathway is reduced by incorporating nucleotides with sugar modifications in the first 2-6 nucleotides of the complementary strand. It should be noted that such sugar modifications can be combined with the 5' terminal modifications described above to further enhance miRNA activities.

In some embodiments, the complementary strand is designed so that nucleotides in the 3' end of the complementary strand are not complementary to the active strand. This results in double-strand hybrid RNAs that are stable at the 3' end of the active strand but relatively unstable at the 5' end of the active strand. This difference in stability enhances the uptake of the active strand by the miRNA pathway, while reducing uptake of the complementary strand, thereby enhancing miRNA activity.

Small nucleic acid and/or antisense constructs of the methods and compositions presented herein can be delivered, for example, as an expression plasmid which, when transcribed in the cell, produces RNA which is complementary to at least a unique portion of cellular nucleic acids (*e.g*., small RNAs, mRNA, and/or genomic DNA). Alternatively, the small nucleic acid molecules can produce RNA which encodes mRNA, miRNA, pre-miRNA, pri-miRNA, miRNA*, anti-miRNA, or a miRNA binding site, or a variant thereof. For example, selection of plasmids suitable for expressing the miRNAs, methods for inserting nucleic acid sequences into the plasmid, and methods of delivering the recombinant plasmid to the cells of interest are within the skill in the art. See, for example, Zeng et al. (2002) Mol. Cell 9:1327-1333; Tuschl (2002), Nat. Biotechnol. 20:446-448; Brummelkamp et al. (2002) Science 296:550-553; Miyagishi et al. (2002) Nat. Biotechnol. 20:497-500; Paddison et al. (2002) Genes Dev. 16:948-958; Lee et al. (2002) Nat. Biotechnol. 20:500-505; and Paul et al. (2002) Nat. Biotechnol. 20:505-508, the entire disclosures of which are herein incorporated by reference.

Alternatively, small nucleic acids and/or antisense constructs are oligonucleotide probes that are generated *ex vivo* and which, when introduced into the cell, results in hybridization with cellular nucleic acids. Such oligonucleotide probes are preferably modified oligonucleotides that are resistant to endogenous nucleases, e.g., exonucleases and/or endonucleases, and are therefore stable *in vivo.* Exemplary nucleic acid molecules for use as small nucleic acids and/or antisense oligonucleotides are phosphoramidate, phosphothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol et al. (1988) BioTechniques 6:958-976; and Stein et al. (1988) Cancer Res 48:2659-2668.

Antisense approaches may involve the design of oligonucleotides (either DNA or RNA) that are complementary to cellular nucleic acids (*e.g*., complementary to biomarkers listed in Table 1). Absolute complementarity is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a nucleic acid (*e.g.,* RNA) it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, *e.g*., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well (Wagner (1994) Nature 372:333). Therefore, oligonucleotides complementary to either the 5' or 3' untranslated, non-coding regions of genes could be used in an antisense approach to inhibit translation of endogenous mRNAs. Oligonucleotides complementary to the 5' untranslated region of the mRNA may include the complement of the AUG start codon. Antisense oligonucleotides complementary to mRNA coding regions are less efficient inhibitors of translation but could also be used in accordance with the methods and compositions presented herein. Whether designed to hybridize to the 5', 3' or coding region of cellular mRNAs, small nucleic acids and/or antisense nucleic acids should be at least six nucleotides in length, and can be less than about 1000, 900, 800, 700, 600, 500, 400, 300, 200, 100, 50, 40, 30, 25, 24, 23, 22, 21,20, 19, 18, 17, 16, 15, or 10 nucleotides in length.

Regardless of the choice of target sequence, it is preferred that *in vitro* studies are first performed to quantitate the ability of the antisense oligonucleotide to inhibit gene expression. In one embodiment these studies utilize controls that distinguish between antisense gene inhibition and nonspecific biological effects of oligonucleotides. In another embodiment these studies compare levels of the target nucleic acid or protein with that of an internal control nucleic acid or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

Small nucleic acids and/or antisense oligonucleotides can be DNA or RNA or chimeric mixtures or derivatives or modified versions thereof, single-stranded or double-stranded. Small nucleic acids and/or antisense oligonucleotides can be modified at the base moiety, sugar moiety, or phosphate backbone, for example, to improve stability of the molecule, hybridization, etc., and may include other appended groups such as peptides (*e.g.,* for targeting host cell receptors), or agents facilitating transport across the cell membrane (see, *e.g.,* Letsinger et al. (1989) Proc. Natl. Acad. Sci. U.S.A. 86:6553-6556; Lemaitre et al. (1987) Proc. Natl. Acad. Sci. U.S.A. 84:648-652; PCT Publication No. WO88/09810) or the blood-brain barrier (see, *e.g.,* PCT Publication No. WO89/10134), hybridization-triggered cleavage agents. (See, *e.g.,* Krol et al. (1988) BioTech. 6:958-976) or intercalating agents. (See, *e.g.,* Zon (1988) Pharm. Res. 5:539-549). To this end, small nucleic acids and/or antisense oligonucleotides may be conjugated to another molecule, *e.g.,* a peptide, hybridization triggered cross-linking agent, transport agent, hybridization-triggered cleavage agent, etc.

Small nucleic acids and/or antisense oligonucleotides may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytiethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Small nucleic acids and/or antisense oligonucleotides may also comprise at least one modified sugar moiety selected from the group including but not limited to arabinose, 2-fluoroarabinose, xylulose, and hexose.

In certain embodiments, a compound comprises an oligonucleotide (*e.g*., a miRNA or miRNA encoding oligonucleotide) conjugated to one or more moieties which enhance the activity, cellular distribution or cellular uptake of the resulting oligonucleotide. In certain such embodiments, the moiety is a cholesterol moiety (*e.g*., antagomirs) or a lipid moiety or liposome conjugate. Additional moieties for conjugation include carbohydrates, phospholipids, biotin, phenazine, folate, phenanthridine, anthraquinone, acridine, fluoresceins, rhodamines, coumarins, and dyes. In certain embodiments, a conjugate group is attached directly to the oligonucleotide. In certain embodiments, a conjugate group is attached to the oligonucleotide by a linking moiety selected from amino, hydroxyl, carboxylic acid, thiol, unsaturations (*e.g*., double or triple bonds), 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C1-C10 alkyl, substituted or unsubstituted C2-C10 alkenyl, and substituted or unsubstituted C2-C10 alkynyl. In certain such embodiments, a substituent group is selected from hydroxyl, amino, alkoxy, carboxy, benzyl, phenyl, nitro, thiol, thioalkoxy, halogen, alkyl, aryl, alkenyl and alkynyl.

In certain such embodiments, the compound comprises the oligonucleotide having one or more stabilizing groups that are attached to one or both termini of the oligonucleotide to enhance properties such as, for example, nuclease stability. Included in stabilizing groups are cap structures. These terminal modifications protect the oligonucleotide from exonuclease degradation, and can help in delivery and/or localization within a cell. The cap can be present at the 5'-terminus (5'-cap), or at the 3'-terminus (3'-cap), or can be present on both termini. Cap structures include, for example, inverted deoxy abasic caps.

Suitable cap structures include a 4',5'-methylene nucleotide, a 1-(beta-D-erythrofuranosyl) nucleotide, a 4'-thio nucleotide, a carbocyclic nucleotide, a 1,5-anhydrohexitol nucleotide, an L-nucleotide, an alpha-nucleotide, a modified base nucleotide, a phosphorodithioate linkage, a threo-pentofuranosyl nucleotide, an acyclic 3',4'-seco nucleotide, an acyclic 3,4-dihydroxybutyl nucleotide, an acyclic 3,5-dihydroxypentyl nucleotide, a 3'-3'-inverted nucleotide moiety, a 3'-3'-inverted abasic moiety, a 3'-2'-inverted nucleotide moiety, a 3'-2'-inverted abasic moiety, a 1,4-butanediol phosphate, a 3'-phosphoramidate, a hexylphosphate, an aminohexyl phosphate, a 3'-phosphate, a 3'-phosphorothioate, a phosphorodithioate, a bridging methylphosphonate moiety, and a non-bridging methylphosphonate moiety 5'-amino-alkyl phosphate, a 1,3-diamino-2-propyl phosphate, 3-aminopropyl phosphate, a 6-aminohexyl phosphate, a 1,2-aminododecyl phosphate, a hydroxypropyl phosphate, a 5'-5'-inverted nucleotide moiety, a 5'-5'-inverted abasic moiety, a 5'-phosphoramidate, a 5'-phosphorothioate, a 5'-amino, a bridging and/or non-bridging 5'-phosphoramidate, a phosphorothioate, and a 5'-mercapto moiety.

Small nucleic acids and/or antisense oligonucleotides can also contain a neutral peptide-like backbone. Such molecules are termed peptide nucleic acid (PNA)-oligomers and are described, *e.g*., in Perry-O'Keefe et al. (1996) Proc. Natl. Acad. Sci. U.S.A. 93:14670 and in Eglom et al. (1993) Nature 365:566. One advantage of PNA oligomers is their capability to bind to complementary DNA essentially independently from the ionic strength of the medium due to the neutral backbone of the DNA. In yet another embodiment, small nucleic acids and/or antisense oligonucleotides comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In a further embodiment, small nucleic acids and/or antisense oligonucleotides are α-anomeric oligonucleotides. An α-anomeric oligonucleotide forms specific double-stranded hybrids with complementary RNA in which, contrary to the usual b-units, the strands run parallel to each other (Gautier et al. (1987) Nucl. Acids Res. 15:6625-6641). The oligonucleotide is a 2'-0-methylribonucleotide (Inoue et al. (1987) Nucl. Acids Res. 15:6131-6148), or a chimeric RNA-DNA analogue (Inoue et al. (1987) FEBS Lett. 215:327-330).

Small nucleic acids and/or antisense oligonucleotides of the methods and compositions presented herein may be synthesized by standard methods known in the art, *e.g.,* by use of an automated DNA synthesizer (such as are commercially available from Biosearch, Applied Biosystems, etc.). As examples, phosphorothioate oligonucleotides may be synthesized by the method of Stein et al. (1988) Nucl. Acids Res. 16:3209, methylphosphonate oligonucleotides can be prepared by use of controlled pore glass polymer supports (Sarin et al. (1988) Proc. Natl. Acad. Sci. U.S.A. 85:7448-7451), etc. For example, an isolated miRNA can be chemically synthesized or recombinantly produced using methods known in the art. In some instances, miRNA are chemically synthesized using appropriately protected ribonucleoside phosphoramidites and a conventional DNA/RNA synthesizer. Commercial suppliers of synthetic RNA molecules or synthesis reagents include, *e.g*., Proligo (Hamburg, Germany), Dharmacon Research (Lafayette, Colo., USA), Pierce Chemical (part of Perbio Science, Rockford, Ill., USA), Glen Research (Sterling, Va., USA), ChemGenes (Ashland, Mass., USA), Cruachem (Glasgow, UK), and Exiqon (Vedbaek, Denmark).

Small nucleic acids and/or antisense oligonucleotides can be delivered to cells *in vivo. A* number of methods have been developed for delivering small nucleic acids and/or antisense oligonucleotides DNA or RNA to cells; *e.g*., antisense molecules can be injected directly into the tissue site, or modified antisense molecules, designed to target the desired cells (*e.g*., antisense linked to peptides or antibodies that specifically bind receptors or antigens expressed on the target cell surface) can be administered systematically.

In one embodiment, small nucleic acids and/or antisense oligonucleotides may comprise or be generated from double stranded small interfering RNAs (siRNAs), in which sequences fully complementary to cellular nucleic acids (*e.g*. mRNAs) sequences mediate degradation or in which sequences incompletely complementary to cellular nucleic acids (*e.g*., mRNAs) mediate translational repression when expressed within cells. In another embodiment, double stranded siRNAs can be processed into single stranded antisense RNAs that bind single stranded cellular RNAs (*e.g*., microRNAs) and inhibit their expression. RNA interference (RNAi) is the process of sequence-specific, post-transcriptional gene silencing in animals and plants, initiated by double-stranded RNA (dsRNA) that is homologous in sequence to the silenced gene. *in vivo,* long dsRNA is cleaved by ribonuclease III to generate 21- and 22-nucleotide siRNAs. It has been shown that 21-nucleotide siRNA duplexes specifically suppress expression of endogenous and heterologous genes in different mammalian cell lines, including human embryonic kidney (293) and HeLa cells (Elbashir et al. (2001) Nature 411:494-498). Accordingly, translation of a gene in a cell can be inhibited by contacting the cell with short double stranded RNAs having a length of about 15 to 30 nucleotides or of about 18 to 21 nucleotides or of about 19 to 21 nucleotides. Alternatively, a vector encoding for such siRNAs or short hairpin RNAs (shRNAs) that are metabolized into siRNAs can be introduced into a target cell (see, *e.g*., McManus et al. (2002) RNA 8:842; Xia et al. (2002) Nature Biotechnology 20:1006; and Brummelkamp et al. (2002) Science 296:550). Vectors that can be used are commercially available, *e.g*., from OligoEngine under the name pSuper RNAi System™.

Ribozyme molecules designed to catalytically cleave cellular mRNA transcripts can also be used to prevent translation of cellular mRNAs and expression of cellular polypeptides, or both (See, *e.g.,* PCT International Publication WO90/11364, published October 4, 1990; Sarver et al. (1990) Science 247:1222-1225 and U.S. Patent No. 5,093,246). While ribozymes that cleave mRNA at site specific recognition sequences can be used to destroy cellular mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach (1988) Nature 334:585-591. The ribozyme may be engineered so that the cleavage recognition site is located near the 5' end of cellular mRNAs; *i.e*., to increase efficiency and minimize the intracellular accumulation of non-functional mRNA transcripts.

The ribozymes of the methods presented herein also include RNA endoribonucleases (hereinafter "Cech-type ribozymes") such as the one which occurs naturally in *Tetrahymena thermophila* (known as the IVS, or L-19 IVS RNA) and which has been extensively described by Thomas Cech and collaborators (Zaug et al. (1984) Science 224:574-578; Zaug et al. (1986) Science 231:470-475; Zaug et al. (1986) Nature 324:429-433; WO 88/04300; and Been et al. (1986) Cell 47:207-216). The Cech-type ribozymes have an eight base pair active site which hybridizes to a target RNA sequence whereafter cleavage of the target RNA takes place. The methods and compositions presented herein encompasses those Cech-type ribozymes which target eight base-pair active site sequences that are present in cellular genes.

As in the antisense approach, the ribozymes can be composed of modified oligonucleotides (*e.g*., for improved stability, targeting, etc.). A preferred method of delivery involves using a DNA construct "encoding" the ribozyme under the control of a strong constitutive pol III or pol II promoter, so that transfected cells will produce sufficient quantities of the ribozyme to destroy endogenous cellular messages and inhibit translation. Because ribozymes unlike antisense molecules, are catalytic, a lower intracellular concentration is required for efficiency.

Nucleic acid molecules to be used in triple helix formation for the inhibition of transcription of cellular genes are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Small nucleic acids (*e.g.*, miRNAs, pre-miRNAs, pri-miRNAs, miRNA*, anti-miRNA, or a miRNA binding site, or a variant thereof), antisense oligonucleotides, ribozymes, and triple helix molecules of the methods and compositions presented herein may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by in vitro and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone. One of skill in the art will readily understand that polypeptides, small nucleic acids, and antisense oligonucleotides can be further linked to another peptide or polypeptide (*e.g*., a heterologous peptide), *e.g.,* that serves as a means of protein detection. Non-limiting examples of label peptide or polypeptide moieties useful for detection in the invention include, without limitation, suitable enzymes such as horseradish peroxidase, alkaline phosphatase, beta-galactosidase, or acetylcholinesterase; epitope tags, such as FLAG, MYC, HA, or HIS tags; fluorophores such as green fluorescent protein; dyes; radioisotopes; digoxygenin; biotin; antibodies; polymers; as well as others known in the art, for example, in Principles of Fluorescence Spectroscopy, Joseph R. Lakowicz (Editor), Plenum Pub Corp, 2nd edition (July 1999).

The modulatory agents described herein (*e.g.,* antibodies, small molecules, peptides, fusion proteins, or small nucleic acids) can be incorporated into pharmaceutical compositions and administered to a subject *in vivo.* The compositions may contain a single such molecule or agent or any combination of agents described herein. "Single active agents" described herein can be combined with other pharmacologically active compounds ("second active agents") known in the art according to the methods and compositions provided herein. It is believed that certain combinations work synergistically in the treatment of conditions that would benefit from the modulation of immune responses. Second active agents can be large molecules (e.g., proteins) or small molecules (*e.g.,* synthetic inorganic, organometallic, or organic molecules). For example, anti-PD-Ll and anti-TIM-3 antibodies can be further combined with anti-LAG-3, anti-PD-1, anti-PD-L2, anti-CTLA4, etc. antibodies or combinations thereof.

Examples of large molecule active agents include, but are not limited to, hematopoietic growth factors, cytokines, and monoclonal and polyclonal antibodies. Typical large molecule active agents are biological molecules, such as naturally occurring or artificially made proteins. Proteins that are particularly useful in this invention include proteins that stimulate the survival and/or proliferation of hematopoietic precursor cells and immunologically active poietic cells *in vitro* or *in vivo.* Others stimulate the division and differentiation of committed erythroid progenitors in cells *in vitro* or *in vivo.* Particular proteins include, but are not limited to: interleukins, such as IL-2 (including recombinant IL-2 ("rIL2") and canarypox IL-2), IL-10, IL-12, and IL-18; interferons, such as interferon alfa-2a, interferon alfa-2b, interferon alpha-nl, interferon alpha-n3, interferon beta-la, and interferon gamma-Ib; GM-CF and GM-CSF; and EPO.

Particular proteins that can be used in the methods and compositions provided herein include, but are not limited to: filgrastim, which is sold in the United States under the trade name Neupogen® (Amgen, Thousand Oaks, Calif.); sargramostim, which is sold in the United States under the trade name Leukine® (Immunex, Seattle, Wash.); and recombinant EPO, which is sold in the United States under the trade name Epogen® (Amgen, Thousand Oaks, Calif.). Recombinant and mutated forms of GM-CSF can be prepared as described in U.S. Pat. Nos. 5,391,485; 5,393,870; and 5,229,496; all of which are incorporated herein by reference. Recombinant and mutated forms of G-CSF can be prepared as described in U.S. Pat. Nos. 4,810,643; 4,999,291; 5,528,823; and 5,580,755; all of which are incorporated herein by reference.

Similarly, chemotherapeutic agents are well known in the art. For example, chemotherapeutic agents include alkylating agents such as thiotepa and cyclophosphamide (Cytoxan™); alkyl sulfonates such as busulfan, improsulfan and piposulfan; aziridines such as benzodopa, carboquone, meturedopa, and uredopa; emylerumines and memylamelamines including alfretamine, triemylenemelamine, triethylenephosphoramide, triethylenethiophosphoramide, and trimemylolomelamine; acetogenins (especially bullatacin and bullatacinone); a camptothecin (including synthetic analogue topotecan); bryostatin; callystatin; CC-1065 (including its adozelesin, carzelesin and bizelesin synthetic analogues); cryptophycins (articularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, foremustine, lomustine, nimustine, ranimustine; antibiotics such as the enediyne antibiotics (*e.g.,* calicheamicin, especially calicheamicin gammall and calicheamicin phili); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carrninomycin, carzinophilin, chromomycins, dactinomycin, daunorubicin, detorubicin, 6-diazo-5-oxo-L-norleucine, doxorubicin (Adramycin™) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as demopterin, methotrexate, pteropterin, trimetrexate; purine analogs such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replinisher such as frolinic acid; aceglatone; aldophosphamide glycoside; aminolevulinic acid; eniluracil; amsacrine; hestrabucil; bisantrene; edatraxate; defofamine; demecolcine; diaziquone; elformthine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK™; razoxane; rhizoxin; sizofiran; spirogermanium; tenuazonic acid; triaziquone; 2,2',2"-tricUorotriemylamine; trichothecenes (especially T-2 toxin, verracurin A, roridin A and anguidine); urethane; vindesine; dacarbazine; mannomustine; mitobronitol; mitolactol; pipobroman; gacytosine; arabinoside ("Ara-C"); cyclophosphamide; thiopeta; taxoids, e.g., paclitaxel (Taxol™, Bristol Meyers Squibb Oncology, Princeton, N.J.) and docetaxel (Taxoteret™, Rhone-Poulenc Rorer, Antony, France); chlorambucil; gemcitabine (Gemzar™); 6-thioguanine; mercaptopurine; methotrexate; platinum analogs such as cisplatin and carboplatin; vinblastine; platinum; etoposide (VP-16); ifosfamide; mitroxantrone; vancristine; vinorelbine (Navelbine™); novantrone; teniposide; edatrexate; daunomycin; aminopterin; xeoloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included in the definition of "chemotherapeutic agent" are anti-hormonal agents that act to regulate or inhibit hormone action on tumors such as anti-estrogens and selective estrogen receptor modulators (SERMs), including, for example, tamoxifen (including Nolvadex™), raloxifene, droloxifene, 4-hydroxytamoxifen, trioxifene, keoxifene, LY117018, onapristone, and toremifene (Fareston™); inhibitors of the enzyme aromatase, which regulates estrogen production in the adrenal glands, such as, for example, 4(5)-imidazoles, aminoglutethimide, megestrol acetate (Megace™), exemestane, formestane, fadrozole, vorozole (Rivisor™), letrozole (Femara™), and anastrozole (Arimidex™); and anti-androgens such as flutamide, nilutamide, bicalutamide, leuprohde, and goserelin; and pharmaceutically acceptable salts, acids or derivatives of any of the above. In some embodiments, the inhibitor downregulates Rac1 output. Additional examples of chemotherapeutic and other anti-cancer agents are described in US Pat. Publs. 2013/0239239 and 2009/0053224.

### b. Pharmaceutical Compositions

Agents that modulate (*e.g.,* inhibit or block) the function of PD-1 or PD-L1 and LAG-3, CTLA-4 or TIM-3, including, *e.g.,* blocking antibodies, peptides, fusion proteins, or small molecules, can be incorporated into pharmaceutical compositions suitable for administration to a subject. Such compositions typically comprise the antibody, peptide, fusion protein or small molecule and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (*e.g.,* inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition should be sterile and should be fluid to the extent that easy syringeability exists. It must be stable under the conditions of manufacture and storage and should be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

In one embodiment, modulatory agents are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations should be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by, and directly dependent on, the unique characteristics of the active compound, the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, *e.g*., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC50 (*i.e.,* the concentration of the test compound which achieves a half-maximal inhibition of symptoms) as determined in cell culture. Such information can be used to more accurately determine useful doses in humans. Levels in plasma can be measured, for example, by high performance liquid chromatography.

The above described modulating agents may be administered it he form of expressible nucleic acids which encode said agents. Such nucleic acids and compositions in which they are contained, are also encompassed by the present invention. For instance, the nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see *e.g.,* Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells which produce the gene delivery system.

The pharmaceutical compositions can be included in a container, pack, or dispenser together with instructions for administration.

### c. Prophylactic Methods

In one aspect, the present invention provides a method for preventing in a subject, a cancer, such as a hematologic cancer like multiple myeloma, associated with a less than desirable immune response. Subjects at risk for such a disease can be identified, for example, by any or a combination of diagnostic or prognostic assays known in the art. Administration of a prophylactic agent(s) can occur prior to the manifestation of symptoms associated with an unwanted or less than desirable immune response. The appropriate agent(s) used for treatment (*e.g.* antibodies, peptides, fusion proteins or small molecules) can be determined based on clinical indications and can be identified using diagnostic assays well known in the art, as well as those described herein.

### d. Therapeutic Methods

Another aspect of the invention pertains to therapeutic methods of modulating an immune response, *e.g.,* by inhibiting or blocking the function of PD-1 or PD-L1 and LAG-3, CTLA-4 or TIM-3.

Modulatory methods of the present invention involve contacting a cell with an agent that inhibits or blocks the function of PD-1 or PD-L1 and LAG-3, CTLA-4 or TIM-3. Exemplary agents useful in such methods are described above. Such agents can be administered *in vitro* or *ex vivo* (*e.g.,* by contacting the cell with the agent) or, alternatively, *in vivo* (*e.g.,* by administering the agent to a subject). As such, the present invention provides methods useful for treating an individual afflicted with a cancer, such as a hematologic cancer like multiple myeloma.

Agents that upregulate immune responses can be in the form of enhancing an existing immune response or eliciting an initial immune response. Thus, enhancing an immune response using the subject compositions and methods is useful for treating cancer, but can also be useful for treating an infectious disease (*e.g.,* bacteria, viruses, or parasites), a parasitic infection, and an immunosuppressive disease.

Immune responses can also be enhanced in an infected patient through an *ex vivo* approach, for instance, by removing immune cells from the patient, contacting immune cells *in vitro* with an agent described herein and reintroducing the *in vitro* stimulated immune cells into the patient.

In certain instances, it may be desirable to further administer other agents that upregulate immune responses, for example, forms of other B7 family members that transduce signals via costimulatory receptors, in order to further augment the immune response.

Agents that upregulate an immune response can be used prophylactically in vaccines against various polypeptides (*e.g.,* polypeptides derived from pathogens). Immunity against a pathogen (*e.g.,* a virus) can be induced by vaccinating with a viral protein along with an agent that upregulates an immune response, in an appropriate adjuvant.

In another embodiment, upregulation or enhancement of an immune response function, as described herein, is useful in the induction of tumor immunity.

In another embodiment, the immune response can be stimulated by the methods described herein, such that preexisting tolerance, clonal deletion, and/or exhaustion (*e.g.,* T cell exhaustion) is overcome. For example, immune responses against antigens to which a subject cannot mount a significant immune response, *e.g.,* to an autologous antigen, such as a tumor specific antigens can be induced by administering appropriate agents described herein that upregulate the immune response. In one embodiment, an autologous antigen, such as a tumor-specific antigen, can be coadministered. In another embodiment, the subject agents can be used as adjuvants to boost responses to foreign antigens in the process of active immunization.

In one embodiment, immune cells are obtained from a subject and cultured *ex vivo* in the presence of an agent as described herein, to expand the population of immune cells and/or to enhance immune cell activation. In a further embodiment the immune cells are then administered to a subject. Immune cells can be stimulated *in vitro* by, for example, providing to the immune cells a primary activation signal and a costimulatory signal, as is known in the art. Various agents can also be used to costimulate proliferation of immune cells. In one embodiment immune cells are cultured *ex vivo* according to the method described in PCT Application No. WO 94/29436. The costimulatory polypeptide can be soluble, attached to a cell membrane, or attached to a solid surface, such as a bead.

In still another embodiment, agents described herein useful for upregulating immune responses can further be linked, or operatively attached, to toxins using techniques that are known in the art, *e.g.,* crosslinking or via recombinant DNA techniques. Such agents can result in cellular destruction of desired cells. In one embodiment, a toxin can be conjugated to an antibody, such as a bispecific antibody. Such antibodies are useful for targeting a specific cell population, *e.g.,* using a marker found only on a certain type of cell. The preparation of immunotoxins is, in general, well known in the art (see, *e.g.,* U.S. Pat. Nos. 4,340,535, and EP 44167). Numerous types of disulfide-bond containing linkers are known which can successfully be employed to conjugate the toxin moiety with a polypeptide. In one embodiment, linkers that contain a disulfide bond that is sterically "hindered" are preferred, due to their greater stability *in vivo,* thus preventing release of the toxin moiety prior to binding at the site of action. A wide variety of toxins are known that may be conjugated to polypeptides or antibodies of the invention. Examples include: numerous useful plant-, fungus- or even bacteria-derived toxins, which, by way of example, include various A chain toxins, particularly ricin A chain, ribosome inactivating proteins such as saporin or gelonin, α-sarcin, aspergillin, restrictocin, ribonucleases, such as placental ribonuclease, angiogenic, diphtheria toxin, and *Pseudomonas* exotoxin, etc. A preferred toxin moiety for use in connection with the invention is toxin A chain which has been treated to modify or remove carbohydrate residues, deglycosylated A chain. (U.S. Patent 5,776,427). Infusion of one or a combination of such cytotoxic agents, (*e.g.,* ricin fusions) into a patient may result in the death of immune cells.

In yet another embodiment, the efficacy of the treatment methods described herein can be enhanced by incorporating a step of lymphodepletion prior to, concurrently with, or after the administration of agents the inhibit or block PD-1, PD-L1, CTLA-4, TIM-3, and/or LAG-3 function. For example, therapeutic benefits of administering the described anti-cancer agents can be synergistically enhanced by performing such administration after or in conjunction with lymphodepletion. Methods for achieving lymphodepletion in various forms and at various levels are well known in the art (see, for example, U.S. Patent 7,138,144). For example, the term "transient lymphodepletion" refers to destruction of lymphocytes and T cells, usually prior to immunotherapy. This can be accomplished in a number of ways, including "sublethal irradiation," which refers to administration of one or more doses of radiation that is generally non-lethal to all members of a population of subjects to which the administration is applied. Transient lymphodepletion is generally not myeloablative, as would be the case in complete lymphodepletion, such that the subjects hematopoietic or immunological capacity remains sufficiently intact to regenerate the destroyed lymphocyte and T cell populations. By contrast, "lethal irradiation" occurs when the administration is generally lethal to some but not all members of the population of subjects and "supralethal irradiation" occurs when the administion is generally lethal to all members of the population of subjects.

Depending on the application and purpose, transient lymphodepletion or complete lymphodepletion may be effected, for example, by any combination of irradiation, treatment with a myeloablative agent, and/or treatment with an immunosuppressive agent, according to standard protocols. For example, biological methods include, for example, administration of immunity-suppressing cells or by administration of biological molecules capable of inhibiting immunoreactivity, such as, for example, Fas-ligand and CTLA4-Ig. Examples of myeloablative agents include busulfan, dimethyl mileran, melphalan and thiotepa. Examples of immunosuppressive agents include prednisone, methyl prednisolone, azathioprine, cyclosporine A, cyclophosphamide, fludarabin, CTLA4-Ig, anti-T cell antibodies, etc.

Regarding irradiation, a sublethal dose of irradiation is generally within the range of 1 to 7.5 Gy whole body irradiation, a lethal dose is generally within the range of 7.5 to 9.5 Gy whole body irradiation, and a supralethal dose is within the range of 9.5 to 16.5 Gy whole body irradiation.

Depending on the purpose and application, the dose of irradiation may be administered as a single dose or as a fractionated dose. Similarly, administering one or more doses of irradiation can be accomplished essentially exclusively to the body part or to a portion thereof, so as to induce myeloreduction or myeloablation essentially exclusively in the body part or the portion thereof. As is widely recognized in the art, a subject can tolerate as sublethal conditioning ultrahigh levels of selective irradiation to a body part such as a limb, which levels constituting lethal or supralethal conditioning when used for whole body irradiation (see, for example, Breitz (2002) Cancer Biother Radiopharm. 17:119; Limit (1997) J. Nucl. Med. 38:1374; and Dritschilo and Sherman (1981) Environ. Health Perspect. 39:59). Such selective irradiation of the body part, or portion thereof, can be advantageously used to target particular blood compartments, such as specific lymph nodes, in treating hematopoietic cancers.

### e. Administration of Agents

The immune modulating agents of the invention are administered to subjects in a biologically compatible form suitable for pharmaceutical administration *in vivo,* to enhance immune cell mediated immune responses. By "biologically compatible form suitable for administration *in vivo"* is meant a form to be administered in which any toxic effects are outweighed by the therapeutic effects. The term "subject" is intended to include living organisms in which an immune response can be elicited, *e.g.,* mammals. Examples of subjects include humans, dogs, cats, mice, rats, and transgenic species thereof. Administration of an agent as described herein can be in any pharmacological form including a therapeutically active amount of an agent alone or in combination with a pharmaceutically acceptable carrier.

Administration of a therapeutically active amount of the therapeutic composition of the present invention is defined as an amount effective, at dosages and for periods of time necessary, to achieve the desired result. For example, a therapeutically active amount of an agent may vary according to factors such as the disease state, age, sex, and weight of the individual, and the ability of peptide to elicit a desired response in the individual. Dosage regimens can be adjusted to provide the optimum therapeutic response. For example, several divided doses can be administered daily or the dose can be proportionally reduced as indicated by the exigencies of the therapeutic situation.

Inhibiting or blocking the function of PD-1 or PD-L1 and LAG-3, CTLA-4 or TIM-3, or in some embodiments, inhibiting or blocking a combination of these agents, can be accomplished by combination therapy with the modulatory agents described herein. Combination therapy describes a therapy in which PD-1 or PD-L1 and LAG-3, CTLA-4 or TIM-3, are inhibited or blocked simultaneously. Simultaneous inhibition or blockade may be achieved by administration of the modulatory agents described herein simultaneously (e.g., in a combination dosage form or by simultaneous administration of single agents) or by administration of single agents according to a schedule that results in effective amounts of each modulatory agent present in the patient at the same time.

The therapeutic agents described herein can be administered in a convenient manner such as by injection (subcutaneous, intravenous, etc.), oral administration, inhalation, transdermal application, or rectal administration. Depending on the route of administration, the active compound can be coated in a material to protect the compound from the action of enzymes, acids and other natural conditions which may inactivate the compound. For example, for administration of agents, by other than parenteral administration, it may be desirable to coat the agent with, or co-administer the agent with, a material to prevent its inactivation.

An agent can be administered to an individual in an appropriate carrier, diluent or adjuvant, co-administered with enzyme inhibitors or in an appropriate carrier such as liposomes. Pharmaceutically acceptable diluents include saline and aqueous buffer solutions. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEEP) and trasylol. Liposomes include water-in-oil-in-water emulsions as well as conventional liposomes (Sterna et al. (1984) J. Neuroimmunol. 7:27*).*

The agent may also be administered parenterally or intraperitoneally. Dispersions can also be prepared in glycerol, liquid polyethylene glycols, and mixtures thereof, and in oils. Under ordinary conditions of storage and use, these preparations may contain a preservative to prevent the growth of microorganisms.

Pharmaceutical compositions of agents suitable for injectable use include sterile aqueous solutions (where water soluble) or dispersions and sterile powders for the extemporaneous preparation of sterile injectable solutions or dispersion. In all cases the composition will preferably be sterile and must be fluid to the extent that easy syringeability exists. It will preferably be stable under the conditions of manufacture and storage and preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating an agent of the invention (*e.g.,* an antibody, peptide, fusion protein or small molecule) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the agent plus any additional desired ingredient from a previously sterile-filtered solution thereof.

When the agent is suitably protected, as described above, the protein can be orally administered, for example, with an inert diluent or an assimilable edible carrier. As used herein "pharmaceutically acceptable carrier" includes any and all solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound, use thereof in the therapeutic compositions is contemplated. Supplementary active compounds can also be incorporated into the compositions.

It is especially advantageous to formulate parenteral compositions in dosage unit form for ease of administration and uniformity of dosage. "Dosage unit form ", as used herein, refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by, and directly dependent on, (a) the unique characteristics of the active compound and the particular therapeutic effect to be achieved, and (b) the limitations inherent in the art of compounding such an active compound for the treatment of sensitivity in individuals.

In one embodiment, an agent of the invention is an antibody. As defined herein, a therapeutically effective amount of antibody (*i.e.,* an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of an antibody can include a single treatment or, preferably, can include a series of treatments. In a preferred example, a subject is treated with antibody in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of antibody used for treatment may increase or decrease over the course of a particular treatment. Changes in dosage may result from the results of diagnostic assays.

This invention is further illustrated by the following examples which should not be construed as limiting. The contents of all references, patents and published patent applications cited throughout this application, as well as the Figures, are incorporated herein by reference.

### III. Kits

The present invention also encompasses kits for treating cancers, such as hematologic cancers like multiple myeloma, using agents that inhibit or block PD-1 or PD-L1 and LAG-3, CTLA-4 or TIM-3function. For example, the kit can comprise an antibody as described herein, e.g., an antibody against PD-1, PD-L1, LAG-3, CTLA-4 and/or TIM-3, packaged in a suitable container and can further comprise instructions for using such antibodies to treat cancers in a patient in need thereof. The kit may also contain other components, such as administration tools like packaged in a separate container.

### EXAMPLES

### Example 1: Combined PD-L1 and TIM-3 Blockade as Immunotherapy For Hematologic Cancers

### a. Materials and Methods

### Mice

All mice were housed in the Medical College of Wisconsin Biomedical Resource Center, an AAALAC-accredited facility. C47BL6/KaLwRij mice were used in the experiments. All animal work was reviewed and approved by the Medical College of Wisconsin Institutional Animal Care and Use Committee.

### Tumor cells

The 5T33 murine multiple myeloma (MM) cell line was derived from myeloma that spontaneously arose in a C57Bl/KaLwRij mouse (Radl et al. (1988) Am. J. Pathol. 132:593-597; and Manning et al. (1992) Br. J. Cancer 66:1088-1093). For experiments, 5T33 cells were thawed from a large frozen stock and cultured in RPMI 1640 + 10% fetal bovine serum for no longer than 2 weeks prior to inoculation of mice. Mice were inoculated with tumor as follows: 2x10⁶ 5T33 cells intravenously (i.v.). 5T33-bearing mice were considered moribund and euthanized when they developed paraparesis or paraplegia. Occasionally, 5T33-inoculated mice developed tumor masses or lesions and were euthanized when the size of the mass or lesion exceeded 250 mm²; other symptoms of advanced 5T33 included splenomegaly, hepatomegaly, or neurologic impairment.

### Antibodies

The clone designations for the antibodies used are as follows: anti-PD-Ll (clone 10F.9G2; Paterson et al. (2011) J. Immunol. 187:1097-1105; Maier et al. (2007) J. Immunol. 178:2714-2720), anti-PD-1 (clone 332.8H3), anti-Lag-3 (clone C9B7W; available from eBioscience as catalog number eBioC9B7W and other manufacturers), anti-Tim-3 (clone 5D12; available from EMD Millipore as catalog number MABF73). Isotype control antibodies included Armenian hamster IgG and rat IgG2a kappa.

### Irradiation

In general, myeloma-bearing recipient mice were given total body irradiation as a single sublethal (500 cGy) dose seven days after myeloma inoculation. Radiation was administered by a Shepherd Mark I Cesium Irradiator in accordance with established guidelines.

### Statistics

Survival curves were compared using the log rank (Mantel Cox) test based on n=5-6 mice per group. P-values of <0.05 were considered significant. Statistical analysis was done using Prism version 5.0a software (GraphPad Software, La Jolla, CA).

Other materials and methods are described in the "Results" section below.

### b. Results

Multiple myeloma is characterized by the presence of transformed neoplastic plasma cells in the bone marrow and is generally considered to be an incurable disease. Successful treatments will likely require multi-faceted approaches incorporating conventional drug therapies, immunotherapy and other novel treatments. It has previously been determined that a combination of transient lymphodepletion (sublethal whole body irradiation) and PD-1 blockade generated anti-myeloma T cell reactivity capable of eliminating established disease (Kearl et al. (2013) J. Immunol. 190:5620-5628; Hallett et al. (2011) Biol. Blood Marrow Transplant. 17:1133-1145). Besides expression of the immune checkpoint protein PD-1, T cells within tumor environment may develop a dysfunctional phenotype accompanied by the increased expression of other checkpoint proteins.

It was hypothesized that the anti-myeloma effect of transient lymphodepletion and PD-1 blockade would be increased by blocking other immune checkpoint protein interactions.

### Expression of immune checkpoint proteins on T cells in bone marrow of myeloma bearing mice over time

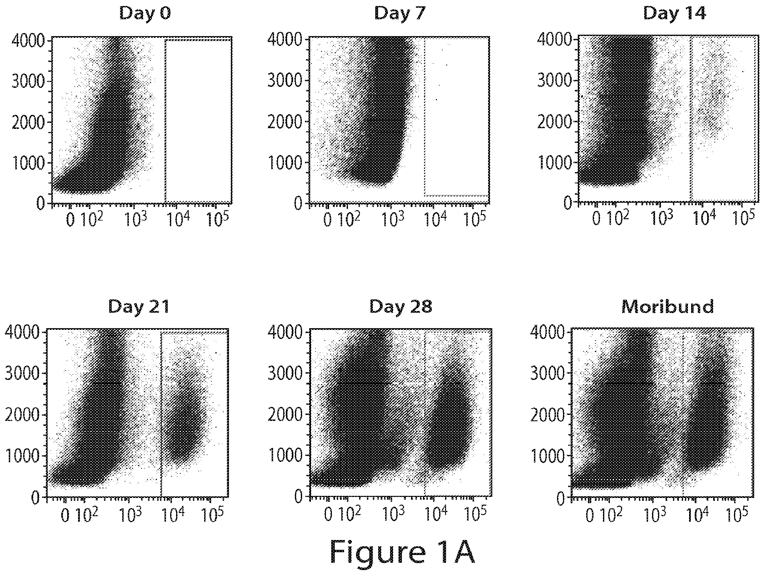

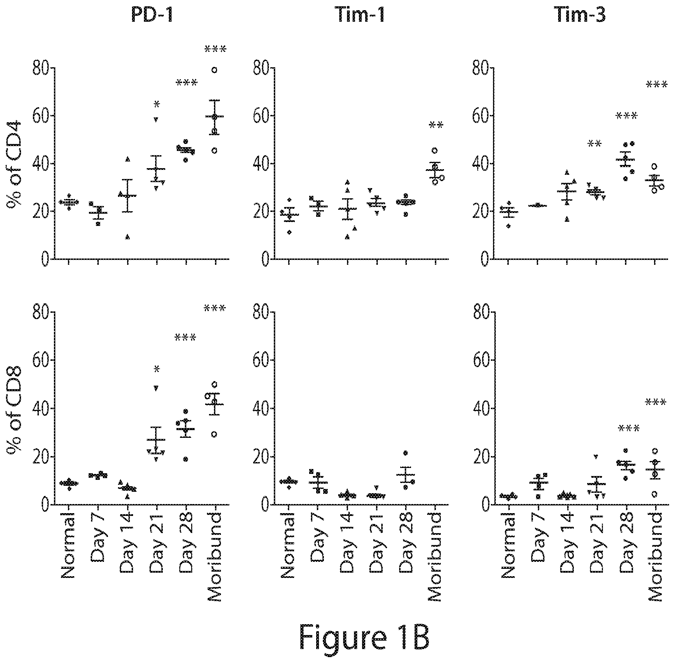

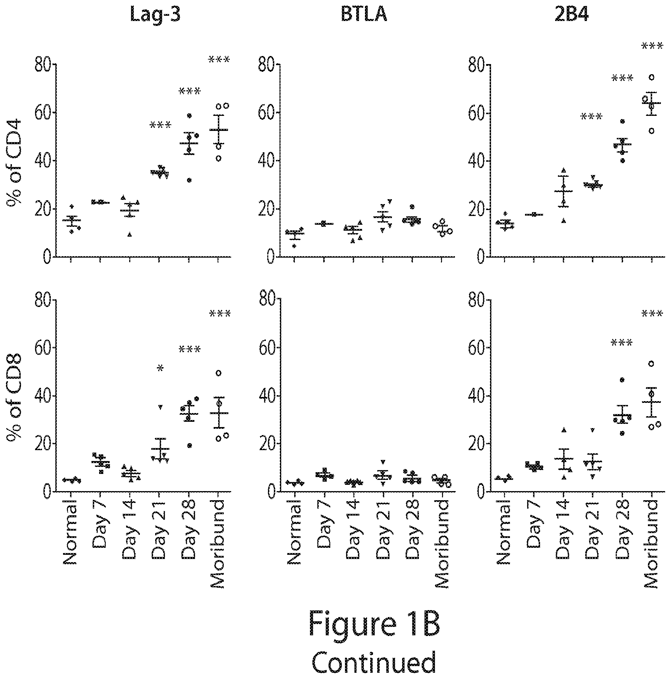

Accordingly, an extensive phenotypic analysis (flow cytometry) of bone marrow and splenic tissues from myeloma-bearing mice was performed to temporally examine T cells for expression of immune checkpoint proteins and assess the tissues for presence of immune regulatory T (Treg) cells. KaLwRij mice were inoculated with 2 × 10⁶ 5T33-GFP cells intravenously. Myeloma bearing mice were euthanized between days 7 and 28 after inoculation or when moribund (day 29-40), and femoral bone marrow cells were harvested. Tumor cell accumulation was monitored by flow cytometry (GFP+ tumor cells) (Figure 1A), and CD4⁺ (top) and CD8⁺ (bottom) T cells were analyzed by flow cytometry for expression of various immune checkpoint proteins over time (Figure 1B). Naive non-myeloma bearing mice were analyzed as controls. Immune checkpoint protein percentages were based on isotype controls.

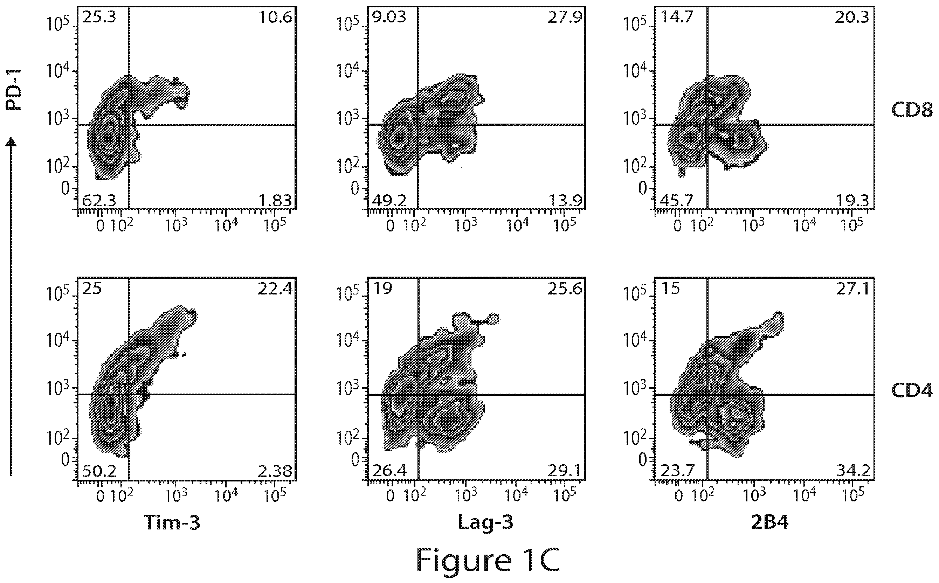

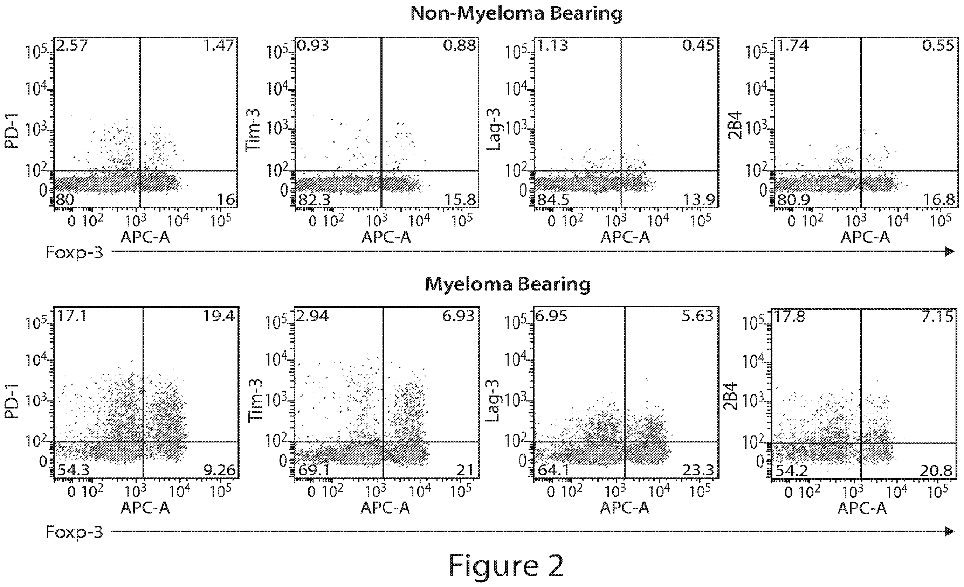

As shown in Figure 1B, PD-1, 2B4, LAG3, and TIM-3 were the most prominent immune checkpoint proteins present on T cells in myeloma bearing mice. As shown in Figure 1C, a relatively large percentage of PD-1⁺ T cells co-expressed other inhibitory checkpoint proteins such as Tim-3, Lag-3 and 2B4. It was also determined that Treg cells in the tumor microenvironment also had increased expression of PD-1 and other inhibitory receptors (such as Tim-3, Lag-3 and 2B4 shown in Figure 2) compared to Tregs cells from non-myeloma bearing mice, which is believed to be related with enhanced suppressive function by these cells.

### Increased expression of immune checkpoint proteins on T cells in mice treated with sublethal whole body irradiation and anti-PD-L1 antibody

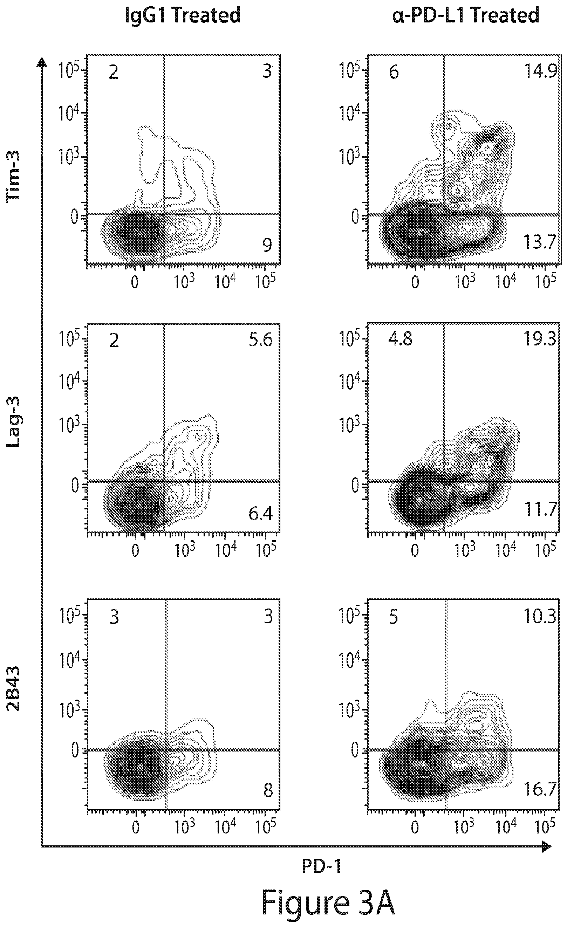

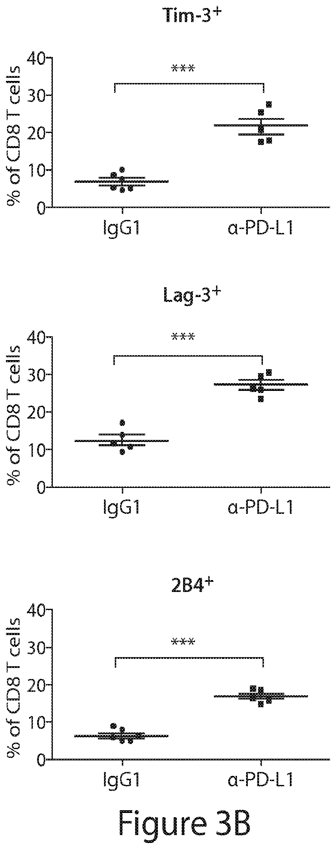

Myeloma bearing KaLwRij mice were treated with 500 cGy whole body irradiation 7 days after tumor cell inoculation. Treatment with anti-PD-Ll antibody or control IgG (200 µg i.p.) was initiated 5 days later and specifically given 12, 14, and 19 days after tumor inoculation. Mice were euthanized at day 21, splenocytes were harvested, and the CD8 T cells were analyzed by flow cytometry for immune checkpoint protein expression. As shown in Figures 3A-3B, the frequencies of CD8⁺Tim-3⁺, CD8⁺Lag-3⁺ and CD8⁺2B4⁺ cells in spleens of anti-PD-Ll antibody treated mice were higher compared with spleens of control antibody treated.

### Blocking PD-L1 in combination with Tim-3 after lymphodepleting whole body irradiation synergistically improved survival

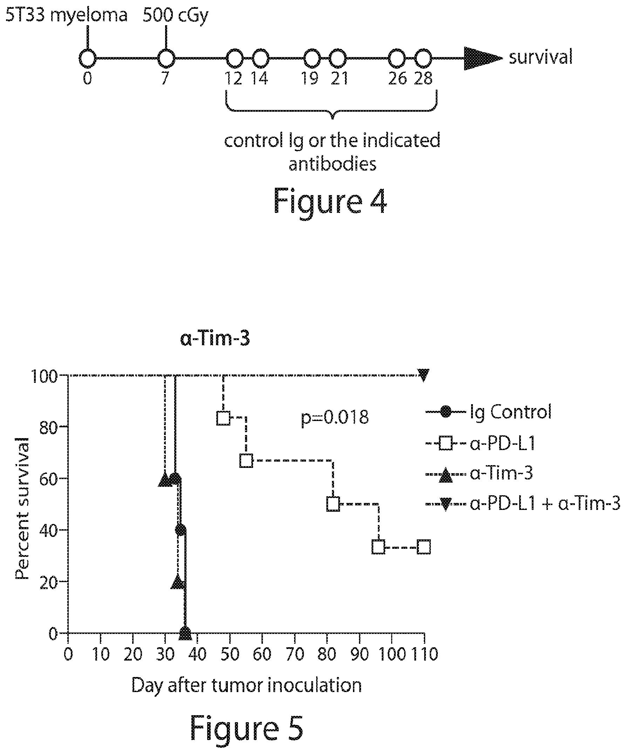

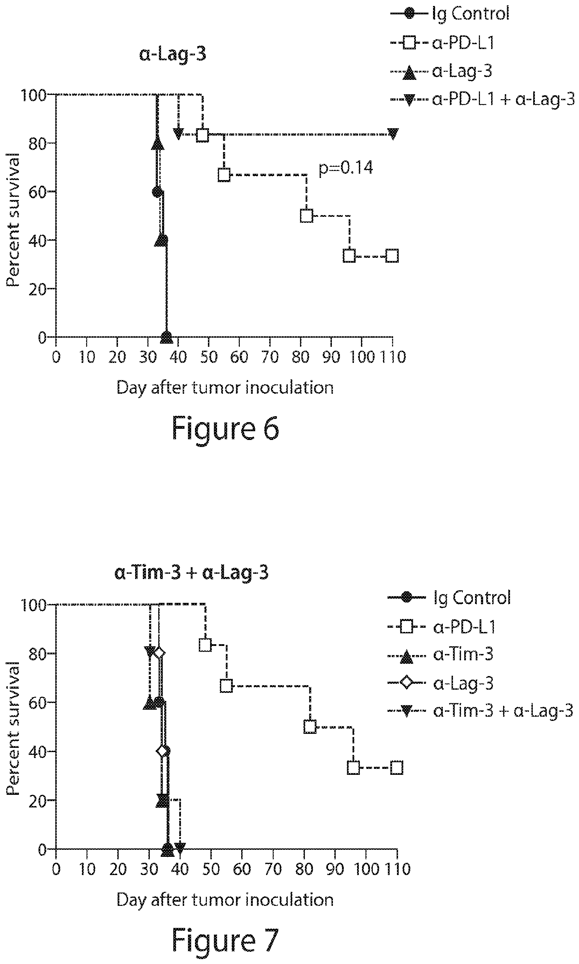

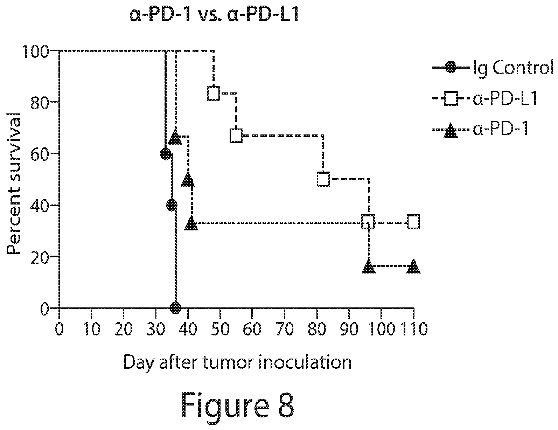

It was then examined whether blocking various immune checkpoint proteins could provide additive or synergistic anti-myeloma effects when combined with PD-L1 blockade (Figure 4). In this Example, the combined blockade of PD-1 and TIM-3 was most effective and proved to be synergistic, as myeloma was surprisingly rejected in 100% of these mice (Figure 5). Inhibition of certain other immune checkpoint proteins, either alone or in combinations, did not produce such robust therapeutic benefits (Figures 6-8).

Thus, the data indicate that dual blockade of PD-L1 and TIM-3 represents a surprisingly and unexpectedly potent immunotherapeutic intervention for treating hematologic cancers, such as multiple myeloma.

### Example 2: Combined Immune Checkpoint Protein Blockade and Lymphodepletion as Immunotherapy for Hematologic Cancers

a. Materials and methods are essentially the same as described in Example 1 unless specifically indicated below in the "Results" section below.
b. Results

As shown in this Example, combined immune checkpoint protein blockade and lymphodepletion provide an effective immunotherapy for hematologic cancers such as myeloma.

### Blocking of PD-L1 in combination with Tim-3, Lag-3 or CTLA-4 after lymphodepleting whole body irradiation synergistically improved survival

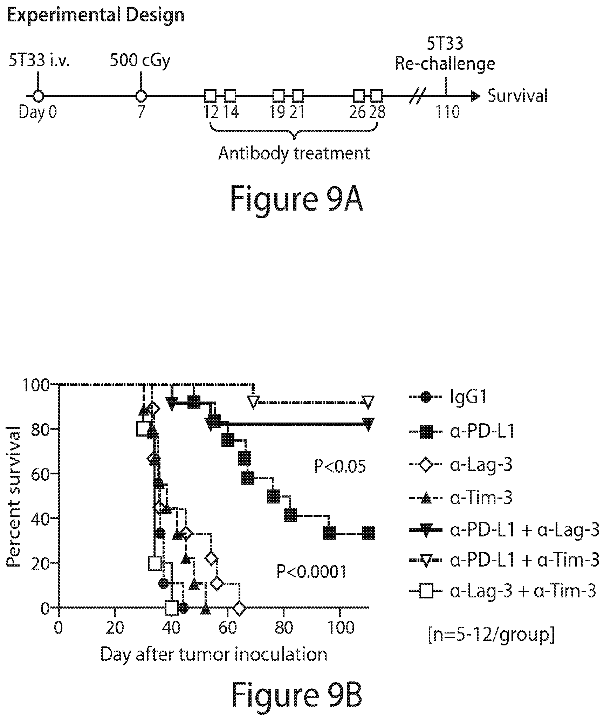

Figure 9A depicts the experimental design. KaLwRij mice received 500 cGy irradiation 7 days after tumor cell inoculation. The treatment with blocking antibody or control IgG (200 µg i.p.) was initiated 5 days later and specifically given 12, 14, 19, 21, 26, and 28 days after tumor inoculation.

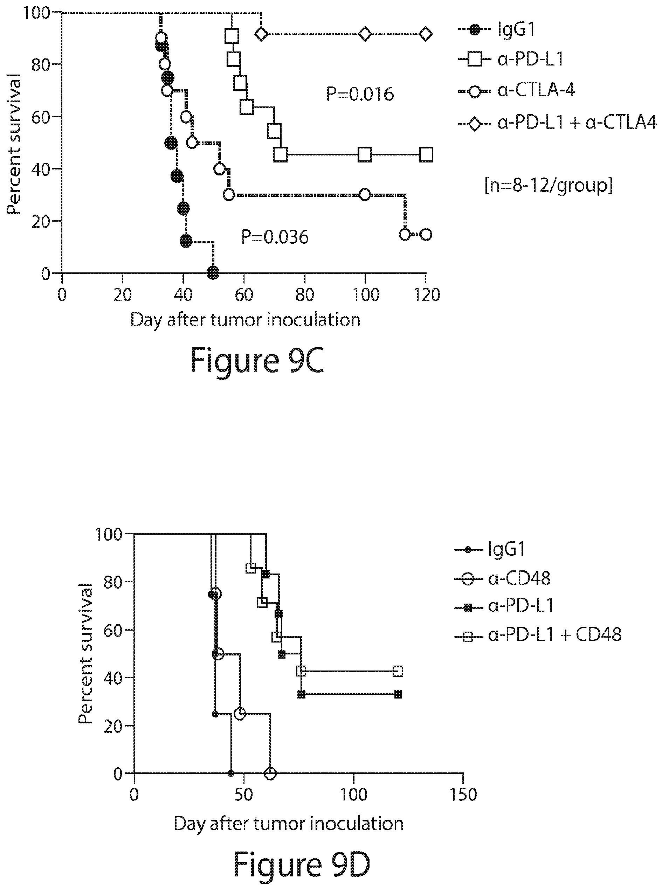

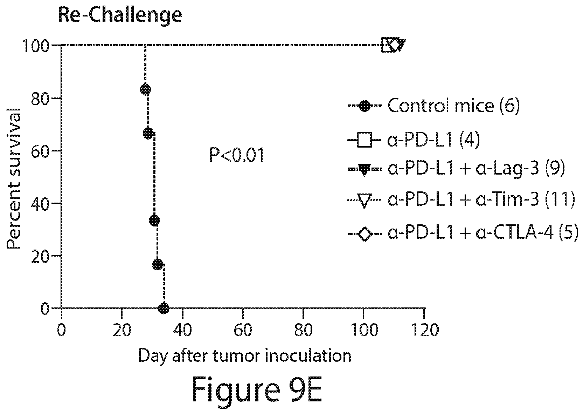

As shown in Figures 9B-9D, blocking PD-L1 in combination with Tim-3 (Figure 9B), Lag-3 (Figure 9B), or CTLA-4 (Figure 9C) after lymphodepleting whole body irradiation synergistically improved survival of myeloma bearing mice, whereas blocking PD-L1 in combination with CD48 (Figure 9D) did not have synergistic effect on survival. As shown in Figure 9E, 100% of re-challenged mice that had received anti-PD-Ll antibody alone, or the combination of anti-PD-Ll antibody with anti-Tim-3 antibody, anti-Lag-3 antibody, or anti-CTLA-4 antibody, survived to day 110, compared to control mice.

### Combined checkpoint blockade after lymphodepleting whole body irradiation increased frequencies of tumor-reactive T cells

The experimental design shown in Figure 9A was used. CD4⁺ or CD8⁺ T cells were isolated from spleens and bone marrow 21 days after tumor cell inoculation (*i.e.,* 14 days after irradiation) in mice treated with control IgG, anti-PD-Ll antibody only, or the combination of anti-PD-Ll antibody with anti-Tim-3 antibody, anti-Lag-3 antibody, or anti-CTLA-4 antibody. The CD8⁺ or CD4⁺ T cells were tested in IFN-γ ELISPOT assays using 5T33 or MHC class II+ 5T33 tumor cells as stimulators, respectively, to determine tumor-reactive IFN-γ-secreting cell frequencies.

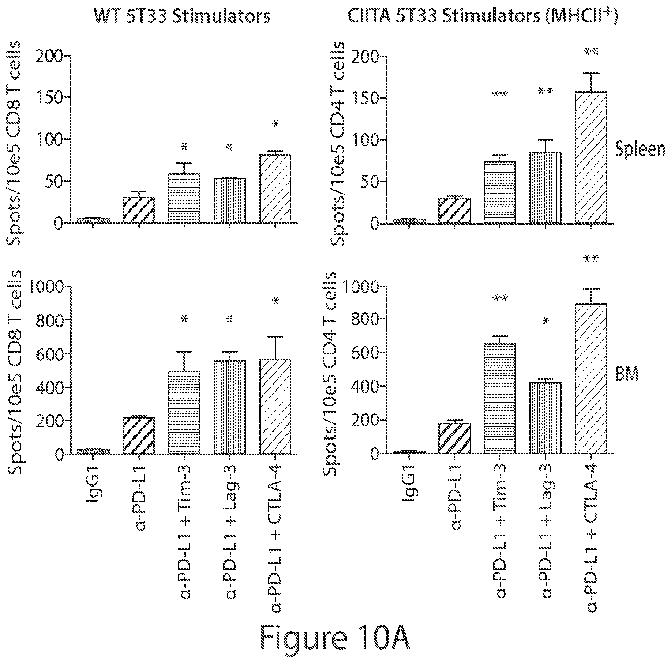

As shown in Figure 10A, the frequencies of tumor-reactive CD8⁺ and CD4⁺ T cells were increased in the spleens (top row) and bone marrow (bottom row) of mice treated with combinations of immune checkpoint protein blockade. Combined checkpoint blockade, such as the combination of anti-PD-Ll antibody with anti-Tim-3 antibody, anti-Lag-3 antibody, or anti-CTLA-4 antibody, increased frequencies of tumor-reactive T cells.

### Combined checkpoint blockade after lymphodepleting whole body irradiation increased cytokine production by CD8⁺ T cells

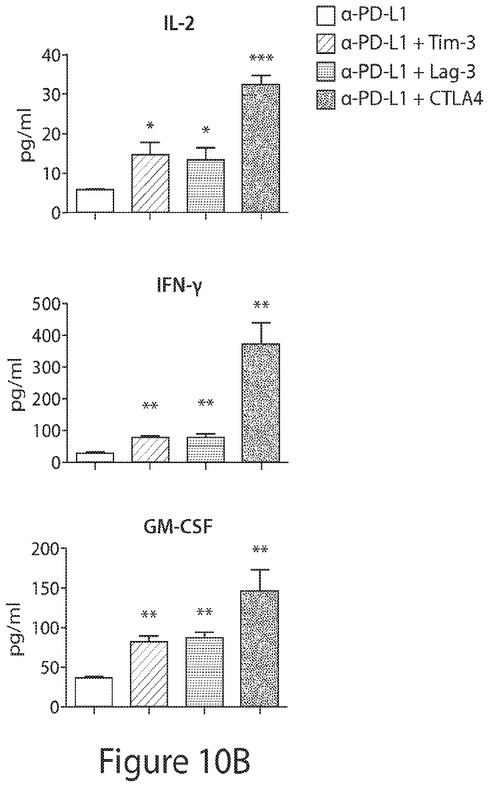

CD8⁺ T cells purified from the spleens of myeloma bearing mice treated with anti-PD-Ll antibody only, or the combination of anti-PD-Ll antibody with anti-Tim-3 antibody, anti-Lag-3 antibody or anti-CTLA4 antibody, were stimulated with 5T33 for 48 hours. Supernatants were collected and cytokine levels from were determined using a multiplex cytokine assay. As shown in Figure 10B, combined checkpoint blockade, such as the combination of anti-PD-Ll antibody with anti-Tim-3 antibody, anti-Lag-3 antibody, or anti-CTLA-4 antibody, increased the production of cytokines (*e.g*., IL-2, IFN-γ and GM-CSF) by CD8⁺ T cells.

### Combined blockade of immune checkpoint proteins increased PD-1 expression on CD8⁺ T cells as well as increased frequency of tumor specific cytotoxic T lymphocytes

The experimental design in Figure 9A was used. CD8⁺ T cells were isolated from spleens and bone marrow 21 days after tumor cell inoculation in mice treated with control IgG, anti-PD-L1 antibody only, or the combination of anti-PD-Ll antibody with anti-Tim-3 antibody, anti-Lag-3 antibody, or anti-CTLA4 antibody.

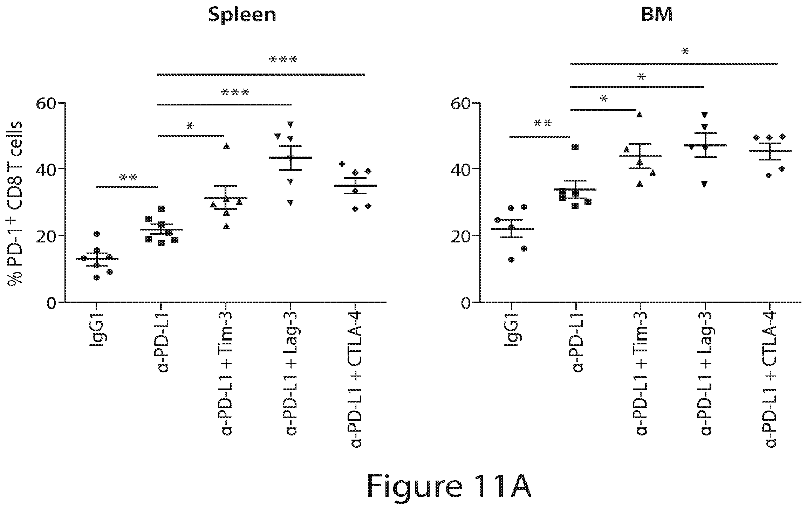

Figure 11A shows increased expression of PD-1 on gated CD8⁺ T cells from spleens and bone marrow (BM) of mice treated with different blocking antibodies or control IgG.

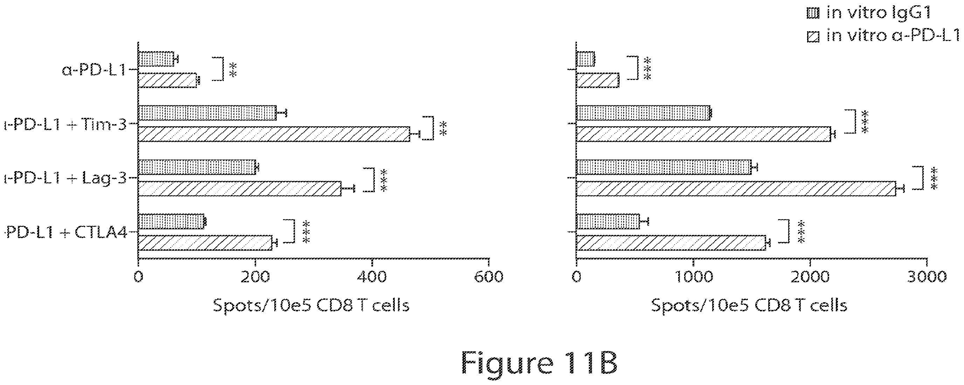

The CD8⁺ T cells were assayed in IFN-γ ELISPOT assays with tumor cell stimulators to determine tumor-reactive IFN-γ-secreting cell frequencies in the presence of anti-PD-Ll antibody or control IgG (10 µg/ml). As shown in Figure 11B, in the presence of blocking anti-PD-L1 antibody *in vitro,* all combinations of blocking antibodies *in vivo* resulted in significantly increased cytotoxic T lymphocyte (CTL) frequencies versus blockade of PD-L1 only (p<0.001).

### Combined blockade of immune checkpoint proteins enhanced Th1 and Th2 cytokine secretion

The experimental design shown in Figure 9A was used. CD4⁺ T cells were isolated from spleens 21 days after tumor cell inoculation (*i.e.,* 14 days after irradiation) in myeloma bearing mice treated with control anti-PD-Ll, or the combination of anti-PD-Ll antibody with anti-Tim-3 antibody, anti-Lag-3 antibody, or anti-CTLA4 antibody. CD4⁺ T cells purified from the spleen were stimulated with MHC class II negative 5T33-WT, or MHC class II positive 5T33-CIITA, or no stimulation for 48 hours. Supernatants were collected and cytokine (IFN-y, IL-4 and IL-5) levels from were determined using a multiplex cytokine assay.

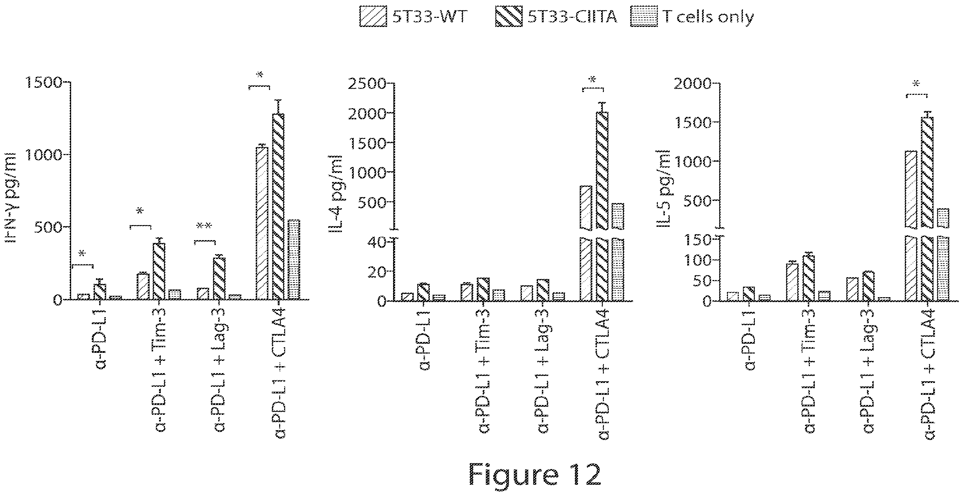

As shown in Figure 12, combined blockade of immune checkpoint proteins such as anti-PD-L1 and anti-CTLA-4 antibodies enhanced Th1 and Th2 cytokine secretion.

### Expression of immune checkpoints on T cells in mice with other hematologic cancers

Murine hematologic cancers other than myeloma express PD-L1 and respond to whole body irradiation plus PD-L1 blockade (Kearl et al. (2013) J. Immunol. 190:5620-5628).

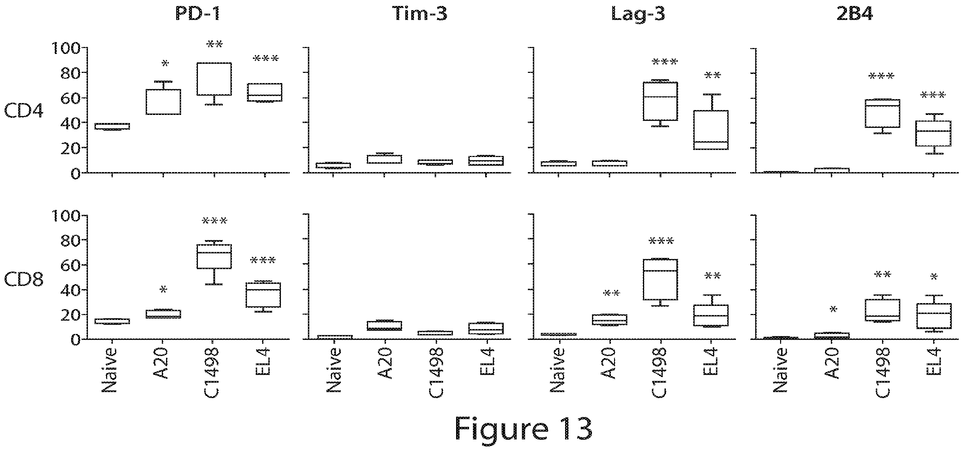

To examine the expression of immune checkpoint proteins on T cells in mice with other hematologic cancers, mice were injected i.v. with A20 B cell lymphoma cells, C1498 acute myeloid leukemia cells, or EL4 lymphoma cells (x-axis in Figure 13). Bone marrow was collected from moribund animals. CD4⁺ (top row in Figure 13) and CD8⁺ (bottom row in Figure 13) T cells in the bone marrow were analyzed for expression of checkpoint proteins PD-1, Tim-3, Lag-3 and 2B4 by flow cytometry (n=4-5 mice for each). Naive non-cancer-bearing mice were used as controls. Figure 13 shows expression of PD-1, Tim-3, Lag-3 and 2B4 on T cells in mice bearing the indicated hematologic cancer cells. A correlation can exist between the T cell expression profile of these checkpoint proteins and increased anti-tumor response after co-blockade of the respective pathway(s) in hematologic malignancy models other than myeloma.

### A model of combined immune checkpoint blockade and lymphodepleting whole body irradiation for treating hematologic cancers

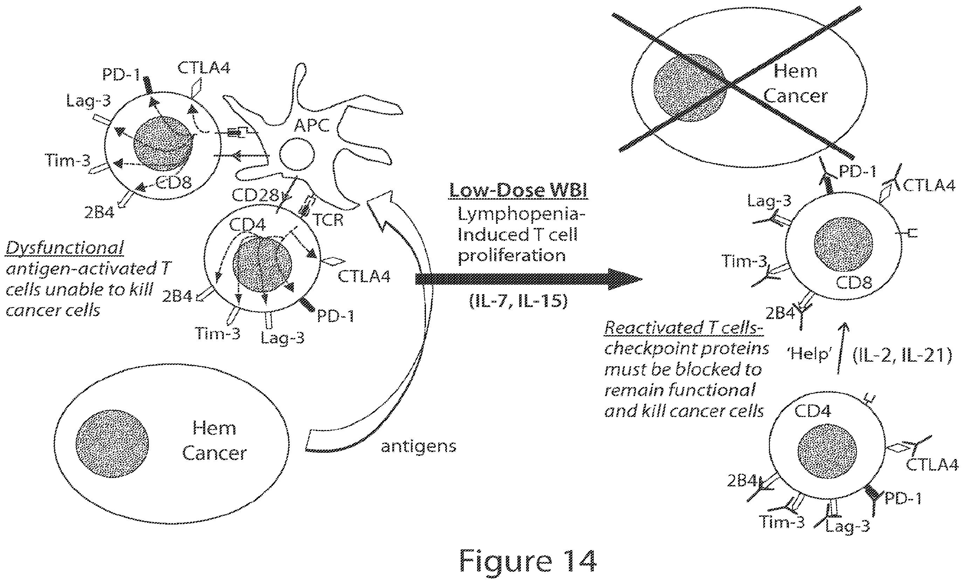

A working model of combined immune checkpoint blockade and lymphodepleting whole body irradiation is illustrated in Figure 14. In hematologic cancers, dysfunctional antigen-activated T cells (*e.g.,* CD4⁺ and CD8⁺ T cells) are unable to kill cancer cells. Lymphopenia-induced T cell proliferation allows for reactivation of those T cells. For reactivated T cells to remain functional and kill cancer cells, immune checkpoint proteins must be blocked. Lymphopenic environment can be achieved by low-dose whole body irradiation (WBI). Lymphodepleting chemotherapy or low doses of T cell-depleting antibodies can also be used instead of whole body irradiation.

### Incorporation by Reference

All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

Also incorporated by reference in their entirety are any polynucleotide and polypeptide sequences which reference an accession number correlating to an entry in a public database, such as those maintained by The Institute for Genomic Research (TIGR) on the world wide web at tigr.org and/or the National Center for Biotechnology Information (NCBI) on the World Wide Web at ncbi.nlm.nih.gov.

### Equivalents

Those skilled in the art will recognize, or be able to ascertain using no more than routine experimentation, many equivalents to the specific embodiments of the invention described herein. Such equivalents are intended to be encompassed by the following embodiments.

### Embodiments:

1. A method of treating a subject afflicted with a hematologic cancer comprising administering to the subject a therapeutically effective amount of an inhibitor of PD-L1 or PD-1 and an inhibitor of TIM-3, LAG-3 or CTLA4.
2. The method of embodiment 1 wherein an inhibitor of PD-L1 is administered in combination with an inhibitor of TIM-3.
3. The method of embodiment 1 wherein an inhibitor of PD-1 is administered in combination with an inhibitor of TIM-3.
4. The method of embodiment 1 wherein an inhibitor of PD-L1 is administered in combination with an inhibitor of LAG-3.
5. The method of embodiment 1 wherein an inhibitor of PD-1 is administered in combination with an inhibitor of LAG-3.
6. The method of embodiment 1 wherein an inhibitor of PD-L1 is administered in combination with an inhibitor of CTLA-4.
7. The method of embodiment 1 wherein an inhibitor of PD-1 is administered in combination with an inhibitor of CTLA-4.
8. The method of any of embodiments 1-7, wherein the inhibitor is chosen from an inhibitory nucleic acid, a soluble ligand, or an antibody or antigen-binding fragment thereof, that binds to one or more of PD-1, PD-L1, TIM-3, LAG-3 or CTLA-4.
9. The method of any of embodiments 1-7, wherein the inhibitor is a bispecific or multispecific antibody, or antigen binding fragment thereof, selective for PD-1 or PD-L1 and TIM-3, LAG-3 or CTLA4.
10. The method of any of embodiments 1-7, wherein a combination of inhibitors comprising a first inhibitor that selectively inhibits or blocks PD-1 or PD-L1 and a second inhibitor that selectively inhibits or blocks TIM-3, LAG-3 or CTLA4 are administered.
11. The method of any of embodiments 1-7, wherein the inhibitor is a soluble ligand of PD-1, PD-L1, TIM-3, LAG-3 or CTLA-4.
12. The method of embodiment 10, wherein the first inhibitor, second inhibitor, or both inhibitors, is an antibody or an antigen binding fragment thereof, which specifically binds to PD-1 or PD-L1 and/or TIM-3, LAG-3 or CTLA4.
13. The method of embodiment 12, wherein said antibody, or antigen binding fragment thereof, is murine, chimeric, humanized, composite, or human.
14. The method of embodiment 12, wherein said antibody, or antigen binding fragment thereof, is detectably labeled, comprises an effector domain, comprises an Fc domain, and/or is selected from the group consisting of Fv, Fav, F(ab')2), Fab', dsFv, scFv, sc(Fv)2, and diabodies fragments.
15. The method of embodiment 12, wherein said antibody, or antigen binding fragment thereof, is conjugated to a cytotoxic agent.
16. The method of embodiment 15, wherein said cytotoxic agent is selected from the group consisting of a chemotherapeutic agent, a biologic agent, a toxin, and a radioactive isotope.
17. The method of embodiment 8, wherein said inhibitory nucleic acid comprises an RNA interfering agent which inhibits expression of PD-1, PD-L1, TIM-3, LAG-3 or CTLA-4.
18. The method of embodiment 17, wherein said RNA interfering agent is a small interfering RNA (siRNA), small hairpin RNA (shRNA), or a microRNA (miRNA).
19. The method of embodiment 8, wherein said inhibitory nucleic acid comprises an antisense oligonucleotide complementary to PD-1, PD-L1, TIM-3, LAG-3 or CTLA-4.
20. The method of any of embodiments 1-7, wherein said inhibitor comprises a peptide or peptidomimetic that inhibits or blocks PD-1, PD-L1, TIM-3, LAG-3 or CTLA-4.
21. The method of any of embodiments 1-7, wherein said inhibitor comprises a small molecule that inhibits or blocks PD-1, PD-L1, TIM-3, LAG-3 or CTLA-4.
22. The method of any of embodiments 1-7, wherein said inhibitor comprises an aptamer that inhibits or blocks PD-1, PD-L1, TIM-3, LAG-3 or CTLA-4.
23. The method of any of embodiments 1-7, wherein said inhibitor is administered in a pharmaceutically acceptable formulation.
24. The method of any of embodiments 1-7, further comprising administering to the subject a therapeutic agent for treating the hematologic cancer.
25. The method of any of embodiments 1-7, further comprising a step of transient or complete lymphodepletion.
26. The method of embodiment 25, wherein sublethal whole body irradiation is used for transient lymphodepletion.
27. The method of embodiment 25, wherein lethal whole body irradiation is used for complete lymphodepletion.
28. The method of embodiment 25, wherein the step of lymphodepletion occurs before, concurrently with, or after the step of agent administration.
29. The method of any of embodiments 1-7, wherein the hematologic cancer is selected from the group consisting of multiple myeloma, acute lymphocytic leukemia, acute myeloid leukemia, chronic lymphocytic leukemia, small lymphocytic lymphoma, non-Hodgkin's lymphoma, Hodgkin's lymphoma, mantle cell lymphoma, follicular lymphoma, Waldenstrom's macroglobulinemia, B-cell lymphoma and diffuse large B-cell lymphoma, precursor B-lymphoblastic leukemia/lymphoma, B-cell chronic lymphocytic leukemia/small lymphocytic lymphoma, B-cell prolymphocytic leukemia, lymphoplasmacytic lymphoma, splenic marginal zone B-cell lymphoma (with or without villous lymphocytes), hairy cell leukemia, plasma cell myeloma/plasmacytoma, extranodal marginal zone B-cell lymphoma of the MALT type, nodal marginal zone B-cell lymphoma (with or without monocytoid B cells), Burkitt's lymphoma; precursor T-lymphoblastic lymphoma/leukemia, T-cell prolymphocytic leukemia, T-cell granular lymphocytic leukemia, aggressive NK cell leukemia, adult T-cell lymphoma/leukemia (HTLV 1-positive), nasal-type extranodal NK/T-cell lymphoma, enteropathy-type T-cell lymphoma, hepatosplenic γ-δ T-cell lymphoma, subcutaneous panniculitis-like T-cell lymphoma, mycosis fungoides/Sézary syndrome, anaplastic large cell lymphoma (T/null cell, primary cutaneous type), anaplastic large cell lymphoma (T-/null-cell, primary systemic type), peripheral T-cell lymphoma not otherwise characterized, angioimmunoblastic T-cell lymphoma, polycythemia vera (PV), myelodysplastic syndrome (MDS), indolent Non-Hodgkin's Lymphoma (iNHL) and aggressive Non-Hodgkin's Lymphoma (aNHL).
30. The method of any of embodiments 1-7, wherein the hematologic cancer is selected from the group consisting of B-cell lymphoma, myeloid leukemia and multiple myeloma.
31. The method of any of embodiments 1-7, wherein the hematologic cancer is multiple myeloma.
32. The method of any of embodiments 1-7, wherein the subject is a human.
33. A kit for treating a subject afflicted with a hematologic cancer comprising one or more agents, wherein at least one agent selectively inhibits or blocks PD-1 or PD-L1 and TIM-3, LAG-3 or CTLA4.
34. The kit of embodiment 33, wherein the agent is a bispecific or multispecific antibody, or antigen binding fragment thereof, selective for PD-1 or PD-L1 and TIM-3, LAG-3 or CTLA4.
35. A kit for treating a subject afflicted with a hematologic cancer comprising a first agent that selectively inhibits or blocks PD-1 or PD-L1 and a second agent that selectively inhibits or blocks TIM-3, LAG-3 or CTLA4.
36. The kit of embodiment 35, wherein said first agent and/or second agent is an antibody, or an antigen binding fragment thereof, which specifically binds to PD-1 or PD-L1 protein and/or TIM-3, LAG-3 or CTLA4 protein.
37. The kit of embodiment 35 or 36, wherein said antibody, or antigen binding fragment thereof, is murine, chimeric, humanized, composite, or human.
38. The kit of embodiment 35 or 36, wherein said antibody, or antigen binding fragment thereof, is detectably labeled, comprises an effector domain, comprises an Fc domain, and/or is selected from the group consisting of Fv, Fav, F(ab')2), Fab', dsFv, scFv, sc(Fv)2, and diabodies fragments.
39. The kit of embodiment 35 or 36, wherein said antibody, or antigen binding fragment thereof, is conjugated to a cytotoxic agent.
40. The kit of embodiment 39, wherein said cytotoxic agent is selected from the group consisting of a chemotherapeutic agent, a biologic agent, a toxin, and a radioactive isotope.
41. The kit of embodiment 35 or 36, wherein said agent is selected from the group consisting of a) an RNA interfering agent which inhibits expression of PD-L1 or PD-1 and TIM-3, LAG-3 or CTLA4, optionally wherein said RNA interfering agent is an small interfering RNA (siRNA), small hairpin RNA (shRNA), or a microRNA (miRNA); b) an antisense oligonucleotide complementary to PD-1 or PD-L1 and/or TIM-3, LAG-3 or CTLA4; c) a peptide or peptidomimetics that inhibits or blocks PD-1 or PD-L1 and/or TIM-3, LAG-3 or CTLA4; d) a small molecule that inhibits or blocks PD-1 or PD-L1 and/or TIM-3, LAG-3 or CTLA4, optionally wherein said small molecule inhibits a protein-protein interaction between PD-1 or PD-L1 and/or TIM-3, LAG-3 or CTLA4 and a receptor; and e) an aptamer that inhibits or blocks PD-1 or PD-L1 and/or TIM-3, LAG-3 or CTLA4.

## Claims

1. A combination of a therapeutically effective amount of an inhibitor of PD-L1 that selectively inhibits or blocks PD-L1 and an inhibitor of TIM-3 that selectively inhibits or blocks TIM-3, for use in a method of treating a hematologic cancer in a subject comprising administering to the subject the inhibitor of PD-L1 and the inhibitor of TIM-3,
wherein the inhibitor of PD-L1:
(i) is an antibody or antigen-binding fragment thereof that binds to PD-L1, or an inhibitory nucleic acid that inhibits expression of PD-L1, wherein said inhibitory nucleic acid that inhibits expression of PD-L1 comprises:
(a) an RNA interfering agent which inhibits expression of PD-L1, optionally wherein said RNA interfering agent is a small interfering RNA (siRNA), small hairpin RNA (shRNA), or a microRNA (miRNA); or
(b) an antisense oligonucleotide complementary to a nucleotide sequence of PD-L1; or
(ii) is a bispecific or multispecific antibody, or antigen binding fragment thereof, selective for PD-L1 and TIM-3;
and optionally wherein the inhibitor of PD-L1 is in a pharmaceutically acceptable formulation; and
wherein the inhibitor of TIM-3:
(i) is an antibody or antigen-binding fragment thereof that binds to TIM-3, or an inhibitory nucleic acid that inhibits expression of TIM-3, wherein the inhibitory nucleic acid that inhibits expression of TIM-3 comprises:
(a) an RNA interfering agent which inhibits expression of TIM-3, optionally wherein said RNA interfering agent is a small interfering RNA (siRNA), small hairpin RNA (shRNA), or a microRNA (miRNA); or
(b) an antisense oligonucleotide complementary to a nucleotide sequence of TIM-3;
or
(ii) is a bispecific or multispecific antibody, or antigen binding fragment thereof, selective for PD-L1 and TIM-3;
and optionally wherein the inhibitor of TIM-3 is in a pharmaceutically acceptable formulation.

2. The combination for use of claim 1, wherein the first inhibitor, second inhibitor, or both inhibitors, is an antibody or an antigen binding fragment thereof, which specifically binds to PD-L1 and/or TIM-3.

3. The combination for use of claim 1 or 2, wherein said antibody or antigen binding fragment thereof:
(i) is murine, chimeric, humanized, composite, or human;
(ii) is detectably labeled, comprises an effector domain, comprises an Fc domain, and/or is selected from the group consisting of Fv, Fav, F(ab')2), Fab', dsFv, scFv, sc(Fv)2, and diabodies fragments; or
(iii) is conjugated to a cytotoxic agent, wherein said cytotoxic agent is selected from the group consisting of a chemotherapeutic agent, a biologic agent, a toxin, and a radioactive isotope.

4. The combination for use of any of claims 1-3, wherein the method further comprises administering to the subject a therapeutic agent for treating the hematologic cancer or a step of transient or complete lymphodepletion, optionally wherein:
(i) sublethal whole body irradiation is used for transient lymphodepletion;
or
(ii) the step of lymphodepletion occurs before, concurrently with, or after the step of agent administration.

5. The combination for use of any of claims 1-7, wherein the hematologic cancer is selected from the group consisting of multiple myeloma, acute lymphocytic leukemia, acute myeloid leukemia, chronic lymphocytic leukemia, small lymphocytic lymphoma, non-Hodgkin's lymphoma, Hodgkin's lymphoma, mantle cell lymphoma, follicular lymphoma, Waldenstrom's macroglobulinemia, B-cell lymphoma and diffuse large B-cell lymphoma, precursor B-lymphoblastic leukemia/lymphoma, B-cell chronic lymphocytic leukemia/small lymphocytic lymphoma, B-cell prolymphocytic leukemia, lymphoplasmacytic lymphoma, splenic marginal zone B-cell lymphoma (with or without villous lymphocytes), hairy cell leukemia, plasma cell myeloma/plasmacytoma, extranodal marginal zone B-cell lymphoma of the MALT type, nodal marginal zone B-cell lymphoma (with or without monocytoid B cells), Burkitt's lymphoma; precursor T-lymphoblastic lymphoma/leukemia, T-cell prolymphocytic leukemia, T-cell granular lymphocytic leukemia, aggressive NK cell leukemia, adult T-cell lymphoma/leukemia (HTLV 1-positive), nasal-type extranodal NK/T-cell lymphoma, enteropathy-type T-cell lymphoma, hepatosplenic γ-δ T-cell lymphoma, subcutaneous panniculitis-like T-cell lymphoma, mycosis fungoides/Sézary syndrome, anaplastic large cell lymphoma (T/null cell, primary cutaneous type), anaplastic large cell lymphoma (T-/null-cell, primary systemic type), peripheral T-cell lymphoma not otherwise characterized, angioimmunoblastic T-cell lymphoma, polycythemia vera (PV), myelodysplastic syndrome (MDS), indolent Non-Hodgkin's Lymphoma (iNHL) and aggressive Non-Hodgkin's Lymphoma (aNHL),
or wherein the hematologic cancer is selected from the group consisting of B-cell lymphoma, myeloid leukemia and multiple myeloma, optionally wherein the hematologic cancer is multiple myeloma.

6. The combination for use of any of claims 1-5, wherein the subject is a human.

7. The combination for use according to any of claims 1-6, wherein the therapy comprises:
(a) simultaneous administration of the inhibitor of PD-L1 and the inhibitor of TIM-3, optionally in a combination dosage form or by simultaneous administration of single inhibitors; or
(b) administration of the single inhibitors according to a schedule that results in effective amounts of each inhibitor present in the patient at the same time.

8. A kit for use in a method of treatment of a subject afflicted with a hematologic cancer, comprising a first agent that selectively inhibits or blocks PD-L1 and a second agent that selectively inhibits or blocks TIM-3,
wherein the first agent:
(i) is chosen from an inhibitory nucleic acid that inhibits expression of PD-L1, wherein the inhibitory nucleic acid that inhibits expression of PD-L1 comprises:
(a) an RNA interfering agent which inhibits expression of PD-L1, optionally wherein said RNA interfering agent is a small interfering RNA (siRNA), small hairpin RNA (shRNA), or a microRNA (miRNA); or
(b) an antisense oligonucleotide complementary to a nucleotide sequence of PD-L1,
or an antibody or antigen-binding fragment thereof, that binds to PD-L1;
(ii) is a bispecific or multispecific antibody, or antigen binding fragment thereof, selective for PD-L1 and TIM-3;
and optionally wherein the second agent is in a pharmaceutically acceptable formulation
wherein the second agent:
(i) is chosen from an inhibitory nucleic acid that inhibits expression of TIM-3, wherein the inhibitory nucleic acid that inhibits expression of TIM-3 comprises:
(a) an RNA interfering agent which inhibits expression of TIM-3, optionally wherein said RNA interfering agent is a small interfering RNA (siRNA), small hairpin RNA (shRNA), or a microRNA (miRNA); or
(b) antisense oligonucleotide complementary to a nucleotide sequence of TIM-3, or an antibody or antigen-binding fragment thereof, that binds to TIM-3;
(ii) is a bispecific or multispecific antibody, or antigen binding fragment thereof, selective for PD-L1 and TIM-3;
and optionally wherein the second agent is in a pharmaceutically acceptable formulation.

9. The kit for use of claim 8, wherein said first agent and/or second agent is an antibody, or an antigen binding fragment thereof, which specifically binds to PD-L1 protein and/or TIM-3 protein.

10. The kit for use of claim 8 or 9, wherein said antibody, or antigen binding fragment thereof:
(i) is murine, chimeric, humanized, composite, or human;
(ii) is detectably labeled, comprises an effector domain, comprises an Fc domain, and/or is selected from the group consisting of Fv, Fav, F(ab')2), Fab', dsFv, scFv, sc(Fv)2, and diabodies fragments; or
(iii) is conjugated to a cytotoxic agent, optionally wherein said cytotoxic agent is selected from the group consisting of a chemotherapeutic agent, a biologic agent, a toxin, and a radioactive isotope.


SEQUENCE LISTING

<110> COSTIM PHARMACEUTICALS INC.
      THE MEDICAL COLLEGE OF WISCONSIN, INC.

<120> METHODS FOR TREATING HEMATOLOGIC CANCERS

<130> C2160-7004WO

<140> PCT/US2014/057491
<141> 2014-09-25

<150> 62/017,192
<151> 2014-06-25

<150> 61/882,702
<151> 2013-09-26

<160> 16

<170> PatentIn version 3.5

<210> 1
<211> 921
<212> DNA
<213> Homo sapiens


<220>
<221> CDS
<222> (25)..(888)

<400> 1
cactctggtg gggctgctcc aggc atg cag atc cca cag gcg ccc tgg cca        51
gtc gtc tgg gcg gtg cta caa ctg ggc tgg cgg cca gga tgg ttc tta       99
gac tcc cca gac agg ccc tgg aac ccc ccc acc ttc tcc cca gcc ctg      147
ctc gtg gtg acc gaa ggg gac aac gcc acc ttc acc tgc agc ttc tcc      195
aac aca tcg gag agc ttc gtg cta aac tgg tac cgc atg agc ccc agc      243
aac cag acg gac aag ctg gcc gcc ttc ccc gag gac cgc agc cag ccc      291
ggc cag gac tgc cgc ttc cgt gtc aca caa ctg ccc aac ggg cgt gac      339
ttc cac atg agc gtg gtc agg gcc cgg cgc aat gac agc ggc acc tac      387
ctc tgt ggg gcc atc tcc ctg gcc ccc aag gcg cag atc aaa gag agc       435
ctg cgg gca gag ctc agg gtg aca gag aga agg gca gaa gtg ccc aca       483
gcc cac ccc agc ccc tca ccc agg tca gcc ggc cag ttc caa acc ctg       531
gtg gtt ggt gtc gtg ggc ggc ctg ctg ggc agc ctg gtg ctg cta gtc       579
tgg gtc ctg gcc gtc atc tgc tcc cgg gcc gca cga ggg aca ata gga       627
gcc agg cgc acc ggc cag ccc ctg aag gag gac ccc tca gcc gtg cct       675
gtg ttc tct gtg gac tat ggg gag ctg gat ttc cag tgg cga gag aag       723
acc ccg gag ccc ccc gtg ccc tgt gtc cct gag cag acg gag tat gcc       771
acc att gtc ttt cct agc gga atg ggc acc tca tcc ccc gcc cgc agg       819
ggc tca gct gac ggc cct cgg agt gcc cag cca ctg agg cct gag gat       867
gga cac tgc tct tgg ccc ctc tgaccggctt ccttggccac cagtgttctg cag       921

<210> 2
<211> 288
<212> PRT
<213> Homo sapiens

<400> 2

<210> 3

<211> 968
<212> DNA
<213> Homo sapiens

<220>
<221> CDS
<222> (59)..(793)

<400> 3
gcttcccgag gctccgcacc agccgcgctt ctgtccgcct gcagggcatt ccagaaag 58

atg agg ata ttt gct gtc ttt ata ttc atg acc tac tgg cat ttg ctg 106
Met Arg Ile Phe Ala Val Phe Ile Phe Met Thr Tyr Trp His Leu Leu
1               5                   10                  15

aac gca ttt act gtc acg gtt ccc aag gac cta tat gtg gta gag tat 154
Asn Ala Phe Thr Val Thr Val Pro Lys Asp Leu Tyr Val Val Glu Tyr
            20                  25                  30

ggt agc aat atg aca att gaa tgc aaa ttc cca gta gaa aaa caa tta 202
Gly Ser Asn Met Thr Ile Glu Cys Lys Phe Pro Val Glu Lys Gln Leu
        35                  40                  45

gac ctg gct gca cta att gtc tat tgg gaa atg gag gat aag aac att 250
Asp Leu Ala Ala Leu Ile Val Tyr Trp Glu Met Glu Asp Lys Asn Ile
    50                  55                  60

att caa ttt gtg cat gga gag gaa gac ctg aag gtt cag cat agt agc 298
Ile Gln Phe Val His Gly Glu Glu Asp Leu Lys Val Gln His Ser Ser
65                  70                  75                  80

tac aga cag agg gcc cgg ctg ttg aag gac cag ctc tcc ctg gga aat 346
Tyr Arg Gln Arg Ala Arg Leu Leu Lys Asp Gln Leu Ser Leu Gly Asn
                85                  90                  95

gct gca ctt cag atc aca gat gtg aaa ttg cag gat gca ggg gtg tac 394
Ala Ala Leu Gln Ile Thr Asp Val Lys Leu Gln Asp Ala Gly Val Tyr
            100                 105                 110

cgc tgc atg atc agc tat ggt ggt gcc gac tac aag cga att act gtg 442
Arg Cys Met Ile Ser Tyr Gly Gly Ala Asp Tyr Lys Arg Ile Thr Val
        115                 120                 125

aaa gtc aat gcc cca tac aac aaa atc aac caa aga att ttg gtt gtg 490
Lys Val Asn Ala Pro Tyr Asn Lys Ile Asn Gln Arg Ile Leu Val Val
    130                 135                 140

gat cca gtc acc tct gaa cat gaa ctg aca tgt cag gct gag ggc tac 538
Asp Pro Val Thr Ser Glu His Glu Leu Thr Cys Gln Ala Glu Gly Tyr
145                 150                 155                 160

ccc aag gcc gaa gtc atc tgg aca agc agt gac cat caa gtc ctg agt 586
Pro Lys Ala Glu Val Ile Trp Thr Ser Ser Asp His Gln Val Leu Ser
                165                 170                 175

ggt aag acc acc acc acc aat tcc aag aga gag gag aag ctt ttc aat 634
Gly Lys Thr Thr Thr Thr Asn Ser Lys Arg Glu Glu Lys Leu Phe Asn
            180                 185                 190

gtg acc agc aca ctg aga atc aac aca aca act aat gag att ttc tac 682
Val Thr Ser Thr Leu Arg Ile Asn Thr Thr Thr Asn Glu Ile Phe Tyr
        195                 200                 205

tgc act ttt agg aga tta gat cct gag gaa aac cat aca gct gaa ttg 730
Cys Thr Phe Arg Arg Leu Asp Pro Glu Glu Asn His Thr Ala Glu Leu
    210                 215                 220

gtc atc cca ggt aat att ctg aat gtg tcc att aaa ata tgt cta aca 778
Val Ile Pro Gly Asn Ile Leu Asn Val Ser Ile Lys Ile Cys Leu Thr
225                 230                 235                 240

ctg tcc cct agc acc tagcatgatg tctgcctatc atagtcattc agtgattgtt 833
Leu Ser Pro Ser Thr
                245

gaataaatga atgaatgaat aacactatgt ttacaaaata tatcctaatt cctcacctcc 893

attcatccaa accatattgt tacttaataa acattcagca gatatttatg gaataaaaaa 953

aaaaaaaaaa aaaaa 968

<210> 4
<211> 245
<212> PRT
<213> Homo sapiens

<400> 4
Met Arg Ile Phe Ala Val Phe Ile Phe Met Thr Tyr Trp His Leu Leu
1               5                   10                  15

Asn Ala Phe Thr Val Thr Val Pro Lys Asp Leu Tyr Val Val Glu Tyr
            20                  25                  30

Gly Ser Asn Met Thr Ile Glu Cys Lys Phe Pro Val Glu Lys Gln Leu
        35                  40                  45

Asp Leu Ala Ala Leu Ile Val Tyr Trp Glu Met Glu Asp Lys Asn Ile
    50                  55                  60

Ile Gln Phe Val His Gly Glu Glu Asp Leu Lys Val Gln His Ser Ser
65                  70                  75                  80

Tyr Arg Gln Arg Ala Arg Leu Leu Lys Asp Gln Leu Ser Leu Gly Asn
                85                  90                  95

Ala Ala Leu Gln Ile Thr Asp Val Lys Leu Gln Asp Ala Gly Val Tyr
            100                 105                 110

Arg Cys Met Ile Ser Tyr Gly Gly Ala Asp Tyr Lys Arg Ile Thr Val
        115                 120                 125

Lys Val Asn Ala Pro Tyr Asn Lys Ile Asn Gln Arg Ile Leu Val Val
    130                 135                 140

Asp Pro Val Thr Ser Glu His Glu Leu Thr Cys Gln Ala Glu Gly Tyr

145                 150                 155                 160

Pro Lys Ala Glu Val Ile Trp Thr Ser Ser Asp His Gln Val Leu Ser
                165                 170                 175

Gly Lys Thr Thr Thr Thr Asn Ser Lys Arg Glu Glu Lys Leu Phe Asn
            180                 185                 190

Val Thr Ser Thr Leu Arg Ile Asn Thr Thr Thr Asn Glu Ile Phe Tyr
        195                 200                 205

Cys Thr Phe Arg Arg Leu Asp Pro Glu Glu Asn His Thr Ala Glu Leu
    210                 215                 220

Val Ile Pro Gly Asn Ile Leu Asn Val Ser Ile Lys Ile Cys Leu Thr
225                 230                 235                 240

Leu Ser Pro Ser Thr
                245

<210> 5
<211> 1553
<212> DNA
<213> Homo sapiens

<220>
<221> CDS
<222> (53)..(922)

<400> 5
cgaggctccg caccagccgc gcttctgtcc gcctgcaggg cattccagaa ag atg agg      58
                                                          Met Arg
                                                          1

ata ttt gct gtc ttt ata ttc atg acc tac tgg cat ttg ctg aac gca      106
Ile Phe Ala Val Phe Ile Phe Met Thr Tyr Trp His Leu Leu Asn Ala
        5                   10                  15

ttt act gtc acg gtt ccc aag gac cta tat gtg gta gag tat ggt agc      154
Phe Thr Val Thr Val Pro Lys Asp Leu Tyr Val Val Glu Tyr Gly Ser
    20                  25                  30

aat atg aca att gaa tgc aaa ttc cca gta gaa aaa caa tta gac ctg      202
Asn Met Thr Ile Glu Cys Lys Phe Pro Val Glu Lys Gln Leu Asp Leu
35                  40                  45                  50

gct gca cta att gtc tat tgg gaa atg gag gat aag aac att att caa      250
Ala Ala Leu Ile Val Tyr Trp Glu Met Glu Asp Lys Asn Ile Ile Gln
                55                  60                  65

ttt gtg cat gga gag gaa gac ctg aag gtt cag cat agt agc tac aga      298
Phe Val His Gly Glu Glu Asp Leu Lys Val Gln His Ser Ser Tyr Arg
            70                  75                  80

cag agg gcc cgg ctg ttg aag gac cag ctc tcc ctg gga aat gct gca      346

Gln Arg Ala Arg Leu Leu Lys Asp Gln Leu Ser Leu Gly Asn Ala Ala
        85                  90                  95

ctt cag atc aca gat gtg aaa ttg cag gat gca ggg gtg tac cgc tgc 394
Leu Gln Ile Thr Asp Val Lys Leu Gln Asp Ala Gly Val Tyr Arg Cys
    100                 105                 110

atg atc agc tat ggt ggt gcc gac tac aag cga att act gtg aaa gtc 442
Met Ile Ser Tyr Gly Gly Ala Asp Tyr Lys Arg Ile Thr Val Lys Val
115                 120                 125                 130

aat gcc cca tac aac aaa atc aac caa aga att ttg gtt gtg gat cca 490
Asn Ala Pro Tyr Asn Lys Ile Asn Gln Arg Ile Leu Val Val Asp Pro
                135                 140                 145

gtc acc tct gaa cat gaa ctg aca tgt cag gct gag ggc tac ccc aag 538
Val Thr Ser Glu His Glu Leu Thr Cys Gln Ala Glu Gly Tyr Pro Lys
            150                 155                 160

gcc gaa gtc atc tgg aca agc agt gac cat caa gtc ctg agt ggt aag 586
Ala Glu Val Ile Trp Thr Ser Ser Asp His Gln Val Leu Ser Gly Lys
        165                 170                 175

acc acc acc acc aat tcc aag aga gag gag aag ctt ttc aat gtg acc 634
Thr Thr Thr Thr Asn Ser Lys Arg Glu Glu Lys Leu Phe Asn Val Thr
    180                 185                 190

agc aca ctg aga atc aac aca aca act aat gag att ttc tac tgc act 682
Ser Thr Leu Arg Ile Asn Thr Thr Thr Asn Glu Ile Phe Tyr Cys Thr
195                 200                 205                 210

ttt agg aga tta gat cct gag gaa aac cat aca gct gaa ttg gtc atc 730
Phe Arg Arg Leu Asp Pro Glu Glu Asn His Thr Ala Glu Leu Val Ile
                215                 220                 225

cca gaa cta cct ctg gca cat cct cca aat gaa agg act cac ttg gta 778
Pro Glu Leu Pro Leu Ala His Pro Pro Asn Glu Arg Thr His Leu Val
            230                 235                 240

att ctg gga gcc atc tta tta tgc ctt ggt gta gca ctg aca ttc atc 826
Ile Leu Gly Ala Ile Leu Leu Cys Leu Gly Val Ala Leu Thr Phe Ile
        245                 250                 255

ttc cgt tta aga aaa ggg aga atg atg gat gtg aaa aaa tgt ggc atc 874
Phe Arg Leu Arg Lys Gly Arg Met Met Asp Val Lys Lys Cys Gly Ile
    260                 265                 270

caa gat aca aac tca aag aag caa agt gat aca cat ttg gag gag acg 922
Gln Asp Thr Asn Ser Lys Lys Gln Ser Asp Thr His Leu Glu Glu Thr
275                 280                 285                 290

taatccagca ttggaacttc tgatcttcaa gcagggattc tcaacctgtg gtttaggggt 982

tcatcggggc tgagcgtgac aagaggaagg aatgggcccg tgggatgcag gcaatgtggg 1042

acttaaaagg cccaagcact gaaaatggaa cctggcgaaa gcagaggagg agaatgaaga 1102

aagatggagt caaacaggga gcctggaggg agaccttgat actttcaaat gcctgagggg 1162

ctcatcgacg cctgtgacag ggagaaagga tacttctgaa caaggagcct ccaagcaaat 1222

catccattgc tcatcctagg aagacgggtt gagaatccct aatttgaggg tcagttcctg 1282

cagaagtgcc ctttgcctcc actcaatgcc tcaatttgtt ttctgcatga ctgagagtct 1342

cagtgttgga acgggacagt atttatgtat gagtttttcc tatttatttt gagtctgtga 1402

ggtcttcttg tcatgtgagt gtggttgtga atgatttctt ttgaagatat attgtagtag 1462

atgttacaat tttgtcgcca aactaaactt gctgcttaat gatttgctca catctagtaa 1522

aacatggagt atttgtaaaa aaaaaaaaaa a 1553

<210> 6
<211> 290
<212> PRT
<213> Homo sapiens

<400> 6
Met Arg Ile Phe Ala Val Phe Ile Phe Met Thr Tyr Trp His Leu Leu
1               5                   10                  15

Asn Ala Phe Thr Val Thr Val Pro Lys Asp Leu Tyr Val Val Glu Tyr
            20                  25                  30

Gly Ser Asn Met Thr Ile Glu Cys Lys Phe Pro Val Glu Lys Gln Leu
        35                  40                  45

Asp Leu Ala Ala Leu Ile Val Tyr Trp Glu Met Glu Asp Lys Asn Ile
    50                  55                  60

Ile Gln Phe Val His Gly Glu Glu Asp Leu Lys Val Gln His Ser Ser
65                  70                  75                  80

Tyr Arg Gln Arg Ala Arg Leu Leu Lys Asp Gln Leu Ser Leu Gly Asn
                85                  90                  95

Ala Ala Leu Gln Ile Thr Asp Val Lys Leu Gln Asp Ala Gly Val Tyr
            100                 105                 110

Arg Cys Met Ile Ser Tyr Gly Gly Ala Asp Tyr Lys Arg Ile Thr Val
        115                 120                 125

Lys Val Asn Ala Pro Tyr Asn Lys Ile Asn Gln Arg Ile Leu Val Val
    130                 135                 140

Asp Pro Val Thr Ser Glu His Glu Leu Thr Cys Gln Ala Glu Gly Tyr
145                 150                 155                 160

Pro Lys Ala Glu Val Ile Trp Thr Ser Ser Asp His Gln Val Leu Ser
                165                 170                 175

Gly Lys Thr Thr Thr Thr Asn Ser Lys Arg Glu Glu Lys Leu Phe Asn
            180                 185                 190

Val Thr Ser Thr Leu Arg Ile Asn Thr Thr Thr Asn Glu Ile Phe Tyr
        195                 200                 205

Cys Thr Phe Arg Arg Leu Asp Pro Glu Glu Asn His Thr Ala Glu Leu
    210                 215                 220

Val Ile Pro Glu Leu Pro Leu Ala His Pro Pro Asn Glu Arg Thr His
225                 230                 235                 240

Leu Val Ile Leu Gly Ala Ile Leu Leu Cys Leu Gly Val Ala Leu Thr
                245                 250                 255

Phe Ile Phe Arg Leu Arg Lys Gly Arg Met Met Asp Val Lys Lys Cys
            260                 265                 270

Gly Ile Gln Asp Thr Asn Ser Lys Lys Gln Ser Asp Thr His Leu Glu
        275                 280                 285

Glu Thr
    290

<210> 7
<211> 873
<212> DNA
<213> Mus sp.

<400> 7
atgaggatat ttgctggcat tatattcaca gcctgctgtc acttgctacg ggcgtttact 60

atcacggctc caaaggactt gtacgtggtg gagtatggca gcaacgtcac gatggagtgc 120

agattccctg tagaacggga gctggacctg cttgcgttag tggtgtactg ggaaaaggaa 180

gatgagcaag tgattcagtt tgtggcagga gaggaggacc ttaagcctca gcacagcaac 240

ttcaggggga gagcctcgct gccaaaggac cagcttttga agggaaatgc tgcccttcag 300

atcacagacg tcaagctgca ggacgcaggc gtttactgct gcataatcag ctacggtggt 360

gcggactaca agcgaatcac gctgaaagtc aatgccccat accgcaaaat caaccagaga 420

atttccgtgg atccagccac ttctgagcat gaactaatat gtcaggccga gggttatcca 480

gaagctgagg taatctggac aaacagtgac caccaacccg tgagtgggaa gagaagtgtc 540

accacttccc ggacagaggg gatgcttctc aatgtgacca gcagtctgag ggtcaacgcc 600

acagcgaatg atgttttcta ctgtacgttt tggagatcac agccagggca aaaccacaca 660

gcggagctga tcatcccaga actgcctgca acacatcctc cacagaacag gactcactgg 720

gtgcttctgg gatccatcct gttgttcctc attgtagtgt ccacggtcct cctcttcttg 780

agaaaacaag tgagaatgct agatgtggag aaatgtggcg ttgaagatac aagctcaaaa 840

aaccgaaatg atacacaatt cgaggagacg taa                                    873

<210> 8
<211> 290
<212> PRT
<213> Mus sp.

<400> 8
Met Arg Ile Phe Ala Gly Ile Ile Phe Thr Ala Cys Cys His Leu Leu
1               5                   10                  15

Arg Ala Phe Thr Ile Thr Ala Pro Lys Asp Leu Tyr Val Val Glu Tyr
            20                  25                  30

Gly Ser Asn Val Thr Met Glu Cys Arg Phe Pro Val Glu Arg Glu Leu
        35                  40                  45

Asp Leu Leu Ala Leu Val Val Tyr Trp Glu Lys Glu Asp Glu Gln Val
    50                  55                  60

Ile Gln Phe Val Ala Gly Glu Glu Asp Leu Lys Pro Gln His Ser Asn
65                  70                  75                  80

Phe Arg Gly Arg Ala Ser Leu Pro Lys Asp Gln Leu Leu Lys Gly Asn
                85                  90                  95

Ala Ala Leu Gln Ile Thr Asp Val Lys Leu Gln Asp Ala Gly Val Tyr
            100                 105                 110

Cys Cys Ile Ile Ser Tyr Gly Gly Ala Asp Tyr Lys Arg Ile Thr Leu
        115                 120                 125

Lys Val Asn Ala Pro Tyr Arg Lys Ile Asn Gln Arg Ile Ser Val Asp
    130                 135                 140

Pro Ala Thr Ser Glu His Glu Leu Ile Cys Gln Ala Glu Gly Tyr Pro
145                 150                 155                 160

Glu Ala Glu Val Ile Trp Thr Asn Ser Asp His Gln Pro Val Ser Gly
                165                 170                 175

Lys Arg Ser Val Thr Thr Ser Arg Thr Glu Gly Met Leu Leu Asn Val
            180                 185                 190

Thr Ser Ser Leu Arg Val Asn Ala Thr Ala Asn Asp Val Phe Tyr Cys
        195                 200                 205

Thr Phe Trp Arg Ser Gln Pro Gly Gln Asn His Thr Ala Glu Leu Ile

210                 215                 220

Ile Pro Glu Leu Pro Ala Thr His Pro Pro Gln Asn Arg Thr His Trp
225                 230                 235                 240

Val Leu Leu Gly Ser Ile Leu Leu Phe Leu Ile Val Val Ser Thr Val
                245                 250                 255

Leu Leu Phe Leu Arg Lys Gln Val Arg Met Leu Asp Val Glu Lys Cys
            260                 265                 270

Gly Val Glu Asp Thr Ser Ser Lys Asn Arg Asn Asp Thr Gln Phe Glu
        275                 280                 285

Glu Thr
    290

<210> 9
<211> 906
<212> DNA
<213> Homo sapiens

<400> 9
atgttttcac atcttccctt tgactgtgtc ctgctgctgc tgctgctact acttacaagg      60

tcctcagaag tggaatacag agcggaggtc ggtcagaatg cctatctgcc ctgcttctac     120

accccagccg ccccagggaa cctcgtgccc gtctgctggg gcaaaggagc ctgtcctgtg     180

tttgaatgtg gcaacgtggt gctcaggact gatgaaaggg atgtgaatta ttggacatcc     240

agatactggc taaatgggga tttccgcaaa ggagatgtgt ccctgaccat agagaatgtg     300

actctagcag acagtgggat ctactgctgc cggatccaaa tcccaggcat aatgaatgat     360

gaaaaattta acctgaagtt ggtcatcaaa ccagccaagg tcacccctgc accgactcgg     420

cagagagact tcactgcagc ctttccaagg atgcttacca ccaggggaca tggcccagca     480

gagacacaga cactggggag cctccctgat ataaatctaa cacaaatatc cacattggcc     540

aatgagttac gggactctag attggccaat gacttacggg actctggagc aaccatcaga     600

ataggcatct acatcggagc agggatctgt gctgggctgg ctctggctct tatcttcggc     660

gctttaattt tcaaatggta ttctcatagc aaagagaaga tacagaattt aagcctcatc     720

tctttggcca acctccctcc ctcaggattg gcaaatgcag tagcagaggg aattcgctca     780

gaagaaaaca tctataccat tgaagagaac gtatatgaag tggaggagcc caatgagtat     840

tattgctatg tcagcagcag gcagcaaccc tcacaacctt tgggttgtcg ctttgcaatg     900

ccatag                                                                906

<210> 10

<211> 301
<212> PRT
<213> Homo sapiens

<400> 10
Met Phe Ser His Leu Pro Phe Asp Cys Val Leu Leu Leu Leu Leu Leu
1               5                   10                  15

Leu Leu Thr Arg Ser Ser Glu Val Glu Tyr Arg Ala Glu Val Gly Gln
            20                  25                  30

Asn Ala Tyr Leu Pro Cys Phe Tyr Thr Pro Ala Ala Pro Gly Asn Leu
        35                  40                  45

Val Pro Val Cys Trp Gly Lys Gly Ala Cys Pro Val Phe Glu Cys Gly
    50                  55                  60

Asn Val Val Leu Arg Thr Asp Glu Arg Asp Val Asn Tyr Trp Thr Ser
65                  70                  75                  80

Arg Tyr Trp Leu Asn Gly Asp Phe Arg Lys Gly Asp Val Ser Leu Thr
                85                  90                  95

Ile Glu Asn Val Thr Leu Ala Asp Ser Gly Ile Tyr Cys Cys Arg Ile
            100                 105                 110

Gln Ile Pro Gly Ile Met Asn Asp Glu Lys Phe Asn Leu Lys Leu Val
        115                 120                 125

Ile Lys Pro Ala Lys Val Thr Pro Ala Pro Thr Arg Gln Arg Asp Phe
    130                 135                 140

Thr Ala Ala Phe Pro Arg Met Leu Thr Thr Arg Gly His Gly Pro Ala
145                 150                 155                 160

Glu Thr Gln Thr Leu Gly Ser Leu Pro Asp Ile Asn Leu Thr Gln Ile
                165                 170                 175

Ser Thr Leu Ala Asn Glu Leu Arg Asp Ser Arg Leu Ala Asn Asp Leu
            180                 185                 190

Arg Asp Ser Gly Ala Thr Ile Arg Ile Gly Ile Tyr Ile Gly Ala Gly
        195                 200                 205

Ile Cys Ala Gly Leu Ala Leu Ala Leu Ile Phe Gly Ala Leu Ile Phe
    210                 215                 220

Lys Trp Tyr Ser His Ser Lys Glu Lys Ile Gln Asn Leu Ser Leu Ile
225                 230                 235                 240

Ser Leu Ala Asn Leu Pro Pro Ser Gly Leu Ala Asn Ala Val Ala Glu
                245                 250                 255

Gly Ile Arg Ser Glu Glu Asn Ile Tyr Thr Ile Glu Glu Asn Val Tyr
            260                 265                 270

Glu Val Glu Glu Pro Asn Glu Tyr Tyr Cys Tyr Val Ser Ser Arg Gln
        275                 280                 285

Gln Pro Ser Gln Pro Leu Gly Cys Arg Phe Ala Met Pro
    290                 295                 300

<210> 11
<211> 846
<212> DNA
<213> Mus sp.

<400> 11
atgttttcag gtcttaccct caactgtgtc ctgctgctgc tgcaactact acttgcaagg      60

tcattggaaa atgcttatgt gtttgaggtt ggtaagaatg cctatctgcc ctgcagttac     120

actctatcta cacctggggc acttgtgcct atgtgctggg gcaagggatt ctgtccttgg     180

tcacagtgta ccaacgagtt gctcagaact gatgaaagaa atgtgacata tcagaaatcc     240

agcagatacc agctaaaggg cgatctcaac aaaggagacg tgtctctgat cataaagaat     300

gtgactctgg atgaccatgg gacctactgc tgcaggatac agttccctgg tcttatgaat     360

gataaaaaat tagaactgaa attagacatc aaagcagcca aggtcactcc agctcagact     420

gcccatgggg actctactac agcttctcca agaaccctaa ccacggagag aaatggttca     480

gagacacaga cactggtgac cctccataat aacaatggaa caaaaatttc cacatgggct     540

gatgaaatta aggactctgg agaaacgatc agaactgcta tccacattgg agtgggagtc     600

tctgctgggt tgaccctggc acttatcatt ggtgtcttaa tccttaaatg gtattcctgt     660

aagaaaaaga agttatcgag tttgagcctt attacactgg ccaacttgcc tccaggaggg     720

ttggcaaatg caggagcagt caggattcgc tctgaggaaa atatctacac catcgaggag     780

aacgtatatg aagtggagaa ttcaaatgag tactactgct acgtcaacag ccagcagcca     840

tcctga                                                                846

<210> 12
<211> 281
<212> PRT
<213> Mus sp.

<400> 12
Met Phe Ser Gly Leu Thr Leu Asn Cys Val Leu Leu Leu Leu Gln Leu
1               5                   10                  15

Leu Leu Ala Arg Ser Leu Glu Asn Ala Tyr Val Phe Glu Val Gly Lys
            20                  25                  30

Asn Ala Tyr Leu Pro Cys Ser Tyr Thr Leu Ser Thr Pro Gly Ala Leu
        35                  40                  45

Val Pro Met Cys Trp Gly Lys Gly Phe Cys Pro Trp Ser Gln Cys Thr
    50                  55                  60

Asn Glu Leu Leu Arg Thr Asp Glu Arg Asn Val Thr Tyr Gln Lys Ser
65                  70                  75                  80

Ser Arg Tyr Gln Leu Lys Gly Asp Leu Asn Lys Gly Asp Val Ser Leu
                85                  90                  95

Ile Ile Lys Asn Val Thr Leu Asp Asp His Gly Thr Tyr Cys Cys Arg
            100                 105                 110

Ile Gln Phe Pro Gly Leu Met Asn Asp Lys Lys Leu Glu Leu Lys Leu
        115                 120                 125

Asp Ile Lys Ala Ala Lys Val Thr Pro Ala Gln Thr Ala His Gly Asp
    130                 135                 140

Ser Thr Thr Ala Ser Pro Arg Thr Leu Thr Thr Glu Arg Asn Gly Ser
145                 150                 155                 160

Glu Thr Gln Thr Leu Val Thr Leu His Asn Asn Asn Gly Thr Lys Ile
                165                 170                 175

Ser Thr Trp Ala Asp Glu Ile Lys Asp Ser Gly Glu Thr Ile Arg Thr
            180                 185                 190

Ala Ile His Ile Gly Val Gly Val Ser Ala Gly Leu Thr Leu Ala Leu
        195                 200                 205

Ile Ile Gly Val Leu Ile Leu Lys Trp Tyr Ser Cys Lys Lys Lys Lys
    210                 215                 220

Leu Ser Ser Leu Ser Leu Ile Thr Leu Ala Asn Leu Pro Pro Gly Gly
225                 230                 235                 240

Leu Ala Asn Ala Gly Ala Val Arg Ile Arg Ser Glu Glu Asn Ile Tyr
                245                 250                 255

Thr Ile Glu Glu Asn Val Tyr Glu Val Glu Asn Ser Asn Glu Tyr Tyr

260                 265                 270

Cys Tyr Val Asn Ser Gln Gln Pro Ser
        275                 280

<210> 13
<211> 1578
<212> DNA
<213> Homo sapiens

<400> 13
atgtgggagg ctcagttcct gggcttgctg tttctgcagc cgctttgggt ggctccagtg      60

aagcctctcc agccaggggc tgaggtcccg gtggtgtggg cccaggaggg ggctcctgcc     120

cagctcccct gcagccccac aatccccctc caggatctca gccttctgcg aagagcaggg     180

gtcacttggc agcatcagcc agacagtggc ccgcccgctg ccgcccccgg ccatcccctg     240

gcccccggcc ctcacccggc ggcgccctcc tcctgggggc ccaggccccg ccgctacacg     300

gtgctgagcg tgggtcccgg aggcctgcgc agcgggaggc tgcccctgca gccccgcgtc     360

cagctggatg agcgcggccg gcagcgcggg gacttctcgc tatggctgcg cccagcccgg     420

cgcgcggacg ccggcgagta ccgcgccgcg gtgcacctca gggaccgcgc cctctcctgc     480

cgcctccgtc tgcgcctggg ccaggcctcg atgactgcca gccccccagg atctctcaga     540

gcctccgact gggtcatttt gaactgctcc ttcagccgcc ctgaccgccc agcctctgtg     600

cattggttcc ggaaccgggg ccagggccga gtccctgtcc gggagtcccc ccatcaccac     660

ttagcggaaa gcttcctctt cctgccccaa gtcagcccca tggactctgg gccctggggc     720

tgcatcctca cctacagaga tggcttcaac gtctccatca tgtataacct cactgttctg     780

ggtctggagc ccccaactcc cttgacagtg tacgctggag caggttccag ggtggggctg     840

ccctgccgcc tgcctgctgg tgtggggacc cggtctttcc tcactgccaa gtggactcct     900

cctgggggag gccctgacct cctggtgact ggagacaatg gcgactttac ccttcgacta     960

gaggatgtga gccaggccca ggctgggacc tacacctgcc atatccatct gcaggaacag    1020

cagctcaatg ccactgtcac attggcaatc atcacagtga ctcccaaatc ctttgggtca    1080

cctggatccc tggggaagct gctttgtgag gtgactccag tatctggaca agaacgcttt    1140

gtgtggagct ctctggacac cccatcccag aggagtttct caggaccttg gctggaggca    1200

caggaggccc agctcctttc ccagccttgg caatgccagc tgtaccaggg ggagaggctt    1260

cttggagcag cagtgtactt cacagagctg tctagcccag gtgcccaacg ctctgggaga    1320

gccccaggtg ccctcccagc aggccacctc ctgctgtttc tcatccttgg tgtcctttct    1380

ctgctccttt tggtgactgg agcctttggc tttcaccttt ggagaagaca gtggcgacca    1440

agacgatttt ctgccttaga gcaagggatt caccctccgc aggctcagag caagatagag    1500

gagctggagc aagaaccgga gccggagccg gagccggaac cggagcccga gcccgagccc 1560

gagccggagc agctctga 1578

<210> 14
<211> 525
<212> PRT
<213> Homo sapiens

<400> 14
Met Trp Glu Ala Gln Phe Leu Gly Leu Leu Phe Leu Gln Pro Leu Trp
1               5                   10                  15

Val Ala Pro Val Lys Pro Leu Gln Pro Gly Ala Glu Val Pro Val Val
            20                  25                  30

Trp Ala Gln Glu Gly Ala Pro Ala Gln Leu Pro Cys Ser Pro Thr Ile
        35                  40                  45

Pro Leu Gln Asp Leu Ser Leu Leu Arg Arg Ala Gly Val Thr Trp Gln
    50                  55                  60

His Gln Pro Asp Ser Gly Pro Pro Ala Ala Ala Pro Gly His Pro Leu
65                  70                  75                  80

Ala Pro Gly Pro His Pro Ala Ala Pro Ser Ser Trp Gly Pro Arg Pro
                85                  90                  95

Arg Arg Tyr Thr Val Leu Ser Val Gly Pro Gly Gly Leu Arg Ser Gly
            100                 105                 110

Arg Leu Pro Leu Gln Pro Arg Val Gln Leu Asp Glu Arg Gly Arg Gln
        115                 120                 125

Arg Gly Asp Phe Ser Leu Trp Leu Arg Pro Ala Arg Arg Ala Asp Ala
    130                 135                 140

Gly Glu Tyr Arg Ala Ala Val His Leu Arg Asp Arg Ala Leu Ser Cys
145                 150                 155                 160

Arg Leu Arg Leu Arg Leu Gly Gln Ala Ser Met Thr Ala Ser Pro Pro
                165                 170                 175

Gly Ser Leu Arg Ala Ser Asp Trp Val Ile Leu Asn Cys Ser Phe Ser
            180                 185                 190

Arg Pro Asp Arg Pro Ala Ser Val His Trp Phe Arg Asn Arg Gly Gln
        195                 200                 205

Gly Arg Val Pro Val Arg Glu Ser Pro His His His Leu Ala Glu Ser
    210                 215                 220

Phe Leu Phe Leu Pro Gln Val Ser Pro Met Asp Ser Gly Pro Trp Gly
225                 230                 235                 240

Cys Ile Leu Thr Tyr Arg Asp Gly Phe Asn Val Ser Ile Met Tyr Asn
                245                 250                 255

Leu Thr Val Leu Gly Leu Glu Pro Pro Thr Pro Leu Thr Val Tyr Ala
            260                 265                 270

Gly Ala Gly Ser Arg Val Gly Leu Pro Cys Arg Leu Pro Ala Gly Val
        275                 280                 285

Gly Thr Arg Ser Phe Leu Thr Ala Lys Trp Thr Pro Pro Gly Gly Gly
    290                 295                 300

Pro Asp Leu Leu Val Thr Gly Asp Asn Gly Asp Phe Thr Leu Arg Leu
305                 310                 315                 320

Glu Asp Val Ser Gln Ala Gln Ala Gly Thr Tyr Thr Cys His Ile His
                325                 330                 335

Leu Gln Glu Gln Gln Leu Asn Ala Thr Val Thr Leu Ala Ile Ile Thr
            340                 345                 350

Val Thr Pro Lys Ser Phe Gly Ser Pro Gly Ser Leu Gly Lys Leu Leu
        355                 360                 365

Cys Glu Val Thr Pro Val Ser Gly Gln Glu Arg Phe Val Trp Ser Ser
    370                 375                 380

Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415

Gly Glu Arg Leu Leu Gly Ala Ala Val Tyr Phe Thr Glu Leu Ser Ser
            420                 425                 430

Pro Gly Ala Gln Arg Ser Gly Arg Ala Pro Gly Ala Leu Pro Ala Gly
        435                 440                 445

His Leu Leu Leu Phe Leu Ile Leu Gly Val Leu Ser Leu Leu Leu Leu
    450                 455                 460

Val Thr Gly Ala Phe Gly Phe His Leu Trp Arg Arg Gln Trp Arg Pro
465                 470                 475                 480

Arg Arg Phe Ser Ala Leu Glu Gln Gly Ile His Pro Pro Gln Ala Gln
                485                 490                 495

Ser Lys Ile Glu Glu Leu Glu Gln Glu Pro Glu Pro Glu Pro Glu Pro
            500                 505                 510

Glu Pro Glu Pro Glu Pro Glu Pro Glu Pro Glu Gln Leu
        515                 520                 525

<210> 15
<211> 1566
<212> DNA
<213> Mus sp.

<400> 15
atgagggagg acctgctcct tggctttttg cttctgggac tgctttggga agctccagtt      60

gtgtcttcag ggcctgggaa agagctcccc gtggtgtggg cccaggaggg agctcccgtc     120

catcttccct gcagcctcaa atcccccaac ctggatccta actttctacg aagaggaggg     180

gttatctggc aacatcaacc agacagtggc caacccactc ccatcccggc ccttgacctt     240

caccagggga tgccctcgcc tagacaaccc gcacccggtc gctacacggt gctgagcgtg     300

gctccaggag gcctgcgcag cgggaggcag cccctgcatc cccacgtgca gctggaggag     360

cgcggcctcc agcgcgggga cttctctctg tggttgcgcc cagctctgcg caccgatgcg     420

ggcgagtacc acgccaccgt gcgcctcccg aaccgcgccc tctcctgcag tctccgcctg     480

cgcgtcggcc aggcctcgat gattgctagt ccctcaggag tcctcaagct gtctgattgg     540

gtccttttga actgctcctt cagccgtcct gaccgcccag tctctgtgca ctggttccag     600

ggccagaacc gagtgcctgt ctacaactca ccgcgtcatt ttttagctga aactttcctg     660

ttactgcccc aagtcagccc cctggactct gggacctggg gctgtgtcct cacctacaga     720

gatggcttca atgtctccat cacgtacaac ctcaaggttc tgggtctgga gcccgtagcc     780

cctctgacag tgtacgctgc tgaaggttct agggtggagc tgccctgtca tttgccccca     840

ggagtgggga ccccttcttt gctcattgcc aagtggactc ctcctggagg aggtcctgag     900

ctccccgtgg ctggaaagag tggcaatttt acccttcacc ttgaggctgt gggtctggca     960

caggctggga cctacacctg tagcatccat ctgcagggac agcagctcaa tgccactgtc    1020

acgttggcgg tcatcacagt gactcccaaa tccttcgggt tacctggctc ccgggggaag    1080

ctgttgtgtg aggtaacccc ggcatctgga aaggaaagat ttgtgtggcg tcccctgaac    1140

aatctgtcca ggagttgccc gggccctgtg ctggagattc aggaggccag gctccttgct    1200

gagcgatggc agtgtcagct gtacgagggc cagaggcttc ttggagcgac agtgtacgcc 1260

gcagagtcta gctcaggcgc ccacagtgct aggagaatct caggtgacct taaaggaggc 1320

catctcgttc tcgttctcat ccttggtgcc ctctccctgt tccttttggt ggccggggcc 1380

tttggctttc actggtggag aaaacagttg ctactgagaa gattttctgc cttagaacat 1440

gggattcagc catttccggc tcagaggaag atagaggagc tggagcgaga actggagacg 1500

gagatgggac aggagccgga gcccgagccg gagccacagc tggagccaga gcccaggcag 1560

ctctga 1566

<210> 16
<211> 521
<212> PRT
<213> Mus sp.

<400> 16
Met Arg Glu Asp Leu Leu Leu Gly Phe Leu Leu Leu Gly Leu Leu Trp
1               5                   10                  15

Glu Ala Pro Val Val Ser Ser Gly Pro Gly Lys Glu Leu Pro Val Val
            20                  25                  30

Trp Ala Gln Glu Gly Ala Pro Val His Leu Pro Cys Ser Leu Lys Ser
        35                  40                  45

Pro Asn Leu Asp Pro Asn Phe Leu Arg Arg Gly Gly Val Ile Trp Gln
    50                  55                  60

His Gln Pro Asp Ser Gly Gln Pro Thr Pro Ile Pro Ala Leu Asp Leu
65                  70                  75                  80

His Gln Gly Met Pro Ser Pro Arg Gln Pro Ala Pro Gly Arg Tyr Thr
                85                  90                  95

Val Leu Ser Val Ala Pro Gly Gly Leu Arg Ser Gly Arg Gln Pro Leu
            100                 105                 110

His Pro His Val Gln Leu Glu Glu Arg Gly Leu Gln Arg Gly Asp Phe
        115                 120                 125

Ser Leu Trp Leu Arg Pro Ala Leu Arg Thr Asp Ala Gly Glu Tyr His
    130                 135                 140

Ala Thr Val Arg Leu Pro Asn Arg Ala Leu Ser Cys Ser Leu Arg Leu
145                 150                 155                 160

Arg Val Gly Gln Ala Ser Met Ile Ala Ser Pro Ser Gly Val Leu Lys
                165                 170                 175

Leu Ser Asp Trp Val Leu Leu Asn Cys Ser Phe Ser Arg Pro Asp Arg
            180                 185                 190

Pro Val Ser Val His Trp Phe Gln Gly Gln Asn Arg Val Pro Val Tyr
        195                 200                 205

Asn Ser Pro Arg His Phe Leu Ala Glu Thr Phe Leu Leu Leu Pro Gln
    210                 215                 220

Val Ser Pro Leu Asp Ser Gly Thr Trp Gly Cys Val Leu Thr Tyr Arg
225                 230                 235                 240

Asp Gly Phe Asn Val Ser Ile Thr Tyr Asn Leu Lys Val Leu Gly Leu
                245                 250                 255

Glu Pro Val Ala Pro Leu Thr Val Tyr Ala Ala Glu Gly Ser Arg Val
            260                 265                 270

Glu Leu Pro Cys His Leu Pro Pro Gly Val Gly Thr Pro Ser Leu Leu
        275                 280                 285

Ile Ala Lys Trp Thr Pro Pro Gly Gly Gly Pro Glu Leu Pro Val Ala
    290                 295                 300

Gly Lys Ser Gly Asn Phe Thr Leu His Leu Glu Ala Val Gly Leu Ala
305                 310                 315                 320

Gln Ala Gly Thr Tyr Thr Cys Ser Ile His Leu Gln Gly Gln Gln Leu
                325                 330                 335

Asn Ala Thr Val Thr Leu Ala Val Ile Thr Val Thr Pro Lys Ser Phe
            340                 345                 350

Gly Leu Pro Gly Ser Arg Gly Lys Leu Leu Cys Glu Val Thr Pro Ala
        355                 360                 365

Ser Gly Lys Glu Arg Phe Val Trp Arg Pro Leu Asn Asn Leu Ser Arg
    370                 375                 380

Ser Cys Pro Gly Pro Val Leu Glu Ile Gln Glu Ala Arg Leu Leu Ala
385                 390                 395                 400

Glu Arg Trp Gln Cys Gln Leu Tyr Glu Gly Gln Arg Leu Leu Gly Ala
                405                 410                 415

Thr Val Tyr Ala Ala Glu Ser Ser Ser Gly Ala His Ser Ala Arg Arg

420                     425                     430

Ile Ser Gly Asp Leu Lys Gly Gly His Leu Val Leu Val Leu Ile Leu
        435                     440                     445

Gly Ala Leu Ser Leu Phe Leu Leu Val Ala Gly Ala Phe Gly Phe His
    450                     455                     460

Trp Trp Arg Lys Gln Leu Leu Leu Arg Arg Phe Ser Ala Leu Glu His
465                     470                     475                     480

Gly Ile Gln Pro Phe Pro Ala Gln Arg Lys Ile Glu Glu Leu Glu Arg
                485                     490                     495

Glu Leu Glu Thr Glu Met Gly Gln Glu Pro Glu Pro Glu Pro Glu Pro
            500                     505                     510

Gln Leu Glu Pro Glu Pro Arg Gln Leu
        515                     520